(19) Europäisches Patentamt
European Patent Office
Office européen des brevets

(11) EP 3 562 526 B1

(12) **EUROPÄISCHE PATENTSCHRIFT**

(45) Veröffentlichungstag und Bekanntmachung des Hinweises auf die Patenterteilung:
**12.11.2025 Patentblatt 2025/46**

(21) Anmeldenummer: **18700022.9**

(22) Anmeldetag: **02.01.2018**

(51) Internationale Patentklassifikation (IPC):
***A61L 31/02*** *(2006.01)* ***A61L 31/08*** *(2006.01)*
***A61L 31/14*** *(2006.01)*

(52) Gemeinsame Patentklassifikation (CPC):
**A61L 31/028; A61L 31/088; A61L 31/148;**
A61L 2420/08

(86) Internationale Anmeldenummer:
**PCT/EP2018/050084**

(87) Internationale Veröffentlichungsnummer:
**WO 2018/122418 (05.07.2018 Gazette 2018/27)**

(54) **STENT AUS EINER BIOLOGISCH ABBAUBAREN MAGNESIUMLEGIERUNG MIT EINER MAGNESIUMFLUORID-BESCHICHTUNG UND EINER ORGANISCHEN BESCHICHTUNG**

STENT MADE OF A BIODEGRADABLE MAGNESIUM ALLOY WITH A MAGNESIUM FLUORIDE COATING AND AN ORGANIC COATING

ENDOPROTHÈSE EN ALLIAGE DE MAGNÉSIUM BIODÉGRADABLE AYANT UN REVÊTEMENT DE FLUORURE DE MAGNÉSIUM ET UN REVÊTEMENT ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.12.2016 EP 16207023**

(43) Veröffentlichungstag der Anmeldung:
**06.11.2019 Patentblatt 2019/45**

(73) Patentinhaber: **MeKo Manufacturing e.K.**
**31157 Sarstedt (DE)**

(72) Erfinder:
- **MEYER-KOBBE, Clemens**
  **31157 Sarstedt (DE)**
- **STEKKER, Michael**
  **26605 Aurich (DE)**
- **MENZE, Roman**
  **30177 Hannover (DE)**

(74) Vertreter: **Arth, Hans-Lothar**
**ABK Patent Attorneys**
**Jasminweg 9**
**14052 Berlin (DE)**

(56) Entgegenhaltungen:
**WO-A1-2013/024125**
**WO-A2-2010/025078**
**WO-A2-2016/073851**
**CN-A- 101 468 216**
**US-A1- 2010 145 436**

- **WANG ZHENLIN ET AL: "Corrosion resistance and adhesion of poly(L-lactic acid)/ MgF2composite coating on AZ31 magnesium alloy for biomedical application", RUSSIAN JOURNAL OF NON-FERROUS METALS, ALLERTON PRESS, INC, US, vol. 57, no. 4, 10 September 2016 (2016-09-10), pages 381 - 388, XP036053335, ISSN: 1067-8212, [retrieved on 20160910], DOI: 10.3103/S1067821216040155**
- **XIAOLI LIU ET AL: "Multifunctional MgF2/ Polydopamine Coating on Mg Alloy for Vascular Stent Application", JOURNAL OF MATERIALS SCIENCE & TECHNOLOGY, vol. 31, no. 7, 27 March 2015 (2015-03-27), AMSTERDAM, NL, pages 733 - 743, XP055637544, ISSN: 1005-0302, DOI: 10.1016/j.jmst.2015.02.002**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Stents hergestellt aus einer, unter physiologischen Bedingungen abbaubaren, Magnesiumlegierung mit einer anorganischen Beschichtung umfassend Magnesiumfluorid und mit einer organischen Beschichtung. Die erfindungsgemäßen Stents können dabei zusätzlich mit mindestens einem antiinflammatorischen, antiproliferativen, antiangiogenen, antirestenotischen und/oder antithrombogenen Wirkstoff beschichtet sein.

Die Implantation von Gefäßstützen wie beispielsweise Stents ist heutzutage ein gängiger chirurgischer Eingriff zur Behandlung von Stenosen. Sie werden üblicherweise aus Metalllegierungen wie nicht-rostendem Edelstahl oder Nitinol hergestellt. Derartige Metallstents sind in großer Zahl bekannt und haben sich in der Praxis bewährt. Auf Grund ihrer Metallstruktur und Tragkraft sollen derartige Metallstents gewährleisten, dass die Gefäße nach Implantation offen bleiben und der Blutfluss durch die Gefäße dauerhaft gewährleistet wird.

Neuere Untersuchungen haben allerdings gezeigt, dass Gefäßstenosen nicht permanent durch eine Endoprothese, insbesondere in Form eines Stents, aufgeweitet werden müssen. Es ist vollkommen ausreichend, das Blutgefäß für einen begrenzten Zeitraum zu stützen, da das traumatisierte Gewebe des Gefäßes verheilt und die glatten Gefäßmuskelzellen regenerieren und wieder die Aufgabe übernehmen, das Blutgefäß offen zu halten und deshalb der Stent nicht länger als erforderlich im Gefäßlumen verbleiben muss.

Stents werden derzeit in zwei Grundtypen eingeteilt, dauerhafte und degradierbare bzw. resorbierbare Stents. Dauerhafte Stents sind so ausgestaltet, dass sie im Gefäß für einen unbestimmten Zeitraum verbleiben können. Resorbierbare Stents hingegen werden über einen vorbestimmten Zeitraum hinweg im Gefäß abgebaut.

Das Problem der Restenose nach Stentimplantation versucht man zurzeit zu lösen, indem man das Wachstum der glatten Muskelzellen lokal zu hemmen versucht. Dies wird z.B. mit Stents versucht, welche pharmazeutische Wirkstoffe freisetzen, die bevorzugt antiproliferativ wirken. Diese Wirkstoffe werden zumeist aus einer wirkstoffenthaltenden Beschichtung freigesetzt, welche sowohl auf dauerhaften als auch auf resorbierbaren Stents aufgebracht sein können.

Die Stützwirkung durch die Metallstruktur ist häufig nur kurzzeitig erforderlich, da sich nach Implantation des Stents das Körpergewebe erholen kann und die stützende Funktion nicht länger erforderlich ist. Vorzugsweise werden degradierbare und resorbierbare Stents erst abgebaut, wenn das traumatisierte Gewebe des Gefäßes verheilt ist und das Gefäß wieder stabilisiert, so dass der Stent nicht weiter im Gefäßlumen verbleiben muss Besonders im Falle der mit Blut in Kontakt kommenden Stents verursachen diese als körperfremdes Material eine Reaktion des umliegenden Gewebes, was durch Proliferation die Bildung von Restenosen bewirken kann. Bemühungen in der Weiterentwicklung der Stents hin zu einer verbesserten Biokompatibilität des Stentmaterials, höheren Flexibilität bei geringer werdender Materialermüdung und Verkleinerung der Fremdoberfläche sollen das Risiko der Stent-induzierten Restenoserate immer weiter minimieren. Resorbierbare Stents haben hier den Vorteil, dass das körperfremde Material nicht dauerhaft im Gefäß verbleibt und die Gefahr einer Restenose somit zeitlich begrenzt wird. Ein Einsatz von resorbierbaren Stents ist auch bei Kindern von Vorteil, da hier das Gefäßwachstum nicht negativ beeinflusst wird, bzw. der Stent nach einer Weile in der das Kind gewachsen ist, nicht wieder entfernt werden muss.

Aus diesem Grund werden in neuer Zeit in zunehmendem Maße Stents aus bioresorbierbaren Materialien wie z.B. aus Polymeren wie z.B. Polyhydroxybutyrat oder aus Metallen wie Magnesium oder Eisen entwickelt und in klinischen Versuchen getestet.

Die großen Rückstellkräfte der Gefäße in den ersten Tagen nach einer Aufdehnung sind ein wichtiger Grund für Restenosen. Daher müssen resorbierbare Gefäßstützen aus einem Material bestehen, welches zwar gut vom Körper abgebaut werden kann, aber auch eine ausreichend hohe Rückhaltekraft aufweist, um einen erneuten Verschluss des Gefäßes zu verhindern.

Ein einmal eingesetzter Stent muss seine Größe und Form beibehalten, trotz der unterschiedlichen Kräfte, die auf ihn einwirken, wie z.B. die pulsierende Belastung durch das schlagende Herz. Darüber hinaus muss der Stent genügend Flexibilität besitzen, um auf einen Ballon gekrimpt und später im Gefäß expandiert zu werden.

Resorbierbare Polymere, welche zur Herstellung von Stents verwendet werden, weisen geringere mechanische Festigkeitswerte auf als die bislang verwendeten nicht resorbierbaren Metalllegierungen. Eine Kompensation dieses Nachteils kann durch größere Stegbreiten des Stents erfolgen. Dies erhöht jedoch die mechanische Reizung der Gefäßwandung während der Stentimplantation und damit auch die Restenosegefahr. Resorbierbare Stents aus Eisen oder einer eisenbasierten Legierung haben den Nachteil, dass die Verweildauer im Gefäß bis zum vollständigen Abbau länger als notwendig und gewünscht ist. Für resorbierbare Stents liegt der angestrebte Zeitraum der Resorption zwischen 3 und 12 Monaten, wobei die mechanische Belastbarkeit zuvor gewährleistet sein muss. Magnesium ist als Spurenelement im Körper vorhanden und daher als Basis für einen resorbierbaren Stent geeignet. Des Weiteren wurden Legierungsbestandteile aus den Metallen der Gruppe der seltenen Erden gewählt, da diese im Körper natürlich nicht vorkommen. Dies ermöglicht einen Nachweis der Degradationsprodukte im Gewebe und in den Organen.

Magnesium und Magnesiumlegierungen besitzen für eine Vielzahl von Anwendungen hervorragende mechanische und physikalische Eigenschaften. Ihr geringes Gewicht bei gleichzeitig hoher Festigkeit machen Magnesium und

Magnesiumlegierungen zu geeigneten Materialien auch für Endoprothesen. Magnesium und Magnesiumlegierungen sind sehr reaktionsfreudig und daher korrosionsanfällig. Für resorbierbare Implantate sind diese Eigenschaften jedoch wünschenswert. Allerdings bestehen folgende Probleme im Stand der Technik: Obgleich grundsätzlich das angestrebte Ziel einer Resorption des implantierten Stents erreicht wird, besteht das Problem eines zeitlich nicht definierten Abbaus des Stents. Je nach Materialwahl ist der Materialabbau Schwankungen unterworfen, nicht kontrollierbar und im Allgemeinen zu schnell, um ein sicheres Einwachsen des Stents in die Gefäßwände zu gewährleisten. Bei zu schneller Resorption kann der resorbierbare Stent nicht in die Gefäßwand einwachsen und die Stützfunktion bis zur Regeneration des Gefäßabschnitts übernehmen. Er kann sich vielmehr ablösen bzw. Stücke des Stents können sich ablösen und im Blutfluss mitgerissen werden und lebensbedrohliche Probleme für den Patienten verursachen.

Ein bioresorbierbarer Metallstent aus Magnesium und Yttrium ist in dem europäischen Patent EP 1 419 793 B1 offenbart. Eine Magnesiumlegierung mit Yttrium, Neodym und weiteren optionalen Bestandteilen geeignet für die Herstellung von Stents wird in dem europäischen Patent EP 2 169 090 beschrieben. Diese Stents haben den Nachteil, dass sie sich zu schnell und zudem unkontrolliert auflösen. Da der Auflösungsprozess zumeist beginnt, bevor der Stent in die Gefäßwand eingewachsen ist, können sich Bruchstücke lösen, durch die Blutbahn transportiert werden und einen Herzinfarkt auslösen. Ferner hat sich herausgestellt, dass diese Stents aus einer Magnesium-Yttrium-Legierung die Ablagerung von Calciumphosphat auf der luminalen Oberfläche der Stents begünstigen und damit zu einem Wiederverschluss des Stents (In-Stent Restenose) und damit auch des Gefäßes führen, was gerade verhindert werden soll.

Die europäischen Patentanmeldungen EP 2 213 314 A1 und EP 1 842 507 A1 offenbaren ebenfalls Stents aus einer Magnesiumlegierung enthaltend Gadolinium. Um die gewünschten mechanischen Eigenschaften wie beispielsweise Festigkeit oder Duktilität zu erhalten, wird Gadolinium in Mengen größer 5 Gew.-% benötigt. Bei Mengen größer als 5 Gew.-% Gadolinium tritt jedoch das Problem auf, dass die Verarbeitbarkeit der Legierung zu einem Rohr geeignet für die Laserbearbeitung und die Homogenität der Legierung nicht mehr gewährleistet ist. Die schlechtere Verarbeitbarkeit führte zu dickeren Stentstreben, welche ein Problem darstellen, da der Blutstrom behindert wurde, was Thromben zur Folge hat.

Einen Einfluss auf die Kinetik der Resorption bzw. Degradation kann auch durch eine Abstimmung aus einem optimierten Werkstoff für das Grundgerüst und einer Beschichtung, die die Auflösungskinetik positiv beeinflusst, d.h. verlangsamt, erreicht werden. Eine solche Beschichtung muss zudem widerstandsfähig genug sein, um die Belastungen denen ein Stent zum Beispiel während der Implantierung ausgesetzt ist ohne Beschädigung zu überstehen.

Aus dem Stand der Technik sind unter anderen Magnesiumfluorid-Beschichtungen für Stents aus bioresorbierbaren Magnesiumlegierungen bekannt. Lin et al. beschreiben einen schützenden Effekt des Magnesiumfluoridfilms auf die JBDM-Legierung Mg2,5Nd0,2Zn0,4Zr (ACS Applied Materials & Interfaces 2015, 7, S. 5320-5330). Der Magnesiumfluoridfilm wurde durch Behandlung der Legierung mit einer Kaliumfluorid-Lösung erzeugt. In vitro-Untersuchungen zeigten, dass die Degradationsrate um 20% gegenüber der unbeschichteten Legierung gesenkt werden konnte.

Nan et al. untersuchten den Einfluss einer Magnesiumfluorid- und einer Collagenbeschichtung auf die Bildung von Endothelzellen auf bioresorbierbaren Magnesiumstützen (International Journal of Molecular Sciences 2014, 15, S. 5263-5276). Auf einer Magnesiumfluorid-Beschichtung war die Endothelzellanlagerung und Proliferation deutlich ausgeprägter gegenüber der Collagenbeschichtung.

Die internationale Patentanmeldung WO 2014/143521 A1 offenbart Stents aus bioabsorbierbaren Magnesiumlegierung, deren Korrosion durch Passivierung mittels hydrothermaler Behandlung verlangsamt wurde. Die Korrosionsbeständig nimmt mit steigender Schichtdicke zu. Allerdings sind für eine ausreichend lange Beständigkeit Schichtdicken von über 150 μm notwendig, wodurch vermehrt Risse in der Beschichtung aufgrund der dynamischen Belastung des Stents auftreten können. Die Stents können zusätzlich eine Polymerbeschichtung aufweisen, die den Stentkörper von der äußeren Umgebung abschirmt und mithin die Korrosion verlangsamt.

Die internationale Patentanmeldung WO 2016/073851 A2 offenbart ebenfalls Stents aus einer bioabsorbierbaren Magnesiumlegierung, deren Korrosion durch Passivierung mittels hydrothermaler Behandlung verlangsamt wurde, und die eine Feuchtigkeitssperrschicht aufweisen. Diese zusätzliche Schicht besteht aus einem Metall, einem metallorganischen Derivat oder einem polymeren Alkoxid wie Polyaluminiumethylenglykol (Alucon).

In der Offenlegungsschrift US 2010/145436 A1 werden bioabbaubare Stents aus einem Metall oder einem bioabsorbierbaren oder biostabilen Polymer offenbart, die eine degradatationsverzögernde Beschichtung aus Magnesiumfluorid oder eine Polymerbeschichtung aufweisen. Die Polymerbeschichtung löst sich bei der Expansion vom Stent ab, sodass der Stent in Kontakt mit der Gefäßwand tritt.

Biostabile metallische Stents mit einer Wirkstoff enthaltenden, porösen Oberfläche und einer nicht-polymeren bioabbaubaren Beschichtung werden in WO 2010/025078 A2 beschrieben. Die bioabbaubare Beschichtung aus Magnesiumfluorid, Calciumphosphat, Apatit, Calciumcarbonat oder Calciumfluorid dient der verzögerten kontrollierten Freisetzung des Wirkstoffs.

Aus diesem Grund besteht das Bedürfnis einen geeigneten Werkstoff für resorbierbare Stents zu entwickeln und diesen mit einer Beschichtung zu kombinieren, welche es erlaubt, die Degradation des Stents zu steuern. Aufgabe der vorliegenden Erfindung ist es, eine Gefäßstütze bereitzustellen, welche ihre Stützfunktion nur so lange ausübt, bis das regenerierte Gewebe wieder selber in der Lage ist, diese Funktion zu übernehmen und die Nachteile des Standes der

Technik vermeidet.

WO 2013/024125 A1 beschreibt resorbierbare Stents aus einer Magnesiumlegierung mit einer äußeren Polymerbeschichtung. Die Magnesiumlegierungen weisen einen einen relativ hohen Gehalt an Dysprosium und zusätzlich Neodym und/oder Europium und optional Zirkonium und/oder Zink auf, wodurch ein vorteilhaftes Korrosionsverhalten, eine gewünschte Resorptionskinetik und für die Herstellung von Stents geeigneten mechanischen Eigenschaften ergeben. Die Polymerbeschichtung dient der Verlangsamung der Degradation der Magnesiumlegierung.

Die chinesische Patentanmeldung CN101468216 betrifft Stents aus einer Magnesium-Legierung mit einer Fluorid-Konversionsbeschichtung und einer Wirkstoff-enthaltenden Polymer- oder Albumin-Beschichtung. Als Polymer werden Phosphorylcholin, Polylactid und deren Copolymere, Chitosan, PEG, Polyurethan, Polydioxanon und deren Copolymere, Polyanhydrid, Polytrimethylencarbonat-Ester und deren Copolymere, Kollagen, Gelatine oder Chondroitinsulfat verwendet.

Fasst man die Aufgabe konkreter, so besteht die Aufgabe der vorliegenden Erfindung darin, einen Stent aus einer Magnesiumlegierung und einer darauf abgestimmten Beschichtung bereitzustellen, dessen Auflösungskinetik gegenüber den bekannten Stents verzögert bis deutlich verlangsamt ist.

Diese Aufgabe wird durch die technische Lehre des unabhänigigen Anspruchs 1 der vorliegenden Erfindung gelöst. Weitere vorteilhafte Ausgestaltungen der Erfindung ergeben sich aus den abhängigen Ansprüchen, der Beschreibung sowie den Beispielen.

Es hat sich überraschend gezeigt, dass Stents aus einer Magnesiumlegierung sich durch ein vorteilhaftes Korrosionsverhalten, eine gewünschte Resorptionskinetik und eine hohe Biokompatibilität auszeichnen, wenn sie mit einer hierin offenbarten Magnesiumfluorid-Beschichtung und einer organischen Beschichtung versehen werden.

Die Verwendung einer Magnesiumfluorid-Beschichtung und einer zweiten organischen Beschichtung ermöglicht es, bei geringen Schichtdicken von wenigen Mikrometern, eine Rissbildung und damit eine vorzeitige Degradation des Stents zu vermeiden.

Die vorliegende Erfindung betrifft daher Stents bestehend aus einer biologisch abbaubaren Magnesiumlegierung mit einer anorganischen Beschichtung umfassend Magnesiumfluorid und mit einer organischen Beschichtung, wobei die Magnesium- legierung mindestens 80 Gew.-% Magnesium enthält und wobei die anorganische Beschichtung den Stent bedeckt und die organische Beschichtung die anorganische Beschichtung bedeckt, wobei die organische Beschichtung eine oder mehrere Substanzen der folgenden Gruppe umfasst:

Polyvinylpyrrolidon, Glycerin, Polyhydroxyethyl-methacrylate, Polypropylenglycol, Polyvinylalkohol Polygluconat, Polyaminosäuren, Polyphosphatester, Polyvalerolactone, Poly-ε-Decalactone, Polyglycolsäure, Poly-L-Lactid, Poly D,L-Lactid, sowie Blends wie Poly(L-Lactid-co-glycolid), Poly(D,L-lactid-co-glycolid), Poly(L-Lactid-co-D,L-Lactid), Poly-ε-caprolacton, Polyhydroxybuttersäure, Polyhydroxyvalerate, Polyhydroxybutyrate-co-valerate, Polymaleinsäureanhydride, Polyhydroxymethacrylate, Fibrin, Polycyanoacrylate, Polycaprolactondimethylacrylate, Polycaprolactonbutylacrylate, Polyetherestermulti-blockpolymere aus PEG und Polybutylenterephtalat, Polypivotolactone, Polyglycolsäuretrimethylcarbonate Polycaprolactonglycolide, Poly(DTH-Iminocarbonat), Poly(DTE-co-DT-carbonat), Poly(Bisphenol A-iminocarbonat), Polyorthoester, Polytrimethylcarbonate Polyiminocarbonate, Poly(N-vinyl)-Pyrrolidon, Polyesteramide, glycolierte Polyester, Polyphosphoester, Polyphosphazene, Poly[p-carboxyphenoxy)propan], Polyethylenoxid-propylenoxid, Polyalkenoxalate, Lipide, Wachse, Öle, mehrfach ungesättigte Fettsäuren, Eicosapentaensäure, Timnodonsäure, Docosahexaensäure, Arachidonsäure, Linolsäure, α-Linolensäure, γ-Linolensäure, Carrageenane, Fibrinogen, Agar-Agar, Stärke, Zein, Polyhydroxyalkanoate, Pectinsäure, Actinsäure, Carboxymethylsulfat, Hyaluronsäure, Heparansulfate und seine Derivate, Heparine, Dextran, β-Cyclodextrine, Gummi arabicum, Guar, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polyvinylketone, Polyvinylhalogenide, Polyvinyliden-halogenide, Polyvinylether, Polyisobutylene, Polyvinylaromaten, Polyvinylester, Polyoxymethylene, Polytetramethylenoxid, Polyethylen, Polypropylen, Polytetrafluorethylen, Polyolefin-Elastomere, Carboxymethylchitosane, Polyetheretherketone, Polyethylenterephtalat, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Polysulfone, Epoxyharze, ABS-Harze, EPDM-Gummis, Celluloseether, Cellulosetriacetate, Schellack, Poly-para-Xylylene (Parylene) wie Parylen N, Parylen C.

Die anorganische Magnesiumfluorid-Beschichtung des Stents kann durch eine Umwandlung des Grundmaterials im Oberflächenbereich/Randzonenbereich oder als aufgetragenes Substrat hergestellt werden. Die Magnesiumfluorid-Beschichtung des Stents durch eine Umwandlung des Grundmaterials im Oberflächenbereich/ Randzonenbereich bedeutet dabei die Bildung einer anorganischen Schicht aus dem Material des Stents selbst. Zur Umwandlung des Grundmaterials im Oberflächenbereich/Randzonenbereich kann durch eine Ionenimplantation oder aber auch durch eine chemische Umwandlung, wie beispielsweise die Reaktion mit Flusssäure, Kaliumfluorid, usw. erfolgen. Zur Herstellung einer anorganischen Beschichtung eines Stents als aufgetragenes Substrat umfasst die Beschichtung Magnesiumfluorid, das über die unterschiedlichen hierin beschriebenen chemischen und physikalischen Methoden aufgetragen werden kann.

Die Auftragung der anorganischen Beschichtung beschränkt sich nicht auf die Oberfläche eines Stents, sondern

kann nach Bedarf auch innerhalb des Stents erzeugt werden. So kann eine ionenimplantierte Randzone je nach verwendeten Versuchsparametern mittels eines Ionenimplantationverfahrens hergestellt werden.

Die Schichtdicke der Randzone ist durch die Wahl der Versuchsparameter dabei modifizierbar.

Die anorganische Beschichtung oder auch die Oberflächenmodifizierung mittels Ionenimplantation der erfindungsgemäßen Stents betrifft vorzugsweise die Stentstreben des Grundgerüsts selbst oder das gesamte Stentgerüst, d.h. den gesamten Stent. Wahlweise ist der Stent auf beiden Seiten, der abluminalen als auch der luminalen Seite des Stentkörpers oder nur auf einer der beiden Seiten beschichtet. Die Beschichtungen der luminalen bzw. abluminalen Seite des Stents können auch unterschiedlich sein, z.B. im Wirkstoffgehalt oder in der Zusammensetzung der organischen Verbindungen.

Überraschend wurde gefunden, dass die anorganische Beschichtung umfassend oder bestehend aus Magnesiumfluorid die Haftung einer darüber liegenden organischen Beschichtung im Vergleich zur Stentoberfläche verstärkt und gleichzeitig die organische Beschichtung die Festigkeit der Zwischenschicht aus Magnesiumfluorid erhöht. Somit wird ein vorzeitiges Ablösen der organischen Beschichtung aufgrund der adhäsiven Zwischenschicht aus Magnesiumfluorid verhindert und ein Aufreißen und/oder Abplatzen der Zwischenschicht aus Magnesiumfluorid durch die darüber liegende organische Beschichtung nach dem Crimpen und während der Dilatation vermieden.

Erfindungsgemäß verhindert die organische Beschichtung die vorzeitige und inhomogene Degradation des Stents bestehend aus einer Magnesiumlegierung mit einer anorganischen Beschichtung, indem die organische Schicht auf der anorganischen Beschichtung aufgetragen ist. Daher betrifft die vorliegende Erfindung Stents aus einem Grundgerüst aus einer der hierin offenbarten bioabbaubaren Magnesiumlegierungen mit mindestens 80 Gew.-% Magnesium, einer anorganischen Beschichtung umfassend oder bestehend aus Magnesiumfluorid und einer organischen Beschichtung, wobei die organische Beschichtung die anorganische Beschichtung bedeckt. Somit ist bevorzugt, dass die anorganische Beschichtung aus Magnesiumfluorid vorzugsweise den Stent aus der Magnesium-legierung vollständig, d.h. lückenlos auf seiner luminalen und abluminalen Oberfläche bedeckt und die organische Beschichtung, welche vorzugsweise aus organischen Polymeren besteht, wiederum als Schicht die anorganische Beschichtung aus Magnesiumfluorid vollständig bedeckt.

**In** anderen Worten weisen die erfindungsgemäßen Stents zwei Beschichtungen auf, wobei die erste Beschichtung als diejenige bezeichnet wird, welche direkt auf den Stent aufgebracht ist. Als zweite Beschichtung wird diejenige Beschichtung bezeichnet, welche auf diese erste, anorganische Beschichtung aufgebracht wird, nachdem diese bereits auf dem Stent ist. Somit wird die erste Beschichtung auch als Zwischenschicht bezeichnet, da sie sich zwischen der Stentoberfläche und der organischen zweiten Beschichtung befindet.

Dieses Zweischichtsystem aus einer Schicht aus Magnesiumfluorid und einer äußeren Schicht aus einem organischen Polymer oder Polymergemisch ist erfindungswesentlich. Der erfindungsgemäße Effekt der Degradationsverzögerung wird nicht erreicht, wenn die Schichten vertauscht werden, also die untere Schicht das organische Polymer oder Polymergemisch ist und die äußere Schicht aus Magnesiumfluorid besteht. Ebenfalls wird die erfindungsgemäße Wirkung nicht erzielt, wenn kein Zweischichtsystem ausgebildet wird, d.h. Magnesiumfluorid und organisches Polymer oder Polymergemisch als eine einzige homogene oder auch heterogene Schicht auf den Stent aus einer Magnesiumlegierung aufgebracht werden. Daher ist bevorzugt, wenn nicht sogar zwingend, dass die anorganische Beschichtung aus Magnesiumfluorid direkt auf die Stentoberfläche aufgebracht wird. Die Stentoberfläche sollte keine andere Beschichtung aufweisen wie beispielsweise eine Carbidschicht, Nitridschicht, Magnesiumoxidschicht oder eine Schicht aus DLC (diamond like carbon) oder der gleichen.

Hingegen ist es möglich, auf die organische Beschichtung aus dem organischen Polymer oder organischen Polymergemisch eine weitere vorzugsweise organische Schicht aufzubringen. Eine solche zusätzliche äußere und vorzugsweise organische Beschichtung kann beispielsweise dazu dienen, die Gleiteigenschaften der Stentoberfläche zu verbessern oder als Matrix für einen Wirkstoff zu dienen wie z.B. einem Antirestenosewirkstoff wie hierin beschrieben.

## Magnesiumlegierung

Das innere Gerüst bzw. der Körper der Gefäßstütze oder des erfindungsgemäßen Stents besteht aus einer Magnesiumlegierung. Diese Legierung besteht aus 0,1 bis 15,5 Gew.-% Dy und 0,01 bis 1,5 Gew.-% Nd oder 0,01 bis 1,5 Gew.-% Eu oder 0,01 bis 1,5 Gew.-% Nd und Eu zusammen, 0,0 Gew.-% - 2,0 Gew.-% Zink und 0,0 Gew.-% - 1,0 Gew.-% Zirkonium, wobei der restliche Anteil bis hin zu 100 Gew.-% Mg ist. Das heißt diese Legierungen enthalten 80,0 Gew.-% bis 99,89 Gew.-% Magnesium und bevorzugt 80,0 Gew.-% bis 97,0 Gew.-% Magnesium und weiter bevorzugt 85,0 Gew.-% bis 95,0 Gew.-% Magnesium. Diese Legierung kann ferner weitere Metalle und unvermeidbare Verunreinigungen enthalten. Bevorzugte Bereiche für die Bestandteile Dy, Nd, Eu, Zn und Zr werden weiter unten eingehend beschrieben.

In anderen Worten umfasst die Magnesiumlegierung aus der das innere Gerüst bzw. der Körper der Gefäßstütze oder des erfindungsgemäßen Stents besteht

| | |
|---|---|
| 0,1 Gew.-% bis 15,0 Gew.-% | Dy und |
| 0,01 Gew.-% bis 1,5 Gew.-% | Nd oder |
| 0,01 Gew.-% bis 1,5 Gew.-% | Eu oder |
| 0,01 Gew.-% bis 1,5 Gew.-% | Nd und Eu zusammen, |
| 0,0 Gew.-% bis 2,0 Gew.-% | Zink und |
| 0,0 Gew.-% bis 1,0 Gew.-% | Zirkonium und |
| mindestens 80 Gew.-% | Magnesium. |

Das innere Gerüst der Gefäßstütze ist zudem bevorzugt aus Magnesiumlegierungen umfassend 0,1 bis 15,0 Gew.-% Dy und 0,01 bis 1,5 Gew.-% Nd oder 0,01 bis 1,5 Gew.-% Eu oder 0,01 bis 1,5 Gew.-% Nd und Eu zusammen, 0,0 Gew.-% bis 2,0, bevorzugt bis 1,5 Gew.-% Zink, des Weiteren umfassend 0,1 Gew.-% - 0,75 Gew.-% Zirkonium. Auch diese Legierungen können ferner noch unvermeidbare Verunreinigungen enthalten.

Insbesondere bevorzugt ist, wenn das innere Gerüst eines erfindungsgemäßen Stents aus Magnesiumlegierungen besteht, die bezogen auf das Gesamtgewicht der Legierung (angegeben in Gew.-%) folgende Bestandteile enthalten:

| | |
|---|---|
| 81,25 Gew.-% - 99,89 Gew.-% | Magnesium |
| 0,1 Gew.-% - 15,0 Gew.-% | Dysprosium |
| 0,01 Gew.-% - 1,5 Gew.-% | Neodym und/oder Europium |
| 0,0 Gew.-% - 1,5 Gew.-% | Zink |
| 0,0 Gew.-% - 0,75 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Magnesium (Mg) als Hauptbestandteil der Legierung wurde ausgewählt, da Mg biologisch abbaubar und ein notwendiger Körperbestandteil ist, der sich nicht so im Körper anreichert, dass er schädlich wirkt. Überschüssiges Magnesium wird in der Regel auf natürlichem Wege ausgeschieden. Die Magnesiumlegierungen der erfindungsgemäßen Gefäßstützen bestehen zu mindestens 80 Gew.-% aus Magnesium.

Für die Herstellung von Stents wurde insbesondere auf die Festigkeitseigenschaften und das Korrosionsverhalten geachtet, um eine möglichst feste Legierung mit geeignetem Korrosionsverhalten bereitzustellen, wobei die Steuerung des Korrosionsverhaltens über die anorganische Beschichtung und die organische Beschichtung eingestellt wird.

Es wurde gefunden, dass das Minimum der Korrosion der hier beschriebenen Magnesiumlegierungen bei einem Gehalt von 10 Gew.-% Dy liegt. Daher ist es besonders bevorzugt, wenn der Gehalt an Dysprosium in den entsprechenden Legierungen bei circa 10 Gew.-% $\pm$ 2 Gew.-% liegt. Die Korrosion ist die entscheidende Eigenschaft für die Abbaurate des Stents im Gefäß. Es ist wichtig, dass ein biodegradierbarer Stent nicht zu früh seine Stabilität verliert, so dass sich keine Bruchstücke ablösen und die Stabilität durch den Stent gewährleistet wird, bis diese wieder von dem Gefäß alleine aufgebracht werden kann und bis der Stent in die Gefäßwand eingewachsen ist. Die anorganische Beschichtung und die organische Beschichtung dienen insbesondere dem Zweck, die Abstimmung der Resorptionskinetik vorzunehmen und die Abbaurate des Stents so zu steuern, dass der Stent sich nicht auflöst bevor er in die Gefäßwand eingewachsen ist. Zudem bildet Dysprosium zusammen mit Magnesium intermetallische Ausscheidungen. Die hohe Löslichkeit von Dysprosium in Magnesium sorgt zudem dafür, dass man die bei der Herstellung von Stents erforderlichen Wärmebehandlungen erfolgreich durchführen kann, sich Ausscheidungen auflösen und gezielt erneut ausscheiden und man so Eigenschaften wie z.B. Festigkeit, Duktilität und Korrosionsverhalten in einem weiten Rahmen einstellen kann.

Auch Neodym und Europium haben *in vitro* keine negativen Effekte auf Zellen gezeigt. Europium war hier gegenüber Neodym sogar noch etwas besser verträglich. Beide Elemente sind praktisch unlöslich in Magnesium und bilden intermetallische Phasen mit Magnesium, die, auch durch die bei der Herstellung von Stents notwendigen Wärmebehandlungen, nicht aufgelöst werden. Diese Ausscheidungen befinden sich auf den Korngrenzen und stabilisieren diese, so dass das feine Korn aus der Umformung beibehalten wird. Erfindungsgemäß hat sich gezeigt, dass dazu 1 Gew.-% Neodym oder 1 Gew.-% Europium oder 1 Gew.-% von Europium und Neodym zusammen ausreichen.

Zink verbessert das Gießverhalten der Magnesiumlegierung und hat eine festigkeitssteigernde Wirkung. So kann durch einen Zinkzusatz bis zu 3 Gew.-% die Schwing- und Zugfestigkeit erhöht werden. Die Zugfestigkeit sollte vorzugsweise so groß wie möglich sein und vorzugsweise über 180 MPa ($\geq$180 MPa), weiter bevorzugt über 200 MPa ($\geq$200 MPa) liegen. Allerdings steigt bei mehr als 1 Gew.-% Zn die Tendenz zur Heißrissbildung (s. Figur 8). Dadurch entstehen Mikroporen, die sich negativ auf die Zugfestigkeit und Duktilität einer Legierung auswirken. Sie wirken als

innere Kerben, so dass ein Werkstoff im Zugversuch generell deutlich unterhalb der maximal erreichbaren Festigkeit bei einem Bruchteil der theoretischen Duktilität versagt. Generell zeigt sich bei mehr als 2 Gew.-% Zink nachteilige Wirkungen auf das Verarbeitungsverhalten und die mechanischen Eigenschaften der erfindungsgemäßen Legierungen. Zink ist ein essentielles Element für den Menschen, welches Bestandteil vieler Enzyme ist und viele Funktionen hat. Zink hat unter anderem eine entzündungshemmende Wirkung. Dennoch kann bei hohen Dosen eine akute Vergiftung auftreten und eine längerfristige Zufuhr führt zu Störungen, insbesondere des Eisen- und Kupferhaushaltes (vgl. Guidelines for drinking-water quality, World Health Organization, 1996). Bei einem Zinkgehalt von 4 Gew.-% und mehr können daher toxische Nebenwirkungen nicht ausgeschlossen werden. Der Gehalt an Zink in der Legierung sollte unterhalb von 2,0 Gew.-%, bevorzugt unterhalb von 1,8 Gew.-%, weiter bevorzugt unterhalb von 1,6 Gew.-%, noch weiter bevorzugt unterhalb von 1,4 Gew.-% und insbesondere bevorzugt unterhalb von 1,2 Gew.-% liegen. In einigen Ausführungsformen der vorliegenden Erfindung sind die Magnesiumlegierungen der erfindungsgemäßen Stents frei von Zink.

Neben Zink oder auch an Stelle von Zink kann zudem Zirkonium (Zr) in der Magnesiumlegierung enthalten sein. Zr wird hier als Kornfeinungsmittel eingesetzt. Für eine Magnesiumlegierung zur Herstellung der erfindungsgemäßen Stents legiert man Zr in einer Größenordnung von bis zu ca. 0,75 Gew.-% zu. Auch größere Mengen an Zr von 2 Gew.-% oder auch 3 Gew.-% führen zu einer ähnlich guten Kornfeinung, verteuern aber die Legierung deutlich und führen zudem zu einer Versprödung der Legierung, was wiederum zu einer Erniedrigung der Duktilität führt. Da aber mit deutlich geringeren Mengen an Zr von 0,0 Gew.-% bis 0,50 Gew.-% genau so gute Resultate hinsichtlich der Kornfeinung als mit 1 Gew.-%, 2 Gew.-% oder 3 Gew.-% erzielt wurden und zudem bei einer Menge an Zr unterhalb von 0,75 Gew.-% keine Versprödung auftritt, werden erfindungsgemäß 0,0 Gew.-% bis 1,0 Gew.-% und weiter bevorzugt 0,1 Gew.-% bis 0,75 Gew.-% Zirkonium eingesetzt.

Der Einfluss von Zr wurde beispielhaft an der Magnesiumlegierung enthaltend 10 Gew.-% Dy und 1 Gew.-% Nd untersucht. Als Herstellungsverfahren wurde das Tütenguss-Verfahren eingesetzt. Bei den im Tütenguss hergestellten Werkstoffen kann man davon ausgehen, dass ein Gussteil eine homogene Gefügestruktur zeigt und dass auch die Legierungselemente homogen verteilt sind. Allerdings ist das Gefüge auch vergleichsweise grob und die Korngröße liegt im Bereich mehrerer Millimeter (Abb. 1). Die Erfinder konnten zeigen, dass die Zugabe von nur 0,6 Gew.% Zr dagegen eine deutliche Abnahme der Korngröße bewirkt (Abb. 2). Es wurden daher drei unterschiedlich große Zr-Anteile (0,2, 0,4, 0,6 Gew.-%) und ihr Einfluss auf das sich ausbildende Gefüge untersucht. Zur Ermittlung der Korngröße wurde das Linienschnittverfahren angewandt. Überraschenderweise hat bereits ein geringer Anteil von 0,2 Gew.-% eine deutliche Kornfeinung zur Folge (Abb. 2) und die Korngröße liegt im Bereich von 102 μm. Die Zugabe von 0,4 bzw. 0,6 Gew.-% hat Korngrößen im Bereich von 68 μm bzw. 64 μm zur Folge (Abb. 4 und 5). Man kann daher schlussfolgern, dass bereits eine Zugabe von 0,2 Gew.-% Zr eine effektive Kornfeinung verursacht und dass überraschenderweise das gesamte Zr für die Kornfeinung aktiviert werden kann. Dies senkt allein die Kosten für Zr um ca. 50 %.

Es ist daher bevorzugt, wenn eine erfindungsgemäße Legierung des Weiteren 0,02 - 0,80 Gew.-%, bevorzugt 0,04 - 0,60 Gew.-%, bevorzugt 0,05 - 0,55 Gew.-%, weiter bevorzugt 0,06 - 0,50 Gew.-%, noch weiter bevorzugt 0,07 - 0,45 Gew.-%, noch weiter bevorzugt 0,08 - 0,40 Gew.-%, noch weiter bevorzugt 0,09 - 0,35 Gew.-%, noch weiter bevorzugt 0,10 - 0,30 Gew.-%, noch weiter bevorzugt 0,12 - 0,28 Gew.-% und insbesondere bevorzugt 0,15 - 0,25 Gew.-% Zirkonium aufweist.

Die vorliegende Erfindung betrifft zudem bevorzugt Stents aus biologisch abbaubaren Magnesiumlegierungen, die bezogen auf das Gesamtgewicht der Legierung (angegeben in Gew.-%) folgende Bestandteile enthalten:

| | |
|---|---|
| 80 Gew.-% - 94,9 Gew.-% | Magnesium |
| 5,0 Gew.-% - 13,0 Gew.-% | Dysprosium |
| 0,1 Gew.-% - 2,0 Gew.-% | Neodym |
| 0,0 Gew.-% - 2,0 Gew.-% | Zink |
| 0,0 Gew.-% - 3,0 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Optional kann in dieser Legierung der Anteil an Neodym durch Europium ersetzt werden, oder es können 0,1 Gew.-% - 2,0 Gew.-% Europium zusätzlich zugesetzt werden.

Es versteht sich von selbst, dass alle Bestandteile einer Legierung zusammen 100 Gew.-% ergeben müssen. Sofern nicht explizit aufgeführt, können die hierin offenbarten Legierungen unvermeidbare Verunreinigungen enthalten, welche im Bereich von der Nachweisgrenze oder im Bereich von 1 ppm bis zu 0,4 Gew.-% und besonders bevorzugt bis zu 0,1 Gew.-% liegen. Silizium als Hauptbestandteil der Verunreinigungen kann dabei bereits 0,3 Gew.-% ausmachen. Silizium (Si) sollte nicht in Mengen über 0,4 Gew.-%, bevorzugt über 0,3 Gew.-% und weiter bevorzugt über 0,2 Gew.-% in der Legierung vorhanden sein. Es ist daher besonders bevorzugt, wenn die unvermeidbaren Verunreinigungen außer Silizium insgesamt weniger als 0,3 Gew.-%, weiter bevorzugt 0,05 Gew.-% und insbesondere bevorzugt weniger als 300

ppm betragen. Diese Verunreinigungen (einschließlich Si) können auch dann in der Legierung vorhanden sein, wenn sie nicht explizit als Legierungsbestandteil aufgeführt sind und werden im Fall der Nichtnennung dem Gewichtsanteil des Bestandteils der Legierung zugerechnet, durch den sie in die Legierung gelangt sind.

Die Erfindung umfasst ferner Stents bestehend aus Magnesiumlegierungen, die bezogen auf das Gesamtgewicht der Legierung aus folgenden Bestandteilen bestehen:

| | |
|---|---|
| 80,0 Gew.-% - 99,9 Gew.-% | Magnesium |
| 0,1 Gew.-% - 13,0 Gew.-% | Dysprosium |
| 0,0 Gew.-% - 3,0 Gew.-% | Neodym |
| 0,0 Gew.-% - 1,0 Gew.-% | Zink |
| 0,0 Gew.-% - 1,0 Gew.-% | Zirkonium |
| 0,0 Gew.-% - 2,0 Gew.-% | anderen Metallen, Metallsalzen und Nichtmetallen, welche allgemein als Verunreinigungen bezeichnet werden, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Es ist bevorzugt, wenn die erfindungsgemäße Legierung 0,1 - 20,0 Gew.-%, weiter bevorzugt 0,15 - 19,0 Gew.-%, weiter bevorzugt 0,16 - 18,0 Gew.-%, weiter bevorzugt 0,17 - 17,0 Gew.-%, weiter bevorzugt 0,18 - 17,0 Gew.-%, weiter bevorzugt 0,19 - 16,5 Gew.-%, weiter bevorzugt 2,0 - 16,0 Gew.-%, weiter bevorzugt 2,1 - 16,0 Gew.-%, weiter bevorzugt 2,2 - 15,0 Gew.-%, weiter bevorzugt 2,2 - 14,0 Gew.-%, weiter bevorzugt 2,3 - 13,0 Gew.-%, weiter bevorzugt 2,4 - 13,5 Gew.-%, weiter bevorzugt 2,5 - 13,0 Gew.-%, weiter bevorzugt 2,6 - 12,5 Gew.-%, weiter bevorzugt 2,7 - 12,7 Gew.-%, weiter bevorzugt 2,8 - 12,4 Gew.-%, weiter bevorzugt 2,9 - 12,0 Gew.-%, weiter bevorzugt 3,0 - 12,2 Gew.-%, weiter bevorzugt 3,1- 12,0 Gew.-%, weiter bevorzugt 3,2 - 11,5 Gew.-%, weiter bevorzugt 3,3 - 11,5 Gew.-%, weiter bevorzugt 3,4 - 11,0 Gew.-% und weiter bevorzugt 0,35 - 11,0 Gew.-% Dysprosium aufweist.

Bevorzugt liegt die Masse an Neodym im Bereich von 0,0 - 8,0 Gew.-%, weiter bevorzugt 0,1 - 5,0 Gew.-%, noch weiter bevorzugt 0,2 - 4,0 Gew.-%, noch weiter bevorzugt 0,3 - 3,5 Gew.-%, und insbesondere bevorzugt von 0,5 - 3,2 Gew.-%.

Zusammen mit Neodym (Nd) oder anstelle von Nd kann auch Europium (Eu) in der Legierung in Anteilen von 0,0 - 8,0 Gew.-%, weiter bevorzugt 0,1 - 5,0 Gew.-%, noch weiter bevorzugt 0,2 - 4,0 Gew.-%, noch weiter bevorzugt 0,3 - 3,5 Gew.-%, und insbesondere bevorzugt von 0,5 - 3,2 Gew.-%.

Ferner ist bevorzugt, wenn der gemeinsame Anteil an Nd und Eu in der Legierung von 0,01 - 8,0 Gew.-%, weiter bevorzugt 0,1 - 5,0 Gew.-%, noch weiter bevorzugt 0,2 - 4,0 Gew.-%, und insbesondere bevorzugt von 0,5 - 3,2 Gew.-% beträgt.

Die Summe der Gewichtsanteile an Dysprosium und Neodym liegen bevorzugt im Bereich von 1,1 - 18,0 Gew.-%, weiter bevorzugt zwischen 1,6 - 15,5 Gew.-%, noch weiter bevorzugt , und insbesondere bevorzugt von 2,1 - 13,0 Gew.-%.

Ferner ist bevorzugt, wenn die Legierung zudem 0,0 - 4,0 Gew.-%, weiter bevorzugt 0,0 - 3,0 Gew.-%, noch weiter bevorzugt 0,0 - 2,0 Gew.-% noch weiter bevorzugt 0,1 - 1,5 Gew.-%, und insbesondere bevorzugt von 0,2 - 1,3 Gew.-% Zink (Zn) aufweist.

Zusätzlich zu den zuvor genannten Bestandteilen kann eine Magnesiumlegierung aus welcher das Grundgerüst des erfindungsgemäßen Stents hergestellt wurde auch 0,0 Gew.-% - 5,0 Gew.-%, bevorzugt 0,1 Gew.-% - 4,0 Gew.-%, weiter bevorzugt 0,2 Gew.-% - 3,0 Gew.-% und besonders bevorzugt insgesamt nicht mehr als 2,5 Gew.-% andere Metalle, Metallsalze, Nichtmetalle, Kohlenstoff, Schwefel, Silizium, Stickstoff, Sauerstoff und/oder Wasserstoff enthalten.

Bevorzugt ist zudem, dass die Elemente Beryllium, Aluminium und Mangan jeweils unter 300 ppm, bevorzugt unter 200 ppm und weiter bevorzugt unter 150 ppm in den Magnesiumlegierungen, aus welcher das Grundgerüst des erfindungsgemäßen Stents hergestellt wurde, vorhanden sind.

In der Maximalmenge von 2,5 Gew.-% Verunreinigungen sind die weiteren Metalle oder Nichtmetalle wie z.B. Silizium, Kohlenstoff, Sauerstoff, Stickstoff, Wasserstoff oder Schwefel enthalten, auch wenn diese zusätzlich explizit aufgeführt sind. Der Begriff "Verunreinigungen" wie hierin verwendet bezeichnet alle Legierungsbestandteile außer Magnesium, Dysprosium, Neodym, Europium, Zink und Zirkonium ungeachtet, ob diese explizit aufgeführt sind oder nicht.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,5 Gew.-% | Magnesium |
| 0,1 Gew.-% - 13,0 Gew.-% | Dysprosium |

(fortgesetzt)

| | |
|---|---|
| 0,2 Gew.-% - 4,0 Gew.-% | Neodym |
| 0,2 Gew.-% - 3,0 Gew.-% | Zink |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Neodym |
| 1 Gew.-% | Zink |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80 Gew.-% - 99,65 Gew.-% | Magnesium |
| 0,1 Gew.-% - 14,0 Gew.-% | Dysprosium |
| 0,2 Gew.-% - 4,0 Gew.-% | Neodym |
| 0,05 Gew.-% - 2,0 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere, bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Neodym |
| 1 Gew.-% | Zirkonium, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,45 Gew.-% | Magnesium |
| 0,1 Gew.-% - 13,0 Gew.-% | Dysprosium |
| 0,2 Gew.-% - 4,0 Gew.-% | Neodym |
| 0,2 Gew.-% - 2,0 Gew.-% | Zink |
| 0,05 Gew.-% - 1,0 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87 Gew.-% | Magnesium |

(fortgesetzt)

| | |
|---|---|
| 10 Gew.-% | Dysprosium |
| 1 Gew.-% | Neodym |
| 1 Gew.-% | Zink |
| 1 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,4 Gew.-% | Magnesium |
| 0,1 Gew.-% - 13,0 Gew.-% | Dysprosium |
| 0,2 Gew.-% - 3,5 Gew.-% | Neodym |
| 0,2 Gew.-% - 1,0 Gew.-% | Zink |
| 0,1 Gew.-% - 2,5 Gew.-% | Verunreinigungen wie z.B. andere Metalle, Metallsalze und Nichtmetalle, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,6 Gew.-% | Magnesium |
| 0,1 Gew.-% - 13,0 Gew.-% | Dysprosium |
| 0,1 Gew.-% - 3,0 Gew.-% | Neodym |
| 0,1 Gew.-% - 2,0 Gew.-% | Zink |
| 0,1 Gew.-% - 2,0 Gew.-% | Verunreinigungen wie z.B. andere Metalle, Metallsalze und Nichtmetalle, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,55 Gew.-% - 99,6 Gew.-% | Magnesium |
| 0,1 Gew.-% - 13,0 Gew.-% | Dysprosium |
| 0,1 Gew.-% - 3,2 Gew.-% | Neodym |
| 0,1 Gew.-%- 0,75 Gew.-% | Zirkonium |
| 0,1 Gew.-% - 2,5 Gew.-% | Verunreinigungen wie z.B. andere Metalle, Metallsalze und Nichtmetalle, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,55 Gew.-% - 99,5 Gew.-% | Magnesium |
| 0,1 Gew.-% - 11,0 Gew.-% | Dysprosium |
| 0,1 Gew.-% - 3,2 Gew.-% | Neodym |
| 0,1 Gew.-% - 2,0 Gew.-% | Zink |
| 0,1 Gew.-%- 0,75 Gew.-% | Zirkonium |
| 0,1 Gew.-% - 2,5 Gew.-% | Verunreinigungen wie z.B. andere Metalle, Metallsalze und Nichtmetalle, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,25 Gew.-% - 94,6 Gew.-% | Magnesium |
| 5,0 Gew.-% - 12,5 Gew.-% | Dysprosium |
| 0,1 Gew.-% - 2,0 Gew.-% | Europium |
| 0,1 Gew.-% - 2,0 Gew.-% | Zink |
| 0,1 Gew.-% - 0,75 Gew.-% | Zirkonium |
| 0,1 Gew.-% - 2,5 Gew.-% | Verunreinigungen wie z.B. andere Metalle, Metallsalze und Nichtmetalle, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,4 Gew.-% | Magnesium |
| 0,1 Gew.-% - 11,0 Gew.-% | Dysprosium |
| 0,1 Gew.-% - 2,0 Gew.-% | Europium |
| 0,1 Gew.-% - 3,0 Gew.-% | Neodym |
| 0,1 Gew.-% - 2,0 Gew.-% | Zink |
| 0,1 Gew.-% - 1,0 Gew.-% | Zirkonium |
| 0,1 Gew.-% - 1,0 Gew.-% | Verunreinigungen wie z.B. andere Metalle, Metallsalze und Nichtmetalle, |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,65 Gew.-% | Magnesium |
| 0,1 Gew.-% - 15,0 Gew.-% | Dysprosium |
| 0,2 Gew.-% - 3,0 Gew.-% | Europium |
| 0,05 Gew.-% - 2,0 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Europium |
| 1 Gew.-% | Zirkonium |

wobei der resorbierbare Stent von einer anorganischen biologisch abbaubaren Beschichtung und einer organischen Beschichtung umgeben ist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 94,75 Gew.-% | Magnesium |

(fortgesetzt)

| | |
|---|---|
| 0,1 Gew.-% - 15,0 Gew.-% | Dysprosium |
| 0,2 Gew.-% - 4,0 Gew.-% | Europium |
| 0,05 Gew.-% - 4,0 Gew.-% | Zink |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Europium |
| 1 Gew.-% | Zink |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,6 Gew.-% | Magnesium |
| 0,1 Gew.-% - 14,0 Gew.-% | Dysprosium |
| 0,2 Gew.-% - 2,0 Gew.-% | Europium |
| 0,05 Gew.-% - 2,0 Gew.-% | Zink |
| 0,05 Gew.-% - 2,0 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87 Gew.-% | Magnesium |
| 10 Gew.-% | Dysprosium |
| 1 Gew.-% | Europium |
| 1 Gew.-% | Zink |
| 1 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen

besteht:

| | |
|---|---|
| 86,8 Gew.-% | Magnesium |
| 10,0 Gew.-% | Dysprosium |
| 1,0 Gew.-% | Europium |
| 1,0 Gew.-% | Zink |
| 0,2 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87,8 Gew.-% | Magnesium |
| 10,0 Gew.-% | Dysprosium |
| 1,0 Gew.-% | Neodym |
| 1,0 Gew.-% | Europium |
| 1,0 Gew.-% | Zink |
| 0,2 Gew.-% | Zirkonium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Alle in dieser Offenbarung angegebenen Gew.-% beziehen sich auf das Gesamtgewicht der entsprechenden Legierung. Somit gilt bei allen hierin aufgeführten Zusammensetzungen, dass die Summe aller Bestandteile insgesamt 100,00 Gew.-% ergeben muss. Das heißt, dass nach Addition aller aufgeführten Komponenten der Magnesiumlegierung die Differenz zu 100 Gew.-% aus Magnesium als Hauptbestandteil besteht.

Ferner umfasst die vorliegende Erfindung bevorzugt Stents, deren Grundgerüst aus biologisch abbaubaren Magnesiumlegierungen besteht, die neben mindestens 80 Gew.-% Magnesium, Dysprosium, Neodym, Europium, Zink, Zirkonium und nicht vermeidbaren, herstellungsbedingten Verunreinigungen als weitere Bestandteile Yttrium (Y), Gadolinium (Gd), Calcium (Ca), Mangan (Mn), Cer (Ce), Scandium (Sc), Indium (In), Lithium (Li) oder Erbium (Er) umfassen. Das heißt, es ist bevorzugt, wenn die Bestandteile der Legierung, neben der Basis Magnesium, ausgewählt werden aus der folgenden Gruppe bestehend oder umfassend aus: Dysprosium, Neodym, Europium, Zink, Zirkonium, Yttrium, Gadolinium, Erbium, Calcium, Mangan, Cer, Scandium, Indium, Lithium und nicht vermeidbaren, herstellungsbedingten Verunreinigungen. In einigen Ausführungsformen der vorliegenden Erfindung sind die Magnesiumlegierungen der Stents frei von Yttrium.

Folglich betrifft eine weitere bevorzugte Ausführungsform der Erfindung Stents bestehend aus einer biologisch abbaubaren Magnesiumlegierung, die die folgenden Bestandteile umfasst oder aus den folgenden Bestandteilen besteht:

| | |
|---|---|
| 93,0 Gew.-% - 94,0 Gew.-% | Magnesium |
| 0,3 Gew.-% - 0,4 Gew.-% | Dysprosium |
| 2,0 Gew.-% - 2,1 Gew.-% | Neodym |
| 0,3 Gew.-% - 0,5 Gew.-% | Gadolinium |
| 0,3 Gew.-% - 0,4 Gew.-% | Zirkonium |
| 3,4 Gew.-% - 3,8 Gew.-% | Yttrium |
| 0,0 Gew.-% - 0,02 Gew.-% | Erbium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 92,0 Gew.-% - 93,0 Gew.-% | Magnesium |

(fortgesetzt)

| | |
|---|---|
| 0,9 Gew.-% - 1,1 Gew.-% | Dysprosium und Gadolinium zusammen |
| 2,1 Gew.-% - 2,3 Gew.-% | Neodym |
| 0,4 Gew.-% - 0,6 Gew.-% | Zirkonium |
| 3,9 Gew.-% - 4,3 Gew.-% | Yttrium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 91,0 Gew.-% - 92,0 Gew.-% | Magnesium |
| 0,7 Gew.-% - 0,8 Gew.-% | Dysprosium |
| 0,6 Gew.-% - 0,8 Gew.-% | Gadolinium |
| 1,9 Gew.-% - 2,1 Gew.-% | Neodym |
| 0,6 Gew.-% - 0,8 Gew.-% | Zirkonium |
| 3,9 Gew.-% - 4,2 Gew.-% | Yttrium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren, Yttrium freien Magnesiumlegierung, die die folgenden Bestandteile umfasst oder aus den folgenden Bestandteilen besteht:

| | |
|---|---|
| 93,7 Gew.-% - 96,2 Gew.-% | Magnesium |
| 1,0 Gew.-% - 1,5 Gew.-% | Gadolinium |
| 2,0 Gew.-% - 3,1 Gew.-% | Neodym |
| 0,5 Gew.-% - 0,7 Gew.-% | Zirkonium |
| 0,1 Gew.-% - 0,5 Gew.-% | Calcium |
| 0,2 Gew.-% - 0,5 Gew.-% | Zink |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 96,9 Gew.-% | Magnesium |
| 2,5 Gew.-% | Neodym |
| 0,4 Gew.-% | Zirkonium |
| 0,2 Gew.-% | Zink |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 97,45 Gew.-% | Magnesium |
| 0,75 Gew.-% | Neodym |
| 1,80 Gew.-% | Mangan |

wobei der Stent eine anorganische Beschichtung umfassend Magnesiumfluorid und eine organische Beschichtung

aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 97,45 Gew.-% | Magnesium |
| 0,75 Gew.-% | Cer |
| 1,80 Gew.-% | Mangan |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 90,0 Gew.-% | Magnesium |
| 3,0 Gew.-% | Gadolinium |
| 2,4 Gew.-% | Yttrium |
| 0,4 Gew.-% | Zirkonium |
| 4,2 Gew.-% | Scandium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 90,0 Gew.-% | Magnesium |
| 3,0 Gew.-% | Neodym |
| 2,4 Gew.-% | Yttrium |
| 0,4 Gew.-% | Zirkonium |
| 5,2 Gew.-% | Scandium |
| 2,0 Gew.-% | Indium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 96,0 Gew.-% | Magnesium |
| 4,0 Gew.-% | Lithium |

wobei der Stent eine anorganische Beschichtung umfassend Magnesiumfluorid und eine organische Beschichtung aufweist, und die organische Beschichtung eine oder mehrere hierin beschriebene Substanzen umfasst.

Es handelt sich bei dem erfindungsgemäßen, resorbierbaren Stent bevorzugt um einen Stent für Blutgefäße, Harnwege, Atemwege, Gallenwege oder den Verdauungstrakt. Unter diesen Stents sind wiederum die Stents für Blutgefäße oder allgemeiner für das Herz-Kreislaufsystem bevorzugt. Die hier offenbarten Magnesiumlegierungen sind so ausgewählt, dass sie insbesondere zur Herstellung von resorbierbaren oder degradierbaren Endoprothesen und insbesondere. Stents geeignet sind Des Weiteren umfasst die vorliegende Erfindung daher einen resorbierbaren Stent bestehend aus einer der hier offenbarten Magnesiumlegierungen wobei der Stent eine anorganische Beschichtung umfassend Magnesiumfluorid und eine organische Beschichtung aufweist. Die anorganische Beschichtung der erfindungsgemäßen Stents ist eine Beschichtung, welche auf die Oberfläche des Stents aufgebracht wurde, umfassend mindestens 50%, bevorzugt mindestens 80% und noch weiter bevorzugt mindestens 90% Magnesiumfluorid. Die anorganische Beschichtung kann zudem ebenfalls biodegradierbar sein. Auch die organische Beschichtung kann biodegradierbar sein.

Die Begriffe "bioresorbierbar" oder "biodegradierbar" wie hierin verwendet bedeuten, dass das Implantat sich

über eine gewisse Zeit in einem menschlichen oder tierischen Organismus langsam auflöst und irgendwann nur noch dessen Abbauprodukte im Körper in gelöster Form vorliegen. Zu diesem Zeitpunkt sind feste Bestandteile oder Fragmente des Implantats nicht mehr vorhanden. Die Abbauprodukte sollten physiologisch weitgehend unbedenklich sein und zu Ionen oder Molekülen führen, welche im Organismus sowieso vorhanden oder vom Organismus zu unbedenklichen Stoffen abgebaut oder ausgeschieden werden können.

Das bedeutet, ein Stent ist biologisch abbaubar bzw. biodegradierbar, wenn der Stent eine wesentliche Verringerung des Gewichts aufzeigt oder eine chemische Umwandlung stattfindet, nachdem er in einen Patienten eingeführt wurde. Gewichtsreduzierung kann auftreten, z. B. durch Auflösung des Materials. Dabei bevorzugt stattfindende chemische Umwandlungen sind Oxidation/Reduktion, Hydrolyse, Substitution, und/oder Addition. Die Erosion kann das Ergebnis einer chemischen Wechselwirkung des Stents mit dem Körper (dem Körper selbst oder Körperflüssigkeiten) sein. Vorzugsweise erfolgt die Erosion in einem gewünschten Ausmaß in einem Zeitrahmen, der sicherstellt, dass die wesentlichen Funktionen des Stents, wie die Unterstützung des Lumens, solange aufrechterhalten werden, wie benötigt. Stents aus den hier offenbarten biologisch abbaubaren Magnesiumlegierungen mit den anorganischen Beschichtungen werden innerhalb eines Zeitraumes von 8 bis 50 Wochen, vorzugsweise 10 bis 30 Wochen unter physiologischen Bedingungen resorbiert.

Unter Stents werden hierbei gitterförmige oder netzförmige Endoprothesen verstanden, die in ein Hohlorgan oder einen Körperhohlraum eingesetzt werden, um diesen offen zu halten. Das Grundgerüst eines Stents, womit hierin die metallischen Streben ohne Beschichtung bezeichnet werden, bildet kein massives Rohr, sondern ein Gittergeflecht. Betrachtet man beispielsweise das Grundgerüst eines vaskulären Stents, so wird dieses aus einem massiven Rohr z.B. mittels Laser geschnitten, so dass sich einzelne, möglichst dünne Streben ergeben, welche untereinander verbunden sind. Die Anordnung und Ausformung der Streben und Knotenpunkte wird als Stentdesign bezeichnet. Im Sinne der vorliegenden Erfindung können als erfindungsgemäßer Magnesium-Stent alle üblichen Stentgeometrien verwendet werden.

Zudem wird ein Verfahren zur Herstellung von resorbierbaren Stents offenbart, umfassend die folgenden Schritte:

a) Bereitstellung einer bioresorbierbaren Magnesiumlegierung wie hierin offenbart,
b) Herstellung eines Drahtes oder Stabs oder Rohres aus der Legierung erhalten gemäß Schritt a) durch Strangpressen,
c) Herstellung eines Rohres aus dem Draht oder Stab erhalten gemäß Schritt b),
d) Schneiden von Stents aus dem Rohr erhalten gemäß Schritt b) oder c) und
e) Aufbringen einer anorganischen Beschichtung umfassend Magnesiumfluorid auf zumindest einem Teil der Oberfläche des Stents erhalten gemäß Schritt d),
f) Aufbringen einer organischen Beschichtung auf zumindest einem Teil der gemäß Schritt e) aufgebrachten anorganischen Beschichtung,

wobei die organische Beschichtung eine oder mehrere Substanzen der folgenden Gruppe umfasst:
Polyvinylpyrrolidon, Glycerin, Polyhydroxyethyl-methacrylate, Polypropylenglycol, Polyvinylalkohol Polygluconat, Polyaminosäuren, Polyphosphatester, Polyvalerolactone, Poly-ε-Decalactone, Polyglycolsäure, Poly-L-Lactid, Poly D,L-Lactid, sowie Blends wie Poly(L-Lactid-co-glycolid), Poly(D,L-lactid-co-glycolid), Poly(L-Lactid-co-D,L-Lactid), Poly-ε-caprolacton, Polyhydroxybuttersäure, Polyhydroxyvalerate, Polyhydroxybutyrate-co-valerate, Polymaleinsäureanhydride, Polyhydroxymethacrylate, Fibrin, Polycyanoacrylate, Polycaprolactondimethylacrylate, Polycaprolactonbutylacrylate, Polyetherestermulti-blockpolymere aus PEG und Polybutylenterephtalat, Polypivotolactone, Polyglycolsäuretrimethylcarbonate Polycaprolactonglycolide, Poly(DTH-Iminocarbonat), Poly(DTE-co-DT-carbonat), Poly(Bisphenol A-iminocarbonat), Polyorthoester, Polytrimethylcarbonate Polyiminocarbonate, Poly(N-vinyl)-Pyrrolidon, Polyesteramide, glycolierte Polyester, Polyphosphoester, Polyphosphazene, Poly[p-carboxyphenoxy)propan], Polyethylenoxid-propylenoxid, Polyalkenoxalate, Lipide, Wachse, Öle, mehrfach ungesättigte Fettsäuren, Eicosapentaensäure, Timnodonsäure, Docosahexaensäure, Arachidonsäure, Linolsäure, α-Linolensäure, γ-Linolensäure, Carrageenane, Fibrinogen, Agar-Agar, Stärke, Zein, Polyhydroxyalkanoate, Pectinsäure, Actinsäure, Carboxymethylsulfat, Hyaluronsäure, Heparansulfate und seine Derivate, Heparine, Dextran, β-Cyclodextrine, Gummi arabicum, Guar, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polyvinylketone, Polyvinylhalogenide, Polyvinyliden-halogenide, Polyvinylether, Polyisobutylene, Polyvinylaromaten, Polyvinylester, Polyoxymethylene, Polytetramethylenoxid, Polyethylen, Polypropylen, Polytetrafluorethylen, Polyolefin-Elastomere, Carboxymethylchitosane, Polyetheretherketone, Polyethylenterephtalat, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Polysulfone, Epoxyharze, ABS-Harze, EPDM-Gummis, Celluloseether, Cellulosetriacetate, Schellack, Poly-para-Xylylene (Parylene) wie Parylen N, Parylen C.

Zudem werden hierin resorbierbare Stents erhalten nach dem vorangegangenen Verfahren beschrieben. In

Schritt d) wird bevorzugt ein Laser verwendet, um die Stents aus dem Rohr erhalten gemäß Schritt b) oder c) zu schneiden. Zur Herstellung eines hierin beschriebenen resorbierbaren Stents kann nach Schritt a) zunächst ein Draht oder ein Stab aus der Legierung hergestellt werden, bevor in Schritt c) ein Rohr aus dem Draht oder dem Stab erhalten wird. Daher wird ein Verfahren zur Herstellung von resorbierbaren Stents beschrieben, umfassend folgende Schritte:

a) Bereitstellung einer bioresorbierbaren Magnesiumlegierung wie hierin offenbart,
b) Herstellung eines Drahtes oder Stabs aus der Legierung erhalten gemäß Schritt a) durch Strangpressen,
c) Herstellung eines Rohres aus dem Draht oder Stab erhalten gemäß Schritt b),
d) Schneiden von Stents aus dem Rohr erhalten gemäß Schritt c) und
e) Aufbringen einer anorganischen Beschichtung auf zumindest einem Teil der Oberfläche des Stents erhalten gemäß Schritt d),
f) Aufbringen einer organischen Beschichtung auf zumindest einem Teil der gemäß Schritt e) aufgebrachten anorganischen Beschichtung,

wobei die organische Beschichtung eine oder mehrere der hierin beschriebenen Substanzen umfasst.

Die Offenbarung umfasst auch resorbierbare Stents erhalten nach dem vorgenannten Verfahren.

Weiterhin kann das Rohr bereits in Schritt b) erhalten werden, so dass nicht zuerst ein Stab oder ein Draht hergestellt werden muss. Im Anschluss wird dann der Stent aus dem Rohr erhalten gemäß Schritt b) ausgeschnitten. Somit ist hierin ein Verfahren zur Herstellung von resorbierbaren Stents beschrieben, umfassend folgende Schritte:

a) Bereitstellung einer bioresorbierbaren Magnesiumlegierung wie hierin offenbart,
b) Herstellung eines Rohres aus der Legierung erhalten gemäß Schritt a) durch Strangpressen,
c) Schneiden von Stents aus dem Rohr erhalten gemäß Schritt b) und
d) Aufbringen einer anorganischen Beschichtung auf zumindest einem Teil der Oberfläche des Stents erhalten gemäß Schritt c),
e) Aufbringen einer organischen Beschichtung auf zumindest einem Teil der gemäß Schritt d) aufgebrachten anorganischen Beschichtung,

wobei die organische Beschichtung eine oder mehrere der hierin beschriebenen Substanzen umfasst.

Die Offenbarung umfasst auch resorbierbare Stents erhalten nach dem vorgenannten Verfahren.

Um eine hierin beschriebene Magnesiumlegierung zu erhalten, können vor Schritt a) noch weitere Schritte durchgeführt werden. Bei diesen Schritten werden in einem geschlichteten Stahltiegel bei einer Schmelzbadtemperatur von 660-740 °C durch schrittweise zugeben der Legierungselemente in Form von reinen Elementen oder als Masterlegierungen in einem geschlichteten Stahltiegel erschmolzen. Alle Schmelzoperationen finden unter Schutzgas statt. Nach der Zugabe der Legierungselemente wird die Schmelze mechanisch gerührt. In einem nächsten Schritt wird diese Schmelze in eine dünnwandige Kokille überführt, die auf eine Temperatur von 600 °C vorgeheizt wurde. In einem letzten Schritt wird die Kokille in ein Wasserbad eingetaucht, das eine Temperatur von 15-20 °C aufweist.

Hierin werden auch Magnesiumlegierungen beschrieben, die vor dem Schritt a) in einem Vakuumofen oder einem Schutzgasofen aufgeschmolzen werden. In der Regel erfolgt das Aufschmelzen in einem Schmelz- oder Gießofen. Beim Aufschmelzen in einem Vakuumofen wird die Legierung unter einem verminderten Druck durchgeführt. In einem Schutzgasofen wird ein Schutzgas verwendet, um während des Aufschmelzens der Legierung, diese vor ungewünschten Gasen zu schützen.

In einer tierexperimentellen Studie (s. Beispiel 7) zur Wirksamkeit und Unbedenklichkeit von Stents aus den hierin beschriebenen Magnesiumlegierungen konnte gezeigt werden, dass die erfindungsgemäßen Stents aus den hier offenbarten Magnesiumlegierungen sich ohne Probleme auf einen Ballon crimpen lassen. Die Implantation der Stents erfolgte ohne das Auftreten von bekannten Komplikationen, wie Stent-Malapposition, Thrombosen oder Dissektionen. Bereits nach 4 Wochen war eine vollständige Reendothelialisierung der gestenteten Gefäßabschnitte zu beobachten. Dies spricht dafür, dass keine übermäßigen Endzündungsreaktionen stattfanden und die erfindungsgemäßen Magnesiumlegierungen keine Unverträglichkeitsreaktionen im Gewebe des Gefäßes hervorgerufen haben. Die Restenoserate lag im Bereich der Werte von herkömmlichen Metallstents (BMS) im Stand der Technik, bzw. im Bereich der "schlechteren" Wirkstoff-freisetzenden Stents (DES) (vgl. Abbildungen zum Vortrag R.A. Costa; gehalten i.R. des Euro-PCR, Paris, im Mai 2011).

Das innere metallische Gerüst des erfindungsgemäßen Stents aus einer der hierin beschriebenen, biologisch abbaubaren Magnesiumlegierungen, hat zudem bevorzugt die Eigenschaft, dass es sich schneller auflöst, als die anorganische Beschichtung und/oder organische Beschichtung, d.h. die innere Struktur der Gefäßstütze wird unter physiologischen Bedingungen schneller abgebaut als die anorganische Beschichtung und/oder organische Beschichtung. Bei der Verwendung verschiedener organischer Verbindungen bzw. verschiedener Mischungen von organischen Verbindungen für eine organische Beschichtung auf einem Stent besteht zudem die Möglichkeit zeitlich unterschiedlich

schnell abbaubare oder biostabile organische Verbindungen zu verwenden.

Die **organische Beschichtung** für alle hierin offenbarten Magnesiumstents kann eine oder mehrere biostabile oder biodegradierbare Substanzen ausgewählt aus der Gruppe umfassen oder daraus bestehend: Polyvinylpyrrolidon, Glycerin, Polyhydroxyethyl-methacrylate, Polypropylenglycol, Polyvinylalkohol Polydioxanon, Polycaprolacton, Polygluconat, Polymilchsäure-Polyethylenoxid-Copolymer, modifizierte Cellulose, Poly(hydroxybutyrat), Polyaminosäuren, Polyphosphatester, Polyvalerolactone, Poly-ε-Decalactone, Polyglycolsäure, Polylactide, bevorzugt Poly-L-Lactid, Poly D,L-Lactid, und Copolymere sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)] (PTMC), Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-b-Maleinsäure, Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate, Polycaprolactonglycolide, Poly(g-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Wachse, Öle, mehrfach ungesättigte Fettsäuren, Eicosapentaensäure, Timnodonsäure, Docosahexaensäure, Arachidonsäure, Linolsäure, α-Linolensäure, γ-Linolensäure, Carrageenane, Fibrinogen, Agar-Agar, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, β-Cyclodextrine, Copolymere mit PEG und Polypropylenglycol, Gummi arabicum, Guar, Gelatine, Collagen, Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetatcopolymere, Polysulfone, Epoxyharze, ABS-Harze, EPDM-Gummis, Silicone wie Polysiloxane, Polydimethylsiloxane, Polyvinylhalogene, Celluloseether, Cellulosetriacetate, Schellack, Poly-para-Xylylene (Parylene) wie Parylen N, Parylen C und/oder Parylen D, und Copolymere und/oder Gemische der vorgenannten Polymere.

Bevorzugte Polymere für die äußere organische Beschichtung sind:

Polyvinylpyrrolidon, Polyhydroxyethyl-methacrylate, Polypropylenglycol, Polyvinylalkohol Polydioxanon, Polycaprolacton, Polymilchsäure-Polyethylenoxid-Copolymer, Poly(hydroxybutyrat), Polyvalerolactone, Poly-ε-Decalactone, Polyglycolsäure, Polylactide, bevorzugt Poly-L-Lactid, Poly D,L-Lactid, und Copolymere sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)] (PTMC), Polyglycolide, Copolymere der Polylactide und Polyglycolide, Poly-ε-caprolacton, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polymaleinsäureanhydride, Polyhydroxymethacrylate, Fibrin, Polycaprolactondimethylacrylate, Polycaprolactonbutylacrylate, Polycaprolactonglycolide, Poly(g-ethylglutamat), Polyorthoester, Polyvinylalkohole, Polyesteramide, Polyurethane, Polyacrylate, Polymethylmethacrylat, Polybutylmethacrylat, Polyacrylamid, Polyamide, Polyetheramide, Polyethylenamin, Polyimide, Polycarbonate, Polycarbourethane, Polyvinylketone, Polyvinylether, Polyisobutylene, Polyvinylaromaten, Polyvinylester, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, Fluorosilicone, Polyaryletheretherketone, Polyetheretherketone, Polyethylenterephtalat, Polyvalerate, Polysulfone, Polysiloxane, Polydimethylsiloxane, Poly-para-Xylylene (Parylene) wie Parylen N, Parylen C und/oder Parylen D, und Copolymere und/oder Gemische der vorgenannten Polymere.

Die organische Beschichtung der erfindungsgemäßen Stents ist metallfrei, d.h. sie enthält keine Metall-haltigen Verbindungen mit beispielsweise Natrium, Aluminium Magnesium, Eisen, Zirkon oder Titan. Die organische Beschichtung enthält vorzugsweise keine metallorganischen Verbindungen, Metallalkoxide oder polymere Metallalkoxide. Insbesondere besteht die organische Beschichtung nicht aus Titanethylenglykol, Titanpropylenglykol, Zirkonethylenglykol, Zirkonpropylenglykol, Hafniumethylenglykol, Hafniumpropylenglykol oder Polyaluminiumethylenglykol. Als metallorganische Verbindungen werden Verbindungen mit einer Metall-Kohlenstoff-Bindung bezeichnet.

Somit betrifft die vorliegende Erfindung einen Stent aus einer biologisch abbaubaren Magnesiumlegierung mit

einer anorganischen Beschichtung umfassend Magnesiumfluorid und mit einer organischen Beschichtung, wobei die Magnesiumlegierung mindestens 80 Gew.-% Magnesium und die organische Beschichtung keine metallorganischen Verbindungen, Metallalkoxide oder polymere Metallalkoxide enthält.

Parylen ist zumeist die Bezeichnung für vollständig lineare, teilkristalline, unvernetzte aromatische Polymere. Die verschiedenen Polymere besitzen unterschiedliche Eigenschaften und lassen sich in vier Grundtypen einteilen, nämlich das Parylen C, Parylen **D,** Parylen N und Parylen **F,** deren Struktur im Folgenden gezeigt ist:

**Parylen C**

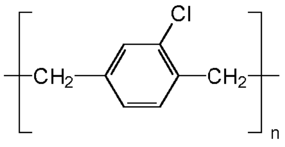

**Parylen D**

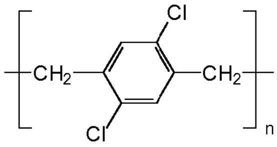

**Parylen N**

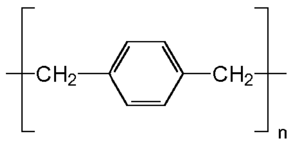

**Parylen F**

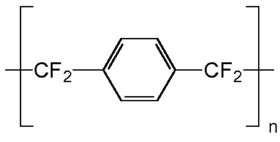

Das einfachste Monomer der Parylengruppe ist Parylen N (Poly-para-Xylylen). Ferner existieren die zwei chlorierten Polymere Parylen C (Chlorpoly-para-Xylylen) und Parylen D (Di-chloro-poly-para-Xylylen). Bei Parylen F (Poly(Tetrafluor-para-Xylylen)) sind die Methyleneinheiten fluoriert.

Parylen C besitzt den niedrigsten Schmelzpunkt der vorgenannten Parylene von nur 290 °C, zeichnet sich durch gute mechanische Eigenschaften und Korrosionsbeständigkeit gegenüber korrosiven Gasen sowie sehr geringe Permeabilität gegenüber Feuchtigkeit aus. Parylen C ist ein biokompatibles Polymer und somit für den Einsatz in physiologischer Umgebung geeignet.

In einer bevorzugten Ausführungsform der vorliegenden Erfindung umfasst die organische Beschichtung eine oder mehrere Substanzen der folgenden Gruppe: Poly-ε-Caprolacton (PCL), Poly-L-Lactid-co-Glycolid (PLGA), Poly-L-Lactid und Parylene.

Daher betrifft die vorliegende Erfindung besonders bevorzugt Stents bestehend aus einer biologisch abbaubaren Magnesiumlegierung, die bezogen auf das Gesamtgewicht der Legierung folgende Bestandteile enthält:

| | |
|---|---|
| 0,1 Gew.-% - 15,5 Gew.-% | Dysprosium |
| 0,01 Gew.-% - 1,5 Gew.-% | Neodym und/oder Europium |
| 0,0 Gew.-% - 2,0 Gew.-% | Zink |
| 0,0 Gew.-% - 1,0 Gew.-% | Zirkonium |
| mindestens 80,0 Gew.-% | umfasst. Magnesium |

Auf die Stents der vorgenannten oder der hierin offenbarten Magnesiumlegierungen wird eine Magnesiumfluoridschicht ($MgF_2$) mit einer Schichtdicke von 0,01 μm bis 100 μm und auf diese Magnesiumfluoridschicht wird eine organische Beschichtung aus Polyvinylpyrrolidon, Polyhydroxyethyl-methacrylate, Polypropylenglycol, Polyvinylalkohol Polydioxanon, Polycaprolacton, Polymilchsäure-Polyethylenoxid-Copolymer, Poly(hydroxybutyrat), Polyvalerolactone, Poly-ε-Decalactone, Polyglycolsäure, Polylactide, bevorzugt Poly-L-Lactid, Poly D,L-Lactid, und Copolymere sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)] (PTMC), Polyglycolide, Copolymere der Polylactide und Polyglycolide, Poly-ε-caprolacton, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polymaleinsäureanhydride, Polyhydroxymethacrylate, Fibrin, Polycaprolactondimethylacrylate, Polycaprolactonbutylacrylate, Polycaprolactonglycolide, Poly(g-ethylglutamat), Polyorthoester, Polyvinylalkohole, Polyesteramide, Polyurethane, Polyacrylate, Polymethylmethacrylat, Polybutylmethacrylat, Polyacrylamid, Polyamide, Polyetheramide, Polyethylenamin, Polyimide, Polycarbonate, Polycarbourethane, Polyvinylketone, Polyvinylether, Polyisobutylene, Polyvinylaromaten, Polyvinylester, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, Fluorosilicone, Polyaryletheretherketone, Polyetheretherketone, Polyethylenterephtalat, Polyvalerate, Polysulfone, Polysiloxane, Polydimethylsiloxane, Poly-para-Xylylene (Parylene) wie Parylen N, Parylen C und/oder Parylen D, und Copolymere und/oder Gemische der vorgenannten Polymere mit einer Schichtdicke von 0,01 μm bis 100 μm aufgetragen.

Ferner bevorzugt sind Stents bestehend aus einer biologisch abbaubaren Magnesiumlegierung, die bezogen auf das Gesamtgewicht der Legierung folgende Bestandteile enthält:

| | |
|---|---|
| 0,1 Gew.-% - 15,5 Gew.-% | Dysprosium |
| 0,01 Gew.-% - 1,5 Gew.-% | Neodym und/oder Europium. |

(fortgesetzt)

| | |
|---|---|
| 0,1 Gew.-% - 2,0 Gew.-% | Zink |
| 0,1 Gew.-% - 1,0 Gew.-% | Zirkonium |
| Rest bis 100,0 Gew.-% | Magnesium, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Insbesondere bevorzugt ist, wenn das innere Gerüst eines erfindungsgemäßen Stents aus Magnesiumlegierungen besteht, die bezogen auf das Gesamtgewicht der Legierung (angegeben in Gew.-%) folgende Bestandteile enthalten:

| | |
|---|---|
| 81,25 Gew.-% - 98,8 Gew.-% | Magnesium |
| 0,1 Gew.-% - 15,0 Gew.-% | Dysprosium |
| 0,5 Gew.-% - 1,5 Gew.-% | Neodym und/oder Europium |
| 0,5 Gew.-% - 1,5 Gew.-% | Zink |
| 0,1 Gew.-% - 0,75 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Die vorliegende Erfindung betrifft zudem bevorzugt Stents aus biologisch abbaubaren Magnesiumlegierungen, die bezogen auf das Gesamtgewicht der Legierung (angegeben in Gew.-%) folgende Bestandteile enthalten:

| | |
|---|---|
| 80,25 Gew.-% - 99,4 Gew.-% | Magnesium |
| 0,3 Gew.-% - 15,0 Gew.-% | Dysprosium |
| 0,1 Gew.-% - 2,0 Gew.-% | Neodym |
| 0,1 Gew.-% - 2,0 Gew.-% | Zink |
| 0,1 Gew.-% - 0,75 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Die Erfindung umfasst ferner Stents bestehend aus Magnesiumlegierungen, die bezogen auf das Gesamtgewicht der Legierung aus folgenden Bestandteilen bestehen:

| | |
|---|---|
| 80,0 Gew.-% - 99,7 Gew.-% | Magnesium |
| 0,3 Gew.-% - 13,0 Gew.-% | Dysprosium |
| 0,0 Gew.-% - 3,0 Gew.-% | Neodym |
| 0,0 Gew.-% - 3,0 Gew.-% | Zink |
| 0,0 Gew.-% - 1,0 Gew.-% | Zirkonium |
| 0,0 Gew.-% - 2,0 Gew.-% | anderen Metallen, Metallsalzen und Nichtmetallen, welche allgemein als Verunreinigungen bezeichnet werden, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,5 Gew.-% | Magnesium |
| 0,1 Gew.-% - 13,0 Gew.-% | Dysprosium |
| 0,2 Gew.-% - 4,0 Gew.-% | Neodym |
| 0,2 Gew.-% - 3,0 Gew.-% | Zink |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Neodym |
| 1 Gew.-% | Zink |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80 Gew.-% - 99,65 Gew.-% | Magnesium |
| 0,1 Gew.-% - 14,0 Gew.-% | Dysprosium |
| 0,2 Gew.-% - 4,0 Gew.-% | Neodym |
| 0,05 Gew.-% - 2,0 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere, bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Neodym |
| 1 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,45 Gew.-% | Magnesium |
| 0,1 Gew.-% - 13,0 Gew.-% | Dysprosium |
| 0,2 Gew.-% - 4,0 Gew.-% | Neodym |
| 0,2 Gew.-% - 2,0 Gew.-% | Zink |
| 0,05 Gew.-% - 1,0 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87 Gew.-% | Magnesium |
| 10 Gew.-% | Dysprosium |
| 1 Gew.-% | Neodym |
| 1 Gew.-% | Zink |
| 1 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine bevorzugte Ausführungsform der Erfindung betrifft Stents bestehend aus einer biologisch abbaubaren Magnesiumlegierung, die die folgenden Bestandteileumfasst oder aus den folgenden Bestandteilen besteht:

| | |
|---|---|
| 80,0 Gew.-% - 99,4 Gew.-% | Magnesium |
| 0,1 Gew.-% - 13,0 Gew.-% | Dysprosium |
| 0,2 Gew.-% - 3,5 Gew.-% | Neodym |
| 0,2 Gew.-% - 1,0 Gew.-% | Zink |
| 0,1 Gew.-% - 2,5 Gew.-% | Verunreinigungen wie z.B. andere Metalle, Metallsalze und Nichtmetalle, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine bevorzugte Ausführungsform der Erfindung betrifft Stents bestehend aus einer biologisch abbaubaren Magnesiumlegierung, die die folgenden Bestandteileumfasst oder aus den folgenden Bestandteilen besteht:

| | |
|---|---|
| 80,0 Gew.-% - 99,6 Gew.-% | Magnesium |
| 0,1 Gew.-% - 13,0 Gew.-% | Dysprosium |
| 0,1 Gew.-% - 3,0 Gew.-% | Neodym |
| 0,1 Gew.-% - 2,0 Gew.-% | Zink |
| 0,1 Gew.-% - 2,0 Gew.-% | Verunreinigungen wie z.B. andere Metalle, Metallsalze und Nichtmetalle, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,55 Gew.-% - 99,6 Gew.-% | Magnesium |
| 0,1 Gew.-% - 13,0 Gew.-% | Dysprosium |
| 0,1 Gew.-% - 3,2 Gew.-% | Neodym |
| 0,1 Gew.-% - 0,75 Gew.-% | Zirkonium |
| 0,1 Gew.-% - 2,5 Gew.-% | Verunreinigungen wie z.B. andere Metalle, Metallsalze und Nichtmetalle, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,55 Gew.-% - 99,5 Gew.-% | Magnesium |
| 0,1 Gew.-% - 11,0 Gew.-% | Dysprosium |
| 0,1 Gew.-% - 3,2 Gew.-% | Neodym |
| 0,1 Gew.-% - 2,0 Gew.-% | Zink |
| 0,1 Gew.-% - 0,75 Gew.-% | Zirkonium |
| 0,1 Gew.-% - 2,5 Gew.-% | Verunreinigungen wie z.B. andere Metalle, Metallsalze und Nichtmetalle, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,25 Gew.-% - 94,6 Gew.-% | Magnesium |
| 5,0 Gew.-% - 12,5 Gew.-% | Dysprosium |
| 0,1 Gew.-% - 2,0 Gew.-% | Europium |
| 0,1 Gew.-% - 2,0 Gew.-% | Zink |
| 0,1 Gew.-% - 0,75 Gew.-% | Zirkonium |
| 0,1 Gew.-% - 2,5 Gew.-% | Verunreinigungen wie z.B. andere Metalle, Metallsalze und Nichtmetalle, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,4 Gew.-% | Magnesium |
| 0,1 Gew.-% - 11,0 Gew.-% | Dysprosium |
| 0,1 Gew.-% - 2,0 Gew.-% | Europium |
| 0,1 Gew.-% - 3,0 Gew.-% | Neodym |
| 0,1 Gew.-% - 2,0 Gew.-% | Zink |
| 0,1 Gew.-% - 1,0 Gew.-% | Zirkonium |
| 0,1 Gew.-% - 1,0 Gew.-% | Verunreinigungen wie z.B. andere Metalle, Metallsalze und Nichtmetalle, |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,65 Gew.-% | Magnesium |
| 0,1 Gew.-% - 15,0 Gew.-% | Dysprosium |
| 0,2 Gew.-% - 3,0 Gew.-% | Europium |
| 0,05 Gew.-% - 2,0 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |

(fortgesetzt)

| | |
|---|---|
| 10 Gew.-% | Dysprosium |
| 1 Gew.-% | Europium |
| 1 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 94,75 Gew.-% | Magnesium |
| 0,1 Gew.-% - 15,0 Gew.-% | Dysprosium |
| 0,2 Gew.-% - 4,0 Gew.-% | Europium |
| 0,05 Gew.-% - 4,0 Gew.-% | Zink |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 88 Gew.-% | Magnesium |
| 10 Gew.-% | Dysprosium |
| 1 Gew.-% | Europium |
| 1 Gew.-% | Zink |

wobei der Stent eine anorganische Beschichtung umfassend oder bestehend aus mindestens einer anorganischen Verbindung aufweist, wobei die mindestens eine anorganische Verbindung Fluoridionen und vorzugsweise Magnesiumfluorid enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 80,0 Gew.-% - 99,6 Gew.-% | Magnesium |
| 0,1 Gew.-% - 14,0 Gew.-% | Dysprosium |
| 0,2 Gew.-% - 2,0 Gew.-% | Europium |
| 0,05 Gew.-% - 2,0 Gew.-% | Zink |
| 0,05 Gew.-% - 2,0 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87 Gew.-% | Magnesium |
| 10 Gew.-% | Dysprosium |
| 1 Gew.-% | Europium |
| 1 Gew.-% | Zink |
| 1 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere besonders bevorzugte Ausführungsform der Erfindung betrifft Stents bestehend aus einer biologisch abbaubaren Magnesiumlegierung, die die folgenden Bestandteile umfasst oder besteht aus:

| | |
|---|---|
| 86,8 Gew.-% | Magnesium |
| 10,0 Gew.-% | Dysprosium |
| 1,0 Gew.-% | Europium |
| 1,0 Gew.-% | Zink |
| 0,2 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere besonders bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 87,8 Gew.-% | Magnesium |
| 10,0 Gew.-% | Dysprosium |
| 1,0 Gew.-% | Neodym |
| 1,0 Gew.-% | Europium |
| 1,0 Gew.-% | Zink |
| 0,2 Gew.-% | Zirkonium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Folglich betrifft eine weitere bevorzugte Ausführungsform der Erfindung Stents bestehend aus einer biologisch abbaubaren Magnesiumlegierung, die die folgenden Bestandteile umfasst oder aus den folgenden Bestandteilen besteht:

| | |
|---|---|
| 93,0 Gew.-% - 94,0 Gew.-% | Magnesium |
| 0,3 Gew.-% - 0,4 Gew.-% | Dysprosium |
| 2,0 Gew.-% - 2,1 Gew.-% | Neodym |
| 0,3 Gew.-% - 0,5 Gew.-% | Gadolinium |
| 0,3 Gew.-% - 0,4 Gew.-% | Zirkonium |
| 3,4 Gew.-% - 3,8 Gew.-% | Yttrium |
| 0,0 Gew.-% - 0,02 Gew.-% | Erbium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 92,0 Gew.-% - 93,0 Gew.-% | Magnesium |
| 0,9 Gew.-% - 1,1 Gew.-% | Dysprosium und Gadolinium zusammen |
| 2,1 Gew.-% - 2,3 Gew.-% | Neodym |
| 0,4 Gew.-% - 0,6 Gew.-% | Zirkonium |
| 3,9 Gew.-% - 4,3 Gew.-% | Yttrium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 91,0 Gew.-% - 92,0 Gew.-% | Magnesium |
| 0,7 Gew.-% - 0,8 Gew.-% | Dysprosium |
| 0,6 Gew.-% - 0,8 Gew.-% | Gadolinium |
| 1,9 Gew.-% - 2,1 Gew.-% | Neodym |
| 0,6 Gew.-% - 0,8 Gew.-% | Zirkonium |
| 3,9 Gew.-% - 4,2 Gew.-% | Yttrium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Zudem betrifft eine weitere bevorzugte Ausführungsform der Erfindung Stents bestehend aus einer biologisch abbaubaren Magnesiumlegierung, die die folgenden Bestandteile umfasst oder aus den folgenden Bestandteilen besteht:

| | |
|---|---|
| 91,0 Gew.-% - 92,0 Gew.-% | Magnesium |
| 0,7 Gew.-% - 0,8 Gew.-% | Dysprosium |
| 0,6 Gew.-% - 0,8 Gew.-% | Gadolinium |
| 1,9 Gew.-% - 2,1 Gew.-% | Neodym |
| 0,6 Gew.-% - 0,8 Gew.-% | Zirkonium |
| 3,9 Gew.-% - 4,2 Gew.-% | Yttrium |

wobei der Stent eine Magnesiumfluorid-Beschichtung und eine organische Beschichtung aus Poly-L-Lactid (PLLA) enthält.

Eine weitere bevorzugte Ausführungsform der Erfindung betrifft Stents bestehend aus einer biologisch abbaubaren, Yttrium freien Magnesiumlegierung, die die folgenden Bestandteile umfasst oder aus den folgenden Bestandteilen besteht:

| | |
|---|---|
| 93,7 Gew.-% - 96,2 Gew.-% | Magnesium |
| 1,0 Gew.-% - 1,5 Gew.-% | Gadolinium |
| 2,0 Gew.-% - 3,1 Gew.-% | Neodym |
| 0,5 Gew.-% - 0,7 Gew.-% | Zirkonium |
| 0,1 Gew.-% - 0,5 Gew.-% | Calcium |
| 0,2 Gew.-% - 0,5 Gew.-% | Zink |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid

und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 96,9 Gew.-% | Magnesium |
| 2,5 Gew.-% | Neodym |
| 0,4 Gew.-% | Zirkonium |
| 0,2 Gew.-% | Zink |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 97,45 Gew.-% | Magnesium |
| 0,75 Gew.-% | Neodym |
| 1,80 Gew.-% | Mangan |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 97,45 Gew.-% | Magnesium |
| 0,75 Gew.-% | Cer |
| 1,80 Gew.-% | Mangan |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 90,0 Gew.-% | Magnesium |
| 3,0 Gew.-% | Gadolinium |
| 2,4 Gew.-% | Yttrium |
| 0,4 Gew.-% | Zirkonium |
| 4,2 Gew.-% | Scandium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 90,0 Gew.-% | Magnesium |
| 3,0 Gew.-% | Neodym |
| 2,4 Gew.-% | Yttrium |
| 0,4 Gew.-% | Zirkonium |
| 5,2 Gew.-% | Scandium |

(fortgesetzt)

| | |
|---|---|
| 2,0 Gew.-% | Indium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 96,0 Gew.-% | Magnesium |
| 4,0 Gew.-% | Lithium |

wobei der Stent eine Magnesiumfluorid-Beschichtung und eine organische Beschichtung, umfassend eine oder mehrere Substanzen der folgenden Gruppe: Poly-ε-Caprolacton (PCL), Poly-L-Lactid-co-Glycolid (PLGA), Poly-L-Lactid und Parylene, enthält.

Als organische Beschichtung sind die Polymere Poly-ε-Caprolacton (PCL), Poly-L-Lactid-co-Glycolid (PLGA), Poly-L-Lactid und Parylene bevorzugt, wobei in Vorversuchen die folgenden Polymere vergleichbare Resultate lieferten: Polyvinylpyrrolidon, Polyhydroxyethyl-methacrylate, Poly(hydroxybutyrat), Polyvalerolactone, Poly-ε-Decalactone, Poly(D,L-lactid-co-glycolid), Polyglycolide, Copolymere der Polylactide und Polyglycolide, Polyhydroxyvalerate, Polyhydroxymethacrylate, Polycaprolactondimethylacrylate, Polycaprolactonbutylacrylate, Polycaprolactonglycolide, Polyurethane, Polymethylmethacrylat, Polyvinylketone, Polyvinylether, Polyvinylaromaten, Polyvinylester, Polyethylenterephtalat, Polyvalerate und Polysulfone.

Die vorliegende Erfindung bezieht sich auf Stents aus einer biologisch abbaubaren Magnesiumlegierung wie hierin offenbart mit mindestens 80 Gew.-% Magnesium, einer anorganischen Beschichtung umfassend oder bestehend aus Magnesiumfluorid und einer organischen Beschichtung.

Die anorganische Beschichtung kann zusätzlich zum Magnesiumfluorid weitere anorganische aber auch organische Stoffe umfassen. Zu den anorganischen Stoffen werden traditionell die Elemente und alle Verbindungen gezählt, die keinen Kohlenstoff enthalten. Dazu kommen einige Ausnahmen von Kohlenstoffverbindungen, die genau wie typische anorganische Stoffe aufgebaut sind oder historisch der Anorganik zugeordnet werden. Hierzu gehören die wasserstofffreien Chalkogenide des Kohlenstoffs, die Kohlensäure und Carbonate, die Carbide sowie die ionischen Cyanide, Cyanate und Thiocyanate. Eine anorganische Beschichtung wie hierin verwendet bezeichnet eine auf die Streben eines Stents aufgebrachte Schicht bestehend aus Magnesiumfluorid und einer dieser anorganischen Verbindungen, Mischungen daraus oder zumindest enthaltend Magnesiumfluorid als Hauptbestandteil, d.h. zu mindestens 50%, bevorzugt zu mindestens 80% und noch weiter bevorzugt zu mindestens 90%. Auf die anorganische Schicht kann eine Schicht aus einem Antirestenose-Wirkstoff oder eine organische Schicht aus einer oder mehreren organischen Verbindungen vorzugsweise organischen Polymeren aufgebracht werden, wobei diese organische Schicht auch den Antirestenose-Wirkstoff enthalten kann, so dass keine dritte Wirkstoffschicht mehr aufgebracht werden muss. Somit kann die Schicht aus einer oder mehreren organischen Verbindungen einen oder mehrere Antirestenose-Wirkstoff(e) enthalten oder der Antirestenose-Wirkstoff kann auf die Schicht aus einer oder mehreren organischen Verbindungen aufgebracht werden, wobei die dann mittlere organische Schicht keinen, denselben oder einen anderen oder mehr als einen Antirestenose-Wirkstoff enthalten kann.

Es kann von Vorteil sein, dass die abluminale (der Gefäßwand zugewandte) Beschichtung sich langsamer löst als die luminale (dem Gefäßlumen zugewandte) Stentbeschichtung. Des Weiteren ist ein Stent bevorzugt, der nur in der luminalen, anorganischen, biologisch abbaubaren Beschichtung und der darüber liegenden luminalen, organischen Beschichtung Mikroporen, Löcher, Öffnungen oder Kanäle aufweist. Beim Abbau von Magnesiumlegierungen wird Wasserstoffgas gebildet. Dies ist ein Grund, dass es bevorzugt ist, dass in der dem Lumen und dem Blutfluss zugewandten Seite der anorganischen, bioabbaubaren Beschichtung und der organischen Beschichtung Mikroporen, Löcher, Öffnungen, Kanäle oder sonstige Strukturen, die das Ausströmen von Gas ermöglichen, enthalten sind aber in der abluminalen Seite der Beschichtung nicht, da so das Gas mit dem Blutstrom weggewaschen und verteilt wird und sich nicht zwischen dem Stent und der Gefäßwand ansammeln kann.

Wenn nicht durch den Beschichtungsprozess selbst erzeugt, können diese Mikroporen, Löcher, Öffnungen und/oder Kanäle mechanisch, chemisch, thermisch oder optisch in die anorganische Beschichtung eingebracht werden nachdem diese auf den Stent aufgebracht wurde. Zudem können Mikroporen, Löcher, Öffnungen und/oder Kanäle mechanisch, chemisch, thermisch oder optisch in die organische Beschichtung, die auf die anorganische Beschichtung aufgebracht ist, eingebracht werden. Beispielsweise kann dies durch mechanische Behandlung wie Sandstrahlung, durch chemische Verfahren wie Ätzung, durch mechanischchemische Verfahren wie Polierverfahren, durch thermische

Verfahren wie Schmelzen oder Einbrennen oder durch optische Verfahren wie Laserbehandlung geschehen.

Erfindungsgemäß ist es bevorzugt, wenn die anorganische Beschichtung und die organische Beschichtung derart ausgestaltet sind, dass sich das innere metallische Gerüst in der Beschichtung auflösen kann und sowohl das Wasserstoffgas als auch die Metallionen vorwiegend an der luminalen Seite der Beschichtung in das Blut abgegeben werden, nicht jedoch direkt in das umliegende Gewebe austreten.

Besonders bevorzugt ist jedoch ein Stent aus einer der hier beschriebenen, bioabbaubaren Magnesiumlegierungen mit mindestens 80 Gew.-% Magnesium, wobei die anorganische Beschichtung und/oder die organische Beschichtung keine Mikroporen, Löcher, Öffnungen oder Kanäle aufweisen. Dies gilt insbesondere für anorganische oder organische Beschichtungen ohne Wirkstoff. Ebenso ist es bevorzugt, wenn ein Stent aus einer der hier beschriebenen, bioabbaubaren Magnesiumlegierungen mit mindestens 80 Gew.-% Magnesium, eine organische Beschichtung aufweist, die keine Mikroporen, Löcher, Öffnungen oder Kanäle besitzt.

Es ist bevorzugt, wenn das innere Grundgerüst aus der biologisch abbaubaren Magnesiumlegierung unter physiologischen Bedingungen abgebaut wird bevor die anorganische Beschichtung und die äußere organische Beschichtung abgebaut werden, so dass nach Degradation des inneren Grundgerüstes eine in der Gefäßwand eingewachsene leere Hülle zurückbleibt, welche jedoch flexibel ist und keinen nennenswerten Druck auf die Gefäßwand mehr ausübt und sich sogar dem neuen Gefäßverlauf gut anpasst. Nach der vollständigen Auflösung des inneren, metallischen Grundgerüsts können auch die anorganische Beschichtung und die organische Beschichtung biologisch abgebaut werden, so dass sich nach einigen Monaten der Stent vollständig aufgelöst hat. Dabei sollte der Abbau der anorganischen Beschichtung und der organischen Beschichtung jeweils gleichmäßig verlaufen und ohne, dass die Gefahr von sich ablösenden Bruchstücken entsteht.

Generell dienen die anorganische Beschichtung und die organische Beschichtung der Regulation der Degradationsgeschwindigkeit des metallischen Stentgerüsts. Durch die Wahl der Verbindungen oder der Mischung aus Verbindungen, die die anorganische Beschichtung oder die organische Beschichtung bilden, kann die Dauer bis zur Auflösung des Grundgerüsts beeinflusst werden. Des Weiteren können die anorganische Beschichtung und die organische Beschichtung als Schutzhülle vor Bruchstücken des Grundgerüsts dienen und die Oberfläche des Stents biokompatibler bzw. hämokompatibler gestaltet werden. Das bedeutet, dass die anorganische Beschichtung und die organische Beschichtung eines erfindungsgemäßen Stents die Blutverträglichkeit verbessert. Dies kann in einer besseren und gleichmäßigen Besiedelung der Oberfläche mit Zellen, insbesondere glatten Muskel- und vorzugsweise Endothelzellen, liegen. Die Stentoberfläche kann durch die anorganische Beschichtung und durch die organische Beschichtung aber auch weniger Blutgerinnung auslösen und so zu einer Reduktion der Thrombosegefahr führen.

Bei weiteren Ausführungsformen befindet sich in oder auf der anorganischen Beschichtung und unter der organischen Beschichtung mindestens ein antiinflammatorischer, antiproliferativer, antiangiogener, antirestenotischer (anti-Restenose), antineoplastischer, antimigrativer und/oder antithrombogener Wirkstoff. Dieser Wirkstoff kann in kovalent gebundener Form oder in adhäsiv oder ionisch gebundener Form in der anorganischen Beschichtung enthalten oder als zusätzliche Schicht aufgebracht sein. Dadurch werden beschichtete Endoprothesen bzw. Stents erhalten, welche mindestens einen Wirkstoff in der anorganischen Beschichtung aufweisen oder die eine zusätzliche Schicht enthaltend den Wirkstoff auf der anorganischen Beschichtung aufweist. Vorzugsweise ist der mindestens eine antiinflammatorische, antiproliferative, antiangiogene, antirestenotische (anti-Restenose), antineoplastische, antimigrative und/oder antithrombogene Wirkstoff in Form einer zusätzlichen, wirkstofffreisetzenden Schicht (drug release system) auf der Oberfläche der anorganischen Beschichtung des Stents aufgebracht.

Bei weiteren bevorzugten Ausführungsformen befindet sich in oder auf der äußeren polymeren organischen Beschichtung mindestens ein Antirestenose-Wirkstoff wie z.B. ein antiinflammatorischer, antiproliferativer, antiangiogener, antirestenotischer, antineoplastischer, antimigrativer und/oder antithrombogener Wirkstoff. Geeignete Antirestenose-Wirkstoffe wurden bereits weiter oben explizit genannt. Bevorzugt sind Derivate von Sirolimus ("Limus"-Derivate) als auch Paclitaxel. Insbesondere bevorzugt ist Sirolimus selbst. Der Wirkstoff kann in kovalent gebundener Form oder in adhäsiv oder ionisch gebundener Form in der polymeren organischen Beschichtung enthalten oder als zusätzliche Schicht aufgebracht sein. Dadurch werden beschichtete Endoprothesen bzw. Stents erhalten, welche mindestens einen Wirkstoff in der polymeren Beschichtung aufweisen oder die eine zusätzliche Schicht enthaltend den Wirkstoff auf der polymeren Beschichtung aufweist. Vorzugsweise ist der mindestens eine antiinflammatorische, antiproliferative, antiangiogene, antirestenotische, antineoplastische, antimigrative und/oder antithrombogene Wirkstoff in Form einer zusätzlichen, wirkstofffreisetzenden Schicht (drug release system) auf der Oberfläche der polymeren organischen Beschichtung des Stents aufgebracht. Bei dieser wirkstofffreisetzenden Schicht kann es sich um eine reine Wirkstoffschicht oder um eine Trägerschicht aus den vorgenannten Trägerstoffen oder Matrixstoffen handeln als auch aus einem weiteren Polymer.

Der mindestens eine eingesetzte, antiinflammatorische, antiproliferative, antiangiogene, antirestenotische, antineoplastische, antimigrative und/oder antithrombogene Wirkstoff ist bevorzugt ausgewählt aus der Gruppe umfassend oder bestehend aus: Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-AII, Aristolochsäure, Ascomycin, Asparaginase,

Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, Mitoxanthrone, Mycophenolatmofetil, β-Lapachon, Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren, Pentaerythrityltetranitrat und Syndnoeimine, S-Nitrosoderivate, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate, 6-a-Hydroxy-Paclitaxel, Taxotere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin Tobramycin, Gentamycin, Penicilline, Dicloxacillin, Oxacillin, Sulfonamide, Metronidazol, Enoxoparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon a, β und γ, Histaminantagonisten, Serotoninblocker, Apoptoseinhibitoren, Apoptoseregulatoren, Halofuginon, Nifedipin, Paracetamol, Dexpanthenol, Clopidogrel, Acetylsalicylsäurederivate, Streptomycin, Neomycin, Framycetin, Paromomycin, Ribostamycin, Kanamycin, Amikacin, Arbekacin, Bekanamycin, Dibekacin, Spectinomycin, Hygromycin B, Paromomycinsulfat, Netilmicin, Sisomicin, Isepamicin, Verdamicin, Astromicin, Apramycin, Geneticin., Amoxicillin, Ampicillin, Bacampicillin, Pivmecillinam, Flucloxacillin, Mezlocillin, Piperacillin, Azlocillin, Temocillin, Ticarcillin, Amoxicillin, Clavulansäure, Ampicillin, Sulbactam, Piperacillin, Tazobactam, Sulbactam, Cefamandol, Cefotiam, Cefuroxim, Cefmenoxim, Cefodizim, Cefoperazon, Cefotaxim, Ceftazidim, Cefsulodin, Ceftriaxon, Cefepim, Cefpirom, Cefoxitin, Cefotetan, Cefalexin, Cefuroxim Axetil, Cefixim, Cefpodoxim, Ceftibuten, Imipenem, Meropenem, Ertapenem, Doripenem, Aztreonam, Spiramycin, Azithromycin, Telithromycin, Quinopristin, Dalfopristin, Clindamycin, Tetracyclin, Doxycyclin, Minocyclin, Trimethoprim, Sulfamethoxazol, Sulfametrol, Nitrofurantoin, Lomefloxacin, Norfloxacin, Ciprofloxacin, Ofloxacin, Fleroxacin, Levofloxacin, Sparfloxacin, Moxifloxacin, Vancomycin, Teicoplanin, Linezolid, Daptomycin, Rifampicin, Fusidinsäure, Fosfomycin, Trometamol, Chloramphenicol, Metronidazol, Colistin, Mupirocin, Bacitracin, Neomycin, Fluconazol, Itraconazol, Voriconazol, Posaconazol, Amphotericin B, 5- Flucytosin, Caspofungin, Anidulafungin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin) und dessen Derivate wie Biolimus A9, Everolimus, Myolimus, Novolimus, Pimecrolimus, Ridaforolimus, Deoxorapamycin, Tacrolimus FK 506, Temsirolimus und Zotarolimus., Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B,Zeorin. und schwefelhaltige Aminosäuren wie Cystin sowie Salze, Hydrate, Solvate, Enantiomere, Racemate, Enantiomerengemische, Diastereomerengemische; Metaboliten, Prodrugs und Mischungen der vorgenannten Wirkstoffe. Die Konzentration pro Wirkstoff liegt vorzugsweise im Bereich von 0,001-500 mg pro $cm^2$ beschichteter Oberfläche der Endoprothese. Besonders bevorzugte Wirkstoffe im Sinne der vorliegenden Erfindung sind Paclitaxel, Rapamycin und deren Derivate, wie 6-$\alpha$-Hydroxy-Paclitaxel, Baccatin oder andere Taxotere, Biolimus A9, Myolimus, Novolimus, Pimecrolimus, Tacroliums, Temsirolimus, Zotarolimus, Everolimus, Ridaforolimus oder weitere "Limus"-Derivate, Erythromycin, Midecamycin, Josamycin und Triazolopyrimidine. Insbesondere bevorzugt ist Sirolimus (Rapamycin). Auch bevorzugt sind Paclitaxel und die "Limus"-Derivate Biolimus A9, Myolimus, Novolimus, Pimecrolimus, Tacroliums, Temsirolimus, Zotarolimus, Everolimus, Ridaforolimus und weitere Sirolimus-Derivate. Sämtliche vorgenannten Verbindungen werden hierin auch der Einfachheit halbe als Antirestenose-Wirkstoffe bezeichnet.

Gemäß einer bevorzugten Ausführungsform weist der Stent eine anorganische Beschichtung auf, die von einer organischen Beschichtung überzogen ist, die mindestens einen antiproliferativen, antiphlogistischen und/oder antithrombotischen Wirkstoff enthält.

In einer besonders bevorzugten Ausführungsform besteht die Stentbeschichtung aus einer ersten anorganischen Beschichtung umfassend oder bestehend aus Magnesiumfluorid, die von einer zweiten organischen Beschichtung überzogen ist, die mindestens einen Antirestenose-Wirkstoff wie z.B. einen antiproliferativen, antiphlogistischen und/oder antithrombotischen Wirkstoff enthält.

Zwischen der anorganischen Beschichtung und der organischen Wirkstoff-enthaltenden Beschichtung kann auch eine zusätzliche haftvermittelnde Schicht aufgetragen werden. Alternativ kann eine Verbindung zur Unterstützung der Anhaftung in der organischen Wirkstoff-enthaltenden Beschichtung enthalten sein.

Eine bevorzugte Ausführungsform der Erfindung besteht also aus einem Stent bestehend aus einem Grundgerüst aus einer der hier offenbarten, bioabbaubaren Magnesiumlegierungen mit mindestens 80 Gew.-% Magnesium, einer anorganischen Beschichtung umfassend Magnesiumfluorid und einer organischen Beschichtung, optional mit mindestens einem Wirkstoff.

Ferner ist bevorzugt, wenn die organische Beschichtung vorzugsweise aus einem organischen Polymer wie z.B. einem Parylen besteht und einen Antirestenose-Wirkstoff enthält und/oder eine dritte Beschichtung aus dem Antirestenose-Wirkstoff auf dieser organischen Beschichtung befindet.

Es ist auch möglich, dass der Wirkstoff auf den Stent aufgebracht wird, nachdem die anorganische Beschichtung bereits und bevor die organische Beschichtung auf dem metallischen Grundgerüst aufgebracht ist und der Wirkstoff keine eigene Schicht bildet, sondern in die bereits bestehende anorganische Beschichtung eindringt. Dann ist es bevorzugt, dass der Wirkstoff nicht die gesamte Beschichtung durchdringt, sondern in einem äußeren Teil verbleibt und einen Konzentrationsgradienten bildet, welcher zum Grundgerüst hin abnimmt.

Die anorganische Schicht bzw. Beschichtung des Stents selbst enthält bevorzugt maximal 10% Polymere und ist noch weiter bevorzugt polymerfrei. Auf der vorzugsweise polymerfreien anorganischen Beschichtung kann sich optional eine Schicht aus mindestens einem Antirestenose-Wirkstoff oder eine organische Beschichtung beispielsweise aus einem Polymer oder einer Trägersubstanz oder einer Matrixsubstanz mit oder ohne mindestens einen Antirestenose-Wirkstoff befinden. Geeignete Träger und Matrices sind hierin beschrieben.

Wird jedoch der mindestens eine Wirkstoff bzw. die Wirkstoffkombination auf die anorganische Beschichtung des Stents aufgebracht, können in Kombination mit dem mindestens einen Wirkstoff oder der Wirkstoffkombination weitere Substanzen als pharmakologisch verträgliche Träger bzw. als Matrix aufgebracht werden. Diese Träger oder Matrices werden auch als organische Beschichtung oder organische Schicht bezeichnet.

Als pharmakologisch verträgliche Träger können sowohl Polymere dienen als auch niedermolekulare Substanzen, wie beispielsweise Lactose, Stärke, Natrium-Carboxymethylstärke, Sorbitol, Sucrose, Magnesiumstearat, Dicalciumphosphat, Calciumsulfat, Talk, Mannitol, Ethylalkohol, Polyvinylalkohole, Polyvinylpyrrolidon, Gelatine, natürliche Zucker, sowohl natürliche als auch synthetische Gummis wie beispielsweise Akaziengummi oder Guar-Gummi, Natriumalginat, Natriumbenzoat, Natriumacetat, Glyceride, Isopropylmyristate und palmitate, Citrate, wie Tributyl- und Triethylcitrate und deren Acetylderivate, Phtalate wie Dimethylphtalat oder Dibutylphtalat, etc., Benzoesäurebenzylester, Triacetin, 2-Pyrrolidon, Borsäure, Magnesium-Aluminum-Silicate, natürliche Johannisbrotkernmehl, Karaya, Guar, Tragacanth, Agar, Cellulose, Cellulosederivate wie Methylcellulose, Natrium-Carboxymethylcellulose, Hydroxypropylmethylcellulose, microkristalline Cellulose sowie Alginate, PLLA, Parylene, Polysulfone, Schellack, Tonerden und Bentonite, Polyethylenglycol als auch Wachse wie z.B. Bienenwachs, Carnaubawachs, Candelillawachs und dergleichen eingesetzt

werden. Die Matrixsubstanz der zweiten Schicht kann aber auch mit einer anorganischen Verbindung der ersten Schicht oder mit der gesamten Zusammensetzung der ersten Schicht identisch sein. Die zusätzlichen Träger- oder Matrixsubstanzen können in einem Gewichtsverhältnis von bis zu 70 Gew.-%, bevorzugt bis 50 Gew.-% bezogen auf den oder die verwendeten Wirkstoffe eingesetzt werden.

Eine bevorzugte Ausführungsform ist ein Stent aus einer der hierin offenbarten Magnesiumlegierungen mit mindestens 80 Gew.-% Magnesium, einer anorganischen Beschichtung umfassend Magnesiumfluorid und einer organischen Beschichtung auf der anorganischen Beschichtung aus einem Parylen. Ferner ist bevorzugt, wenn die organische Beschichtung einen Antirestenose-Wirkstoff enthält und/oder sich darauf befindet, wie vorzugsweise Paclitaxel, Sirolimus, Biolimus A9, Myolimus, Novolimus, Pimecrolimus, Tacrolimus, Temsirolimus, Zotarolimus, Everolimus, Ridaforolimus oder ein anderes Sirolimus-Derivat und insbesondere bevorzugt Sirolimus (Rapamycin).

Eine bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 91,0 Gew.-% - 92,0 Gew.-% | Magnesium |
| 0,7 Gew.-% - 0,8 Gew.-% | Dysprosium |
| 0,6 Gew.-% - 0,8 Gew.-% | Gadolinium |
| 1,9 Gew.-% - 2,1 Gew.-% | Neodym |
| 0,6 Gew.-% - 0,8 Gew.-% | Zirkonium |
| 3,9 Gew.-% - 4,2 Gew.-% | Yttrium |

wobei der Stent eine Magnesiumfluorid-Beschichtung und eine organische Beschichtung aus Poly-L-Lactid (PLLA), in der oder auf der sich mindestens ein antiinflammatorischer, antiproliferativer, antiangiogener, antirestenotischer, antineoplastischer, antimigrativer und/oder antithrombogener Wirkstoff befindet, enthält.

Eine weitere bevorzugte Ausführungsform der Erfindung betrifft Stents bestehend aus einer biologisch abbaubaren Magnesiumlegierung, die die folgenden Bestandteile umfasst oder aus den folgenden Bestandteilen besteht:

| | |
|---|---|
| 91,0 Gew.-% - 92,0 Gew.-% | Magnesium |
| 0,7 Gew.-% - 0,8 Gew.-% | Dysprosium |
| 0,6 Gew.-% - 0,8 Gew.-% | Gadolinium |
| 1,9 Gew.-% - 2,1 Gew.-% | Neodym |
| 0,6 Gew.-% - 0,8 Gew.-% | Zirkonium |
| 3,9 Gew.-% - 4,2 Gew.-% | Yttrium |

wobei der Stent eine Magnesiumfluorid-Beschichtung und eine organische Beschichtung aus Poly-L-Lactid (PLLA) enthält und die organische Beschichtung zusätzlich den Wirkstoff Sirolimus enthält und/oder sich darauf befindet.

Die organische Beschichtung kann generell mittels bekannten Verfahren wie beispielsweise Sprühverfahren, Tauchverfahren, Plasmaverfahren, Pinselverfahren, Spritzenverfahren oder Pipettierverfahren auf die anorganische Beschichtung, die sich bereits auf der Magnesiumlegierung des Grundgerüstes befindet, aufgebracht werden. Der erfindungsgemäße Stent kann also mittels Sprüh-, Pipettier-, Pinsel-, Spritzen-, Plasma- oder Tauchverfahren, beschichtet werden, wobei die organische Verbindung oder Mischungen der Verbindungen in einem Lösungsmittel gelöst werden und diese Lösung auf das Implantat aufgetragen wird. Anschließend wird das Lösungsmittel oder das Lösungsmittelgemisch durch Verdunstung bei Raumtemperatur oder Erwärmung entfernt.

Die Beschichtung der erfindungsgemäßen Stents kann sowohl vor als auch nach dem Crimpen auf einen Katheterballon durchgeführt werden. Sofern die Beschichtung erst aufgebracht wird, nachdem der Stent auf einem Katheterballon befestigt wurde, ist ein Tauch oder Sprühverfahren bevorzugt.

Die organische Beschichtung sollte relativ gleichmäßig erfolgen. Die organische Beschichtung kann eine Dicke von etwa 0,01 bis 100 μm aufweisen und bevorzugt von 0,1 - 50 μm, weiter bevorzugt von 0,2 - 20 μm und am meisten bevorzugt von 0,5 - 10 μm.

Im Falle von Parylen als organische Beschichtung kann die Schichtdicke geringer sein und liegt im Bereich von 0,001 bis 10 μm, vorzugsweise im Bereich von 0,01 - 5 μm und weiter bevorzugt im Bereich von 0,05 - 1,0 μm.

Weiterhin ist bevorzugt, wenn das Verhältnis der Schichtdicke der anorganischen Beschichtung und der organischen Beschichtung der erfindungsgemäßen Stents 500:1 bis 1:1.000.000, mehr bevorzugt 400:1 bis 1:700.000, noch mehr bevorzugt 100:1 bis 1:200.000 und am meisten bevorzugt 20:1 bis 1:100.000 beträgt.

Ebenso ist bevorzugt, wenn das Verhältnis der Schichtdicke der anorganischen Beschichtung und der organischen Beschichtung der erfindungsgemäßen Stents 1:500.000 bis 1:1.000, mehr bevorzugt 1:100.000 bis 1:5.000, noch

mehr bevorzugt 1:50.000 bis 1:10.000 (anorganisch : organisch) beträgt.

Die anorganische Beschichtung kann generell mittels bekannten Verfahren wie beispielsweise Sprühverfahren, Tauchverfahren, Plasmaverfahren, Pinselverfahren, Spritzenverfahren oder Pipettierverfahren auf die Magnesiumlegierung des Grundgerüstes aufgebracht werden. Der erfindungsgemäße Stent kann also mittels Sprüh-, Pipettier-, Pinsel-, Spritzen-, Plasma- oder Tauchverfahren, beschichtet werden, wobei die anorganische Verbindung bzw. das anorganische Material oder Mischungen der Verbindungen in einem Lösungsmittel gelöst werden und diese Lösung auf das Implantat aufgetragen wird. Anschließend wird das Lösungsmittel oder das Lösungsmittelgemisch durch Verdunstung bei Raumtemperatur oder Erwärmung entfernt.

Die Beschichtung der erfindungsgemäßen Stents kann sowohl vor als auch nach dem Crimpen auf einen Katheterballon durchgeführt werden. Sofern die Beschichtung erst aufgebracht wird, nachdem der Stent auf einem Katheterballon befestigt wurde, ist ein Tauch oder Sprühverfahren bevorzugt.

Verschiedene im Stand der Technik bekannte Verfahren können verwendet werden um eine anorganische Beschichtung auf einen Stent aufzubringen. Zum Beispiel können Suspensionen von anorganischen Teilchen in Wasser oder organischen Lösungsmitteln, wie Ethanol, verwendet werden, um die anorganischen Beschichtungen herzustellen.

Gemäß einem Aspekt der vorliegenden Offenbarung kann das Aufbringen und Trocknen der anorganischen Beschichtung mehrfach wiederholt werden, um eine Beschichtung aus mehreren Schichten zu bilden. Ebenso kann das Aufbringen und Trocknen der organischen Beschichtung mehrfach wiederholt werden, um eine Beschichtung aus mehreren Schichten zu bilden. Aufbringen in mehreren Schichten erleichtert die Bildung der anorganischen Beschichtung bzw. organischen Beschichtung mit der gewünschten Dicke (z. B. in der Größenordnung von μm). In eine dickere Beschichtung kann mehr Wirkstoff eingelagert werden.

Zur anorganischen Beschichtung von Oberflächenbereichen/Randzonenbereichen der erfindungsgemäßen Stent kann auch die Ionenimplantation verwendet werden. Bei der Ionenimplantation ist ein Verfahren zur Einbringung von Fremdatomen in Form von Ionen in das Grundmaterial durch den Beschuss der Legierung mit beschleunigten Ionen im Hochvakuum. Dabei werden mittels einer Ionenquelle zunächst die Ionen erzeugt, die durch ein elektrisches Feld extrahiert werden und in einem weiteren Schritt entsprechend ihrer Masse durch einen Massenseparator getrennt werden. Die Ionen werden dann beschleunigt und mit einem elektrischen Feld auf die Legierung gelenkt. Die Ionen werden in die Legierung implantiert. Zu den wichtigsten Parametern der Ionenimplantation gehören u.a. die Beschleunigungsenergie mit der die Ionen beschleunigt werden, die Ionenart und die Implantationsdosis. Die ersten beiden Parameter bestimmen die Eindringtiefe der Ionen in die Legierung und die Implantationsdosis die Konzentration der implantierten Ionen. Mit der Ionenimplantation können unterschiedliche Ionen in die Legierung eingebaut werden z. B. Fluorid-Ionen, Sauerstoff-Ionen, Kohlenstoffionen. Durch die Implantation können in Abhängigkeit von der Masse der implantierten Ionen und der Implantationsdosis Schäden im Kristallgitter der Legierung entstehen. Die Legierung muss in solchen Fällen nach einem Implantationsschritt einem Hochtemperaturprozess unterzogen werden, bei dem die Fremdatome in das Gitter eingebaut werden. Dieser Prozess wird auch als Ausheilen bezeichnet. Der Ausheilprozess kann beispielweise durch einen Ofenprozess bewerkstelligt werden.

Bei der chemischen Umwandlung zur anorganischen Beschichtung der erfindungsgemäßen Stents wird die Oberfläche der Magnesiumlegierung durch eine chemische Reaktion die gewünschte Magnesiumfluorid-Beschichtung in Gegenwart einer Fluorid-haltigen wässrigen Lösung umgewandelt. Die Beschichtung kann in Abhängigkeit von der Zusammensetzung der Ausgangslegierung u.a. Anteile an Dysprosium, Europium, Neodym, Zink, Zirkonium, Yttrium, Erbium, Gadolinium und Calcium beinhalten. Ferner können die Beschichtungen in Folge von unvermeidbaren Verunreinigungen der eingesetzten Magnesiumlegierung Si, Ni, Fe, Cu sowie weitere Metalle und Nichtmetalle umfassen. Bevorzugte Reaktionsmedien sind Flusssäure, wässrige Kaliumfluorid-Lösung und wässrige AmmoniumfluoridLösung.

Die elektrochemische Plasmaoxidation und das Sol-Gel-Verfahren sind ebenfalls geeignete Verfahren um eine anorganische Beschichtung gemäß der vorliegenden Erfindung zu erzeugen. Bei der Plasmaoxidation werden durch Anlegen von Spannungen von bis zu einigen 100 V in wässrigen Elektrolyten auf der Oberfläche des Stents Oxidschichten von bis zu 10 μm Dicke erzeugt.

Sol-Gel-Verfahren beinhalten die Bildung einer kolloidalen Suspension aus sogenannten Prekursoren. Bei den Ausgangsmaterialien für eine Solsynthese handelt es sich oft um Alkoholate von Metallen oder Nichtmetallen. Nachdem die kolloidale Suspension gebildet wurde, werden Wasser, eine Säure, eine Base oder eine Kombination davon zugegeben, um Hydrolyse und Kondensation zu initiieren. Die Hydrolyse von Prekursor-Molekülen und die Kondensation zwischen dabei entstehenden reaktiven Spezies, sind die wesentlichen Grundreaktionen des Sol-Gel-Prozesses. Die dabei ablaufenden Vorgänge und die Eigenschaften der Prekursor-Moleküle haben einen entscheidenden Einfluss auf die resultierenden Materialeigenschaften. Nachdem die Beschichtung auf wenigstens einem Teil des Stents aufgebracht ist, wird die Beschichtungszusammensetzung erwärmt, was zur Alterung und zur Entfernung von organischen Lösungsmitteln erforderlich ist.

Die anorganische Beschichtung sollte relativ gleichmäßig erfolgen. Die anorganische Beschichtung kann eine Dicke von etwa 0,1 pm bis 10 μm aufweisen und hat bevorzugt eine Dicke von 1 pm bis etwa 100 nm, weiter bevorzugt von 10 pm bis etwa 1 nm. Die gewünschte Schichtdicke hängt auch von der jeweiligen anorganischen Verbindung ab, die

zusätzlich zum Magnesiumfluorid in der anorganischen Beschichtung enthalten sein kann, und kann durch mehrmalige Beschichtungsschritte unterbrochen von Trocknungsschritten realisiert werden. Insbesondere bei einer Abscheidung der anorganischen Beschichtung aus einer Gasphase wird die Schicht bei längerer Beschichtungsdauer undurchlässig. Bei kurzen Beschichtungszeiten treten undichte Stellen auf, die die Diffusion von Wasser oder Gasen zulassen.

In einem geeigneten Lösungsmittel eventuell auch zusammen mit der anorganischen Verbindung kann der mindestens eine aufzubringende, antiinflammatorische, antiproliferative, antiangiogene, antirestenotische (anti-Restenose), antineoplastische, antimigrative und/oder antithrombogene Wirkstoff gelöst, emulgiert, suspendiert oder dispergiert werden. Sofern eine Matrix- oder Trägersubstanz in der Wirkstoffschicht enthalten ist, kann diese zusammen mit dem Wirkstoff gelöst und aufgetragen werden, oder in einem Sprüh-, Pipettier- oder Tauchverfahren getrennt, bevorzugt zuvor, aufgetragen werden.

Bei einer bevorzugten Ausführungsform wird zuerst die anorganische Beschichtung auf den Stent aufgebracht, getrocknet, danach auf diese Beschichtung eine organische Beschichtung aufgebracht, getrocknet und zuletzt ein Wirkstoff aufgebracht. Hierzu wird bevorzugt eine Lösung des mindestens einen Wirkstoffs und eventuell einer Matrix- oder Trägersubstanz in einem leichtflüchtigen Lösungsmittel auf die organische Beschichtung der Stents aufgebracht. Anschließend wird das Lösungsmittel oder das Lösungsmittelgemisch durch Verdunstung bei Raumtemperatur entfernt.

Als Lösungsmittel eignen sich Wasser und bevorzugt organische Lösungsmittel wie beispielsweise Chloroform, Methylenchlorid (Dichlormethan), Aceton, Tetrahydrofuran (THF), Diethylether, Methanol, Ethanol, Propanol, Isopropanol, Diethylketon, Dimethylformamid (DMF), Dimethylacetamid, Essigsäureethylester, Dimethylsulfoxid (DMSO), Benzol, Toluol, Xylol, t-Butylmethylether (MTBE), Petrolether (PE), Cyclohexan, Pentan, Hexan, Heptan, wobei Chloroform und Methylenchlorid besonders bevorzugt sind.

Eine weitere bevorzugte Ausführungsform der erfindungsgemäßen Stents weist drei Beschichtungen auf. Bei derartigen Dreischichtsystemen wird als erste Beschichtung diejenige bezeichnet, welche direkt auf den Stent aufgebracht ist. Als zweite Beschichtung wird diejenige Beschichtung bezeichnet, welche auf diese erste Beschichtung aufgebracht wird. Die Beschichtung, welche auf der zweiten Beschichtung aufgebracht ist, wird als dritte Beschichtung bezeichnet.

Gemäß der Dreischichtausführung besteht die erste Beschichtung aus einer reinen anorganischen Beschichtung umfassend Magnesiumfluorid, die von einer zweiten Beschichtung überzogen ist, die mindestens ein organisches Polymer enthält oder nur aus diesem Polymer besteht. Die zweite Beschichtung ist von einer dritten Beschichtung überzogen, die mindestens einen antiproliferativen, antiphlogistischen und/oder antithrombotischen Wirkstoff, d.h. einen Antirestenose-Wirkstoff enthält oder nur aus diesem Wirkstoff besteht.

Anstelle dieses Dreischichtaufbaus kann die äußere Wirkstoffschicht auch entfallen und der Wirkstoff kann in die organische Beschichtung eingelagert werden. Somit wird in einer weiteren bevorzugten Ausführungsform zuerst die anorganische Beschichtung auf den Stent aufgebracht, getrocknet, danach auf diese Beschichtung eine organische Beschichtung zusammen mit einem Wirkstoff aufgebracht und getrocknet.

Es ist natürlich auch möglich, den Wirkstoff als äußere dritte Schicht auf die Wirkstoff enthaltende mittlere organische Schicht aufzutragen. Dabei kann sich in der mittleren organischen Schicht derselbe Wirkstoff in einer anderen oder derselben Konzentration befinden oder es wird in der mittleren organischen Schicht ein anderer Wirkstoff oder auch kein Wirkstoff verwendet.

Eine bevorzugte Ausführungsform der Erfindung besteht also aus einem Stent bestehend aus einem Grundgerüst aus einer hierin offenbarten, bioabbaubaren Magnesiumlegierungen mit mindestens 80 Gew.-% Magnesium, einer anorganischen Beschichtung umfassend Magnesiumfluorid, einer organischen Beschichtung und einer polymeren Beschichtung, optional mindestens einem Antirestenose-Wirkstoff.

Wird der mindestens eine Wirkstoff bzw. die Wirkstoffkombination auf die polymere Beschichtung des Stents aufgebracht, können in Kombination mit dem mindestens einen Wirkstoff oder der Wirkstoffkombination weitere Substanzen als pharmakologisch verträgliche Träger bzw. als Matrix aufgebracht werden.

Als pharmakologisch verträgliche Träger können sowohl die oben bereits aufgelisteten Polymere dienen als auch niedermolekulare Substanzen, wie beispielsweise Lactose, Stärke, Natrium-Carboxymethylstärke, Sorbitol, Sucrose, Magnesiumstearat, Dicalciumphosphat, Calciumsulfat, Talk, Mannitol, Ethylalkohol, Polyvinylalkohole, Polyvinylpyrrolidon, Gelatine, natürliche Zucker, sowohl natürliche als auch synthetische Gummis wie beispielsweise Akaziengummi oder Guar-Gummi, Natriumalginat, Natriumbenzoat, Natriumacetat, Glyceride, Isopropylmyristate und palmitate, Citrate, wie Tributyl- und Triethylcitrate und deren Acetylderivate, Phtalate wie Dimethylphthalat oder Dibutylphthalat, etc., Benzoesäurebenzylester, Triacetin, 2-Pyrrolidon, Borsäure, Magnesium-Aluminum-Silicate, natürliche Johannisbrotkernmehl, Karaya, Guar, Tragacanth, Agar, Cellulose, Cellulosederivate wie Methylcellulose, Natrium-Carboxymethylcellulose, Hydroxypropyl-methylcellulose, microkristalline Cellulose sowie Alginate, Tonerden und Bentonite, Polyethylenglycol als auch Wachse wie z.B. Bienenwachs, Carnaubawachs, Candelillawachs und dergleichen eingesetzt werden. Die Matrixsubstanz der zweiten Schicht kann dabei mit dem Polymer der ersten Schicht identisch sein. Die zusätzlichen Träger- oder Matrixsubstanzen können in einem Gewichtsverhältnis von bis zu 70 Gew.-%, bevorzugt bis 50 Gew.-% bezogen auf den oder die verwendeten Wirkstoffe eingesetzt werden.

Die polymere Beschichtung wird mittels bekannten Verfahren wie beispielsweise Sprühverfahren, Tauchverfahren, Plasmaverfahren, Pinselverfahren, Spritzenverfahren, Elektrospinning oder Pipettierverfahren auf die anorganische Beschichtung, die bereits auf der Magnesiumlegierung des Grundgerüstes enthalten ist, aufgebracht und haftet daran auch vorzugsweise fest. Der erfindungsgemäße Stent kann also mittels Sprüh-, Pipettier-, Pinsel-, Spritzen-, Plasmaabscheidungs- oder Tauchverfahren, Elektrospinning beschichtet werden, wobei die polymere Substanz oder Mischungen der Substanzen in einem Lösungsmittel gelöst werden und diese Lösung auf das Implantat aufgetragen wird. Anschließend wird das Lösungsmittel oder das Lösungsmittelgemisch durch Verdunstung bei Raumtemperatur entfernt. Die Beschichtung der erfindungsgemäßen Stents kann sowohl vor als auch nach dem Crimpen auf einen Katheterballon durchgeführt werden. Sofern die Beschichtung erst aufgebracht wird, nachdem der Stent auf einem Katheterballon befestigt wurde, ist ein Tauch oder Sprühverfahren bevorzugt. Dabei kann auch der Katheterballon, eventuell bis über die Stentenden hinaus, beschichtet werden. Das Polymer kann auch in Form eines Schlauches vorgeformt werden und auf der äußeren bzw. inneren Oberfläche des mit der anorganischen Beschichtung versehenen Grundgerüsts der erfindungsgemäßen Stents aufgebracht werden. Sofern ein Schlauch aufgebracht wird oder die polymere Beschichtung als vollflächige Beschichtung, d.h. eine die Zwischenräume vollflächig überdeckende Beschichtung, aufgebracht wird, ist es bevorzugt, wenn diese polymere Beschichtung über die Länge des Stents oder der Gefäßstütze hinausgeht und nicht mit den Enden der Gefäßstütze abschließt. Die überstehenden Enden der Beschichtung werden in einem weiteren Schritt um die Ränder der Gefäßstütze zur Außenseite gelegt und die entstehenden Kanten unter Druck und erhöhter Temperatur in die darunterliegende Polymerschicht integriert. Damit ist eine verstärkte Beschichtung an den Stentenden gewährleistet und die Gefahr der Ablösung an diesen Schwachstellen ist verringert.

Die polymere Beschichtung sollte relativ gleichmäßig erfolgen und eine Schichtdicke von 0,01 bis 100 μm besitzen. Die gewünschte Schichtdicke hängt auch vom jeweiligen Polymer ab und kann durch mehrmalige Beschichtungsschritte unterbrochen von Trocknungsschritten realisiert werden. Durch die Beschichtungsdicke lässt sich die Dichtigkeit der polymeren Beschichtung einstellen. Insbesondere bei einer Abscheidung des Polymers aus einer Gasphase wird die Schicht bei längerer Beschichtungsdauer undurchlässig. Bei kurzen Beschichtungszeiten treten undichte Stellen auf, die die Diffusion von Wasser oder Gasen zulassen. Besonders bevorzugt sind Schichtdicken der polymeren Beschichtung von 0,01 bis 90 μm, weiter bevorzugt von 0,01 bis 80 μm, weiter bevorzugt von 0,01 bis 70 μm, weiter bevorzugt von 0,01 bis 60 μm, weiter bevorzugt von 0,01 bis 50 μm, weiter bevorzugt von 0,01 bis 40 μm, weiter bevorzugt von 0,01 bis 30 μm, weiter bevorzugt von 0,01 bis 20 μm, weiter bevorzugt von 0,01 bis 10 μm, noch mehr bevorzugt von 0,05 bis 10 μm, besonders bevorzugt von 0,1 bis 10 μm und am bevorzugtesten von 0,5 bis 10 μm.

Als Lösungsmittel eignen sich Wasser und bevorzugt organische Lösungsmittel wie beispielsweise Chloroform, Methylenchlorid (Dichlormethan), Aceton, Tetrahydrofuran (THF), Diethylether, Methanol, Ethanol, Propanol, Isopropanol, Diethylketon, Dimethylformamid (DMF), Dimethylacetamid, Essigsäureethylester, Dimethylsulfoxid (DMSO), Benzol, Toluol, Xylol, t-Butylmethylether (MTBE), Petrolether (PE), Cyclohexan, Pentan, Hexan, Heptan, wobei Chloroform und Methylenchlorid besonders bevorzugt sind.

In einem geeigneten Lösungsmittel oder auch zusammen mit dem Polymer kann auch der mindestens eine aufzubringende, antiinflammatorische, antiproliferative, antiangiogene, antirestenotische (anti-Restenose), antineoplastische, antimigrative und/oder antithrombogene Wirkstoff gelöst, emulgiert, suspendiert oder dispergiert werden. Sofern ein Polymer als Matrixsubstanz in der organischen Beschichtung enthalten ist, kann dieses Polymer zusammen mit dem Wirkstoff gelöst und aufgetragen werden, oder in einem Sprüh-, Pipettier- oder Tauchverfahren getrennt, bevorzugt zuvor, aufgetragen werden.

Bei einer bevorzugten Ausführungsform wird zuerst anorganische Beschichtung auf das Stentgerüst aufgebracht, dann wird die polymere Beschichtung auf die anorganische Beschichtung aufgebracht, getrocknet und anschließend auf die polymere Beschichtung ein Wirkstoff aufgebracht. Hierzu wird bevorzugt eine Lösung des mindestens einen Wirkstoffs und eventuell einer Trägersubstanz in einem leichtflüchtigen Lösungsmittel auf die polymere Beschichtung der Stents aufgebracht. Anschließend wird das Lösungsmittel oder das Lösungsmittelgemisch durch Verdunstung bei Raumtemperatur entfernt.

**Figurenbeschreibung**

**Figur 1** zeigt eine graphische Darstellung der Ergebnisse der Korrosionsversuche mit binären Magnesiumlegierungen enthaltend zwischen 5 und 20 % Dysprosium und den Rest Magnesium. Gemessen wurde die Korrosion in 0,9% iger Kochsalzlösung im Eudiometer. Die Angaben in % beziehen sich auf den Anteil an Dysprosium in Gew.-%.

**Figur 2** zeigt eine graphische Darstellung der Abhängigkeit der Tendenz zur Heißrissbildung im Zusammenhang der Menge an Zink in der Legierung. Getestet wurden Magnesiumlegierungen enthaltend 10% Dysprosium, 1,0 Gew.-% Neodym, steigende Gew.-%Zink, 0,2 Gew.-% Zirkonium und den Rest Magnesium. Die Angaben in % beziehen sich auf den Anteil an Zink in Gew.-%.

**Figur 3** zeigt einen Vergleich der Photometrischen Messungen zu den Degradationsgeschwindigkeiten des $O_2$-Ionen-implantierten Stents, unbeschichteten Stents aus der Magnesiumlegierung A (Bare Metal Stent (BMS)) und des

Magnesiumfluorid-beschichteten Stents aus der Magnesiumlegierung A. Dabei sind die gelösten Massen an Magnesium in PBS über die Zeit aufgetragen. Bei der Probe A handelt es sich um einen Stent aus der Magnesiumlegierung A (Mg10Dy1Nd1Zn0,2Zr) ohne weitere Behandlung. Abgebildet sind die Durchschnittswerte an gelöstem Magnesium über die Zeit von drei gleichen Stents der Probe A.

Bei der Probe B handelt es sich um einen Stent aus der Magnesiumlegierung A (Mg10Dy1Nd1Zn0,2Zr) und anschließender Sauerstoff-Ionenimplantation. Bei der Ionenimplantation werden Sauerstoffionen in die Oberfläche geschossen (implantiert), um eine Magnesiumoxidschicht auszubilden.

Bei der Probe C handelt es sich um einen Stent aus der Magnesiumlegierung A (Mg10Dy1Nd1Zn0,2Zr) und anschließender Oberflächenumwandlung, wobei eine Schicht aus Magnesiumfluorid ($MgF_2$) entsteht. Abgebildet sind die Durchschnittswerte an gelöstem Magnesium über die Zeit von drei gleichen Stents der Probe C.

**Figur 4** zeigt Kameraaufnahmen zur Degradation von einzelnen Stents der Proben A, B und C gemäß Figur 3 und zugehöriger Beschreibung.

In der ersten Zeile ist ein Stent der Probe B (Magnesiumlegierung A (Mg10Dy1Nd1Zn0,2Zr) und anschließende Sauerstoff-Ionenimplantation) abgebildet. Das erste Bild (von links) ist zum Start des Degradationstests aufgenommen. Das zweite Bild zeigt den Stent nach 24 Stunden und das dritte Bild zeigt den Stent nach 36 Stunden im PBS durchflossenen Schlauch.

In der zweiten Zeile ist ein Stent der Probe A (Magnesiumlegierung A (Mg10Dy1Nd1Zn0,2Zr) ohne anschließende Weiterbehandlung) abgebildet. Das erste Bild (von links) ist zum Start des Degradationstests aufgenommen. Das zweite Bild zeigt den Stent nach zwei Stunden und das dritte Bild zeigt den Stent nach sechs Stunden im PBS durchflossenen Schlauch.

In der dritten Zeile ist ein Stent der Probe C (Magnesiumlegierung A (Mg10Dy1Nd1Zn0,2Zr) und anschließend Oberflächenumwandlung zur Erzeugung von $MgF_2$) abgebildet. Das erste Bild (von links) ist zum Start des Degradationstests aufgenommen. Das zweite Bild zeigt den Stent nach 13 Stunden und das dritte Bild zeigt den Stent nach 24 Stunden im PBS durchflossenen Schlauch.

**Figur** 5 zeigt einen Vergleich der Photometrischen Messungen zu den Degradationsgeschwindigkeiten von $MgF_2$-, $MgCO_3$- und $Mg_3(PO_4)_2$-beschichteten Stents. Dabei sind die gelösten Massen an Magnesium in PBS über die Zeit aufgetragen. Es wurden jeweils drei Stents mit der gleichen Beschichtung vermessen und die Durchschnittswerte dieser Messungen sind in Figur 5 dargestellt. Bei der Probe A handelt es sich um einen Stent aus der Magnesiumlegierung A (Mg10Dy1Nd1Zn0,2Zr) mit einer Oberflächenumwandlung zur Erzeugung einer Magnesiumphosphatschicht ($Mg_3(PO_4)_2$). Zur Erzeugung dieser Schicht wurde der Stent für 24 Stunden unter 50°C in eine 10%ige Natriumphosphatlösung (50 ml) getaucht. Der Behälter mit der Natriumphosphatlösung schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Durchschnittswerte an gelöstem Magnesium über die Zeit von drei gleichen Stents der Probe A.

Bei der Probe B handelt es sich um einen Stent aus der Magnesiumlegierung A (Mg10Dy1Nd1Zn0,2Zr) mit einer Oberflächenumwandlung zur Erzeugung einer Magnesiumfluoridschicht ($MgF_2$). Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Durchschnittswerte an gelöstem Magnesium über die Zeit von drei gleichen Stents der Probe B.

Bei der Probe C handelt es sich um einen Stent aus der Magnesiumlegierung A (Mg10Dy1Nd1Zn0,2Zr) mit einer Oberflächenumwandlung zur Erzeugung einer Magnesiumcarbonatschicht ($MgCO_3$). Zur Erzeugung dieser Schicht wurde der Stent für 26 Stunden unter 50°C in eine alkalische 10%ige Natriumcarbonatlösung (50 ml) getaucht. Zur Alkalisierung der Lösung wurde Natriumhydroxid zugegeben. Der Behälter mit der Natriumcarbonatlösung schwamm wiederum in einem beheizten Wasserbad, um die Temperatur von 50°C zu gewährleisten. Nach den 26 Stunden wurde der Stent entnommen und mit deionisiertem Wasser abgespült und anschließend an Luft getrocknet. Abgebildet sind die Durchschnittswerte an gelöstem Magnesium über die Zeit von drei gleichen Stents der Probe B.

Bei der Probe D handelt es sich um einen Stent aus der Magnesiumlegierung A (Mg10Dy1Nd1Zn0,2Zr) ohne weitere Behandlung. Abgebildet sind die Durchschnittswerte an gelöstem Magnesium über die Zeit von drei gleichen Stents der Probe D.

**Figur** 6 bildet einzelne Stents der Proben A, B, C und D gemäß Figur 5 und zugehöriger Beschreibung ab.

In der ersten Zeile ist ein Stent der Probe B (Magnesiumlegierung A (Mg10Dy1Nd1Zn0,2Zr) und anschließende $MgF_2$ Oberflächenumwandlung) abgebildet. Das erste Bild (von links) ist zum Start des Degradationstests aufgenommen. Das zweite Bild zeigt den Stent nach 10 Stunden und das dritte Bild zeigt den Stent nach 20 Stunden im PBS durchflossenen Schlauch. In der zweiten Zeile ist ein Stent der Probe A (Magnesiumlegierung A (Mg10Dy1Nd1Zn0,2Zr) und anschließende $Mg_3(PO_4)_2$ Oberflächenumwandlung) abgebildet. Das erste Bild (von links) ist zum Start des Degradationstests aufgenommen. Das zweite Bild zeigt den Stent nach zehn Stunden und das dritte Bild zeigt den Stent nach 20 Stunden im PBS durchflossenen Schlauch. In der dritten Zeile ist ein Stent der Probe C (Magnesiumlegierung A (Mg10Dy1Nd1Zn0,2Zr) und anschließende $MgCO_3$ Oberflächenumwandlung) abgebildet. Das erste Bild (von links) ist zum Start des Degradationstests aufgenommen. Das zweite Bild zeigt den Stent nach vier Stunden und das dritte Bild zeigt den Stent nach zehn Stunden im PBS durchflossenen Schlauch. In der vierten Zeile ist ein Stent der Probe D (Magnesiumlegierung A (Mg10Dy1Nd1Zn0,2Zr) ohne weitere Behandlung) abgebildet. Das erste Bild (von links) ist zum Start des Degradationstests aufgenommen. Das zweite Bild zeigt den Stent nach vier Stunden und das dritte Bild zeigt den Stent nach zehn Stunden im PBS durchflossenen Schlauch.

**Figur 7** zeigt einen Vergleich der Photometrischen Messungen zu den Degradationsgeschwindigkeiten von $MgF_2$-, $MgCO_3$- und $Mg_3(PO_4)_2$-beschichteten Stents sowie nach einer Wärmebehandlung, nach dem Glühen oder nach einer Behandlung mittels Sauerstoff Plasmaverfahren. Dabei sind die gelösten Massen an Magnesium in PBS über die Zeit aufgetragen. Es sind jeweils die Durchschnittswerte aus zwei Messungen (n=2) abgebildet.

Bei der Probe A handelt es sich um einen Stent aus der Magnesiumlegierung A (Mg10Dy1Nd1Zn0,2Zr) mit einer Oberflächenumwandlung zur Erzeugung einer Magnesiumfluoridschicht ($MgF_2$). Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Daraufhin wurde der Stent für 24,5 Stunden an Luftatmosphäre geglüht. Abgebildet sind die Durchschnittswerte an gelöstem Magnesium über die Zeit von zwei gleichen Stents der Probe A.

Bei der Probe B handelt es sich um einen Stent aus der Magnesiumlegierung A (Mg10Dy1Nd1Zn0,2Zr) mit einer Plasmabehandlung zur Erzeugung einer Magnesiumoxidschicht (MgO). Zur Erzeugung dieser Schicht wurde der Stent für 1,5 Stunden in ein Sauerstoffplasma gelegt. Das Plasma soll die Oberfläche des Stents oxidieren, sodass eine MgO Schicht entsteht. Anschließend wurde der Stent für 24,5 Stunden an Luftatmosphäre geglüht. Abgebildet sind die Durchschnittswerte an gelöstem Magnesium über die Zeit von zwei gleichen Stents der Probe B.

Bei der Probe C handelt es sich um einen Stent aus der Magnesiumlegierung A (Mg10Dy1Nd1Zn0,2Zr) mit einer Oberflächenumwandlung zur Erzeugung einer Magnesiumcarbonatschicht ($MgCO_3$). Zur Erzeugung dieser Schicht wurde der Stent für 24 Stunden bei Raumtemperatur in eine alkalische 10%ige Natriumcarbonatlösung (50 ml) getaucht. Zur Alkalisierung der Lösung wurde Natriumhydroxid zugegeben. Nach den 24 Stunden wurde der Stent entnommen und mit deionisiertem Wasser abgespült und anschließend an Luft getrocknet. Daraufhin wurde der Stent für 24,5 Stunden an Luftatmosphäre geglüht. Abgebildet sind die Durchschnittswerte an gelöstem Magnesium über die Zeit von zwei gleichen Stents der Probe C.

Bei der Probe D handelt es sich um einen Stent aus der Magnesiumlegierung A (Mg10Dy1Nd1Zn0,2Zr) mit einer Oberflächenumwandlung zur Erzeugung einer Magnesiumphosphatschicht ($Mg_3(PO_4)_2$). Zur Erzeugung dieser Schicht wurde der Stent für vier Tage bei Raumtemperatur in eine 10%ige Natriumphosphatlösung (50 ml) getaucht. Nach den vier Stunden wurde der Stent entnommen und mit deionisiertem Wasser abgespült und anschließend an Luft getrocknet. Daraufhin wurde der Stent für 24,5 Stunden an Luftatmosphäre geglüht. Abgebildet sind die Durchschnittswerte an gelöstem Magnesium über die Zeit von zwei gleichen Stents der Probe D.

Bei der Probe E handelt es sich um einen Stent aus der Magnesiumlegierung A (Mg10Dy1Nd1Zn0,2Zr) mit einer Plasmabehandlung zur Erzeugung einer Magnesiumoxidschicht (MgO) und anschließender Oberflächenumwandlung zur Erzeugung einer $MgF_2$ Schicht. Zur Erzeugung dieser Schicht wurde der Stent für 1,5 Stunden in ein Sauerstoffplasma gelegt. Das Plasma soll die Oberfläche des Stents oxidieren, sodass eine MgO Schicht entsteht. Anschließend wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Durchschnittswerte an gelöstem Magnesium über die Zeit von zwei gleichen Stents der Probe E.

Bei der Probe F handelt es sich um einen Stent aus der Magnesiumlegierung A (Mg10Dy1Nd1Zn0,2Zr) ohne weiterer Behandlung. Abgebildet sind die Durchschnittswerte an gelöstem Magnesium über die Zeit von drei gleichen Stents der Probe F.

**Figur 8** zeigt die Degradationsgeschwindigkeit im PBS für einen geglühten $MgF_2$-beschichteten Stents und einem geglühten und Plasma-behandelten Stent.

Dabei sind die gelösten Massen an Magnesium in PBS, die photometrisch bestimmt werden, über die Zeit aufgetragen.

Bei den Proben A und B handelt es sich um jeweils einen Stent aus der Magnesiumlegierung A ($Mg_{10}Dy_1Nd_1Zn_{0,2}Zr$) mit einer Oberflächenumwandlung zur Erzeugung einer Magnesiumfluoridschicht ($MgF_2$). $MgF_2$ Schicht bei 50°C mit Flusssäure und dann geglüht.

Bei den Proben C und D handelt es sich um jeweils einen Stent aus der Magnesiumlegierung A ($Mg_{10}Dy_1Nd_1Zn_{0,2}Zr$) mit einer Plasmabehandlung zur Erzeugung einer Magnesiumoxidschicht (MgO) und anschließend geglüht.

**Figur 9** zeigt die Degradationsgeschwindigkeit im PBS für $MgF_2$-beschichteter Stents, die wärmebehandelt, geglüht oder Plasma-behandelt sind. Dabei sind die gelösten Massen an Magnesium in PBS, die photometrisch bestimmt

werden, über die Zeit aufgetragen.

Bei der Probe A handelt es sich um einen Stent aus der Magnesiumlegierung A (Mg10Dy1Nd1Zn0,2Zr) mit einer Plasmabehandlung zur Erzeugung einer Magnesiumoxidschicht (MgO) und anschließender Oberflächenumwandlung zur Erzeugung einer $MgF_2$ Schicht. Zur Erzeugung dieser Schicht wurde der Stent für 1,5 Stunden in ein Sauerstoffplasma gelegt. Das Plasma soll die Oberfläche des Stents oxidieren, sodass eine MgO Schicht entsteht. Anschließend wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Durchschnittswerte an gelöstem Magnesium über die Zeit von zwei gleichen Stents der Probe A.

Bei der Probe B handelt es sich um einen Stent aus der Magnesiumlegierung A (Mg10Dy1Nd1Zn0,2Zr) mit einer Oberflächenumwandlung zur Erzeugung einer Magnesiumfluoridschicht ($MgF_2$). Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Daraufhin wurde der Stent für 24,5 Stunden an Luftatmosphäre geglüht. Abgebildet sind die Durchschnittswerte an gelöstem Magnesium über die Zeit von zwei gleichen Stents der Probe B.

Bei der Probe C handelt es sich um einen Stent aus der Magnesiumlegierung A (Mg10Dy1Nd1Zn0,2Zr) und anschließender Oberflächenumwandlung, wobei eine Schicht aus Magnesiumfluorid ($MgF_2$) entsteht. Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Durchschnittswerte an gelöstem Magnesium über die Zeit von drei gleichen Stents der Probe C.

**Figur 10** zeigt die Degradationsgeschwindigkeit im PBS für $MgF_2$-beschichteter Stents. Die Beschichtung wird einerseits unter Einwirkung von Ammoniumfluorid für 5 bzw. 24h hergestellt. Andererseits lässt man 40% Flusssäure für 5 sowie 24h auf den Stent einwirken, um die Beschichtung zu erzeugen. Dabei sind die gelösten Massen an Magnesium in PBS, die photometrisch bestimmt werden, über die Zeit aufgetragen. A = $MgF_2$-beschichteter Stent durch Behandlung mit Flusssäure für 24h; B = $MgF_2$-beschichteter Stent durch Behandlung mit Ammoniumfluorid für 24h; C = $MgF_2$-beschichteter Stent durch Behandlung mit Flusssäure für 5h; B = $MgF_2$-beschichteter Stent durch Behandlung mit Ammoniumfluorid für 5h.

Bei der Probe A handelt es sich um einen Stent aus der Magnesiumlegierung A (Mg10Dy1Nd1Zn0,2Zr) und anschließender Oberflächenumwandlung, wobei eine Schicht aus Magnesiumfluorid ($MgF_2$) entsteht. Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Messwerte an gelöstem Magnesium über die Zeit von einem Stent der Probe A.

Bei der Probe B handelt es sich um einen Stent aus der Magnesiumlegierung A (Mg10Dy1Nd1Zn0,2Zr) und anschließender Oberflächenumwandlung, wobei eine Schicht aus Magnesiumfluorid ($MgF_2$) entsteht. Zur Erzeugung dieser Schicht wurde der Stent für 24 Stunden unter 50°C in eine 10%ige Ammoniumfluoridlösung (50 ml) getaucht. Der Behälter mit der Lösung schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Messwerte an gelöstem Magnesium über die Zeit von einem Stent der Probe B.

Bei der Probe C handelt es sich um einen Stent aus der Magnesiumlegierung A (Mg10Dy1Nd1Zn0,2Zr) und anschließender Oberflächenumwandlung, wobei eine Schicht aus Magnesiumfluorid ($MgF_2$) entsteht. Zur Erzeugung dieser Schicht wurde der Stent für fünf Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den fünf Stunden wurde der Stent entnommen und mit deionisiertem Wasser abgespült und anschließend an Luft getrocknet. Abgebildet sind die Messwerte an gelöstem Magnesium über die Zeit von einem Stent der Probe C.

Bei der Probe D handelt es sich um einen Stent aus der Magnesiumlegierung A (Mg10Dy1Nd1Zn0,2Zr) und anschließender Oberflächenumwandlung, wobei eine Schicht aus Magnesiumfluorid ($MgF_2$) entsteht. Zur Erzeugung dieser Schicht wurde der Stent für fünf Stunden unter 50°C in eine 10%ige Ammoniumfluoridlösung (50 ml) getaucht. Der Behälter mit der Lösung schwamm wiederum in einem beheizten Wasserbad, um die Temperatur von 50°C zu gewährleisten. Nach den fünf Stunden wurde der Stent entnommen und mit deionisiertem Wasser abgespült und anschließend an Luft getrocknet. Abgebildet sind die Messwerte an gelöstem Magnesium über die Zeit von einem Stent der Probe D.

**Figur 11** zeigt die Degradationsgeschwindigkeit von Magnesiumlegierungen, die mittels Ionenimplantation (Sauerstoff, Fluor und Kohlenstoff) behandelt wurden.

Bei der Probe A handelt es sich um einen Stent der Magnesiumlegierung A (Mg10Dy1Nd1Zn0,2Zr) mit einer Sauerstoff-Implantation.

Bei der Probe B handelt sich um einen Stent der Magnesiumlegierung A (Mg10Dy1Nd1Zn0,2Zr) mit einer Fluor-Implantation.

Bei der Probe C handelt es sich um einen Stent aus der unbeschichteten Magnesiumlegierung A (Mg10Dy1Nd1Zn0,2Zr).

**Figur 12** zeigt die Degradationsgeschwindigkeit von Stents nach zusätzlicher Wärmebehandlung und anschließender Fluoridierung.

Gruppe A: Zuvor zusätzlich wärmebehandelte Stents aus der Magnesiumlegierung A wurden mit 38-40% Flusssäure behandelt.

Gruppe B: Zuvor wärmehandelte Stents aus der Magnesiumlegierung A wurden und 48% Flusssäure behandelt.

Gruppe C: Unbehandelte Stents der Magnesiumlegierung A (Mg10Dy1Nd1Zn0,2Zr) mit gleichem Design und gleicher Material-Lot wie Gruppe A und B.

**Figur 13** zeigt die Degradationsgeschwindigkeit von Stents mit einer Zwischenschicht aus Magnesiumfluorid und einer weiteren Beschichtung aus Parylen C.

Probe A: Stents aus der Magnesiumlegierung A mit Flusssäure behandelt und anschließend mit Polymer Parylen C beschichtet.

Probe B: Stents mit Polymer Parylen C beschichtet, aber keine Fluoridierung vorgenommen.

**Figur 14** Degradationstests von Stents mit einer Zwischenschicht aus Magnesiumfluorid und einer weiteren Beschichtung aus einem resorbierbaren Polymer

Gruppe A: Stents der Magnesiumlegierung A (Mg10Dy1Nd1Zn0,2Zr) mit einer Schicht aus Poly-l-lactid (PLLA), welche im Sprühverfahren auf den Stent gebracht wurde.

Gruppe B: Stents der Magnesiumlegierung A (Mg10Dy1Nd1Zn0,2Zr) mit einer Schicht aus Poly-ε-caprolacton (PCL), welche im Sprühverfahren auf den Stent gebracht wurde.

Gruppe C: Stents der Magnesiumlegierung A (Mg10Dy1Nd1Zn0,2Zr) mit einer Schicht aus Poly-L-Lactid (PLLA), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

Gruppe D: Stents der Magnesiumlegierung A (Mg10Dy1Nd1Zn0,2Zr) mit einer Schicht aus Poly-ε-caprolacton (PCL), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

**Figur 15** zeigt die Degradationsgeschwindigkeit von Stents der Magnesiumlegierung A (Mg10Dy1Nd1Zn0,2Zr) mit einer Zwischenschicht aus Magnesiumfluorid und einer weiteren Beschichtung aus bioresorbierbarem Polymer (PCL) unterschiedlicher Schichtdicke und eingebrachtem Antirestenose-Wirkstoff (Rapamycin).

Gruppe A: Stents der Magnesiumlegierung A (Mg10Dy1Nd1Zn0,2Zr) mit einer 4 μm Schicht aus Poly-ε-Caprolacton (PCL), welche im Sprühverfahren auf den Stent gebracht wurde.

Gruppe B: Stents der Magnesiumlegierung A (Mg10Dy1Nd1Zn0,2Zr) mit einer 7 μm Schicht aus Poly-ε-Caprolacton (PCL), welche im Sprühverfahren auf den Stent gebracht wurde.

Gruppe C: Stents der Magnesiumlegierung A (Mg10Dy1Nd1Zn0,2Zr) mit einer 4 μm Schicht aus Poly-ε-Caprolacton (PCL), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

Gruppe D: Stents der Magnesiumlegierung A (Mg10Dy1Nd1Zn0,2Zr) mit einer 7 μm Schicht aus Poly-ε-Caprolacton (PCL), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

**Figur 16** zeigt die Degradationsgeschwindigkeit von Stents der Magnesiumlegierung A (Mg10Dy1Nd1Zn0,2Zr) mit und ohne einer Zwischenschicht aus Magnesiumfluorid und einer weiteren Beschichtung aus bioresorbierbarem Polymer (PLLA) und eingebrachtem Antirestenose-Wirkstoff (Rapamycin).

Gruppe A: Stents der Magnesiumlegierung A (Mg10Dy1Nd1Zn0,2Zr) mit einer 7 μm Schicht aus Poly-L-Lactid (PLLA) mit eingebrachtem Medikament (Rapamycin), welche im Sprühverfahren aufgebracht wurde und einer Zwischenschicht aus Magnesiumfluorid.

Gruppe B: Stents der Magnesiumlegierung A (Mg10Dy1Nd1Zn0,2Zr) mit einer 7 μm Schicht aus Poly-L-Lactid (PLLA) mit eingebrachtem Medikament (Rapamycin), welche im Sprühverfahren auf den Stent gebracht wurde.

**Figur 17:** zeigt die Degradationsgeschwindigkeit von Stents der Magnesiumlegierung A (Mg10Dy1Nd1Zn0,2Zr) mit einer anorganischen Zwischenschicht und einer weiteren Beschichtung aus PLLA (bei unterschiedlicher Zusammensetzung der anorganischen Zwischenschicht.

Gruppe A: Stents der Magnesiumlegierung A (Mg10Dy1Nd1Zn0,2Zr) mit einer anorganischen Beschichtung (Oberflächenumwandlung) aus Magnesiumhydroxid und einer 5 μm Schicht aus Poly-L-Lactid (PLLA), die im Sprühverfahren aufgebracht wurde. Die Magnesiumhydroxid Oberfläche wurde in einem nasschemischen auf die Stents gebracht. Dazu wurden die polierten Stents für 2 min in 30%ige $H_2O_2$ Lösung bei Raumtemperatur getaucht. An-

schließend wurden diese mit $H_2O$ gespült und in Ethanol getaucht und bei 80 °C für eine Stunde in einem Trockenschrank getrocknet.

Gruppe B: Stents der Magnesiumlegierung A (Mg10Dy1Nd1Zn0,2Zr) mit einer anorganischen Beschichtung (Oberflächenumwandlung) aus Magnesiumcarbonat/ Magnesiumhydroxid und einer 5 μm Schicht aus Poly-L-Lactid (PLLA), die im Sprühverfahren aufgebracht wurde. Die Die Oberflächenumwandlung wurde mittels eines nasschemischen Verfahrens realisiert. Dabei wurden wie polierten Stents in gesättigte $NaHCO_3$-Lösung für 5 min und 37 °C getaucht. Anschließend wurden die Stents mit $H_2O$ gespült und in Ethanol getaucht. Darauf folgte die Trocknung bei 100 °C für eine Stunde im Trockenschrank.

Gruppe C: Stents der Magnesiumlegierung A (Mg10Dy1Nd1Zn0,2Zr) mit einer anorganischen Beschichtung (Oberflächenumwandlung) aus Magnesiumphosphat/ Magnesiumhydroxid und einer 10 μm Schicht aus Poly-L-Lactid (PLLA), die im Sprühverfahren aufgebracht wurde.

Die Oberflächenumwandlung wurde mittels eines nasschemischen Verfahrens realisiert. Dabei wurden wie polierten Stents in gesättigte $Na_2HPO_4$-Lösung für eine Stunde und 37 °C getaucht. Anschließend wurden die Stents mit $H_2O$ gespült und in Ethanol getaucht. Darauf folgte die Trocknung bei 100 °C für eine Stunde im Trockenschrank.

Gruppe D: Stents der Magnesiumlegierung A (Mg10Dy1Nd1Zn0,2Zr) mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe A) auf die Stents gebracht.

Gruppe E: Stent der Magnesiumlegierung A (Mg10Dy1Nd1Zn0,2Zr) mit einer anorganischen Beschichtung (Oberflächenumwandlung) aus Magnesiumfluorid. Die Magnesiumfluorid-Schicht wurde wie in Beispiel 13 (Gruppe A) auf die Stents gebracht.

**Figur 18** zeigt die Degradationsgeschwindigkeit von Stents der Magnesiumlegierung A (Mg10Dy1Nd1Zn0,2Zr) mit und ohne einer anorganischen Zwischenschicht und einer weiteren Beschichtung aus Parylen N.

Gruppe A: Zwei Stents der Magnesiumlegierung A (Mg10Dy1Nd1Zn0,2Zr) wurden wie in Beispiel 13 Gruppe B behandelt, um eine Magnesiumfluoridschicht zu erhalten. Anschließend wurden die Stents mit dem Polymer Parylen N (Poly-p-xylylen) beschichtet. Parylen N kann direkt aus der Gasphase durch Kondensation auf dem Substrat abgeschieden werden, was zu einer sehr gleichmäßigen Beschichtung führt. Über die Behandlungsdauer lässt sich die Schichtdicke variieren. Gruppe B: Die zwei Stents der Gruppe B wurden direkt (also ohne eine Zwischenschicht aus Magnesiumfluorid) mit dem Polymer Parylen N beschichtet. Die Schichtdicke des Polymers war gleich, da diese im selben Beschichtungszyklus beschichtet wurden wie die Stents der Gruppe A.

Gruppe C: Die zwei Stents wurden behandelt wie in Gruppe A, allerdings wurde kein Polymer appliziert.

Gruppe D: Die drei Stents wiesen keinerlei Beschichtung auf (keine Magnesiumfluorid-Schicht und keine Polymer-Schicht).

**Figur 19:** zeigt die Degradationsgeschwindigkeit von Stents der Magnesiumlegierung A (Mg10Dy1Nd1Zn0,2Zr) mit und ohne einer anorganischen Zwischenschicht und einer weiteren Doppelbeschichtung aus Polyethylenimin (PEI) und Polyacrylsäure (PAA).

Gruppe A: Ein Stent der Magnesiumlegierung A (Mg10Dy1Nd1Zn0,2Zr) und einer Schicht aus PEI und PAA. Die Doppelschicht aus PEI und PAA wurde im Layer-by-Layer Verfahren (Schicht für Schicht) aufgebracht. Der Stent wurde nacheinander in eine wässrige Lösung mit 5 mg/ml PAA für zwei Minuten getaucht, anschließend für eine Minute in deionisiertes Wasser, daraufhin für zwei Minuten in eine wässrige Lösung mit 5 mg/ml PEI und abschließend für eine Minute in deionisiertes Wasser getaucht. Diese Abfolge wurde insgesamt fünf Mal wiederholt und der Stent daraufhin an Luft getrocknet.

Gruppe B: Ein Stent der Magnesiumlegierung A (Mg10Dy1Nd1Zn0,2Zr), welcher wie in Beispiel 13 Gruppe B behandelt wurde um eine Zwischenschicht aus Magnesiumfluorid zu erzeugen. Anschließend folgte die gleiche Beschichtungsabfolge wie bei Stent A (5x PEI und PAA).

Gruppe C: Gleichartig beschichteter Stent wie Stent A allerdings mit 10 Beschichtungsdurchgängen zur Erzeugung der PEI- und PAA-Schicht.

Gruppe D: Gleichartig beschichteter Stent wie Stent B allerdings mit 10 Beschichtungsdurchgängen zur Erzeugung der PEI- und PAA-Schicht.

Gruppe E: Stents der Magnesiumlegierung A (Mg10Dy1Nd1Zn0,2Zr) ohne jegliche weitere Beschichtung.

**Figur 20** zeigt die Degradationsgeschwindigkeit von Stents der Magnesiumlegierung A (Mg10Dy1Nd1Zn0,2Zr) mit und ohne einer anorganischen Zwischenschicht und einer weiteren Beschichtung aus Poly-L-Lactid (PLLA) im Vergleich zu gleich beschichteten Stents der Legierungen L37 und AZ91 (Mg9Al1Zn).

Gruppe A: Stents der Magnesiumlegierung A (Mg10Dy1Nd1Zn0,2Zr) mit einer 5 μm Schicht aus Poly-L-Lactid (PLLA), die im Sprühverfahren aufgebracht wurde.

Gruppe B: Stents der Magnesiumlegierung A (Mg10Dy1Nd1Zn0,2Zr) mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe B) auf die Stents gebracht.

Gruppe C: Stents der Magnesiumlegierung L37 mit einer 5 μm Schicht aus Poly-L-Lactid (PLLA), die im

Sprühverfahren aufgebracht wurde.

Gruppe B: Stents der Magnesiumlegierung L37 mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe B) auf die Stents gebracht.

Gruppe E: Stents der Magnesiumlegierung AZ91 mit einer 5 μm Schicht aus Poly-L-Lactid (PLLA), die im Sprühverfahren aufgebracht wurde.

Gruppe F: Stents der Magnesiumlegierung AZ91 mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe B) auf die Stents gebracht.

**Figur 21** zeigt die Degradationsgeschwindigkeit von Stents der Magnesiumlegierung A (Mg10Dy1Nd1Zn0,2Zr) mit und ohne einer anorganischen Zwischenschicht aus Magnesiumfluorid und einer weiteren Beschichtung aus Poly(lactid-co-glycolid).

Gruppe A: Stents der Magnesiumlegierung A (Mg10Dy1Nd1Zn0,2Zr) mit einer Schicht aus Poly(lactid-co-glycolid) (PLGA), die mittels Dip-Coating aufgebracht wurde. Dabei wurde der Stent in eine Lösung aus PLGA (85:15) und Trichlormethan (5 mg/ml) getaucht, mit 20 mm/min aus der Lösung gezogen und anschließend bei 40°C an Luft getrocknet.

Gruppe B: Stents der Magnesiumlegierung A (Mg10Dy1Nd1Zn0,2Zr) mit einer mit einer anorganischen Beschichtung (Oberflächenumwandlung) aus Magnesiumfluorid und einer darüberliegenden Schicht aus PLGA. Die Magnesiumfluorid Beschichtung wurde wie in Beispiel 13 Gruppe B aufgebracht. Die PLGA Beschichtung erfolgte wie bei der Gruppe A.

Gruppe C: Stents der Magnesiumlegierung A (Mg10Dy1Nd1Zn0,2Zr) ohne jegliche Beschichtung.

**Figur 22** zeigt die Degradationsgeschwindigkeit von Stents der Magnesiumlegierung A (Mg10Dy1Nd1Zn0,2Zr) mit und ohne einer anorganischen Zwischenschicht aus Magnesiumfluorid und einer weiteren Beschichtung aus Polymethacrylamide (PMAA) im Vergleich zu unbeschichteten Stents.

Gruppe A: Drei Stents der Magnesiumlegierung A (Mg10Dy1Nd1Zn0,2Zr) mit einer Schicht aus PMAA, die mittels Dip-Coating aufgebracht wurde (A1, A2, A3).

Gruppe B: Stents der Magnesiumlegierung A (Mg10Dy1Nd1Zn0,2Zr) mit einer anorganischen Beschichtung (Oberflächenumwandlung) aus Magnesiumfluorid und einer darüberliegenden Schicht aus PMAA (B1, B2, B3).

Gruppe C: Stents der Magnesiumlegierung A (Mg10Dy1Nd1Zn0,2Zr) ohne jegliche Beschichtung.

**Figur 23** zeigt die Degradationsgeschwindigkeit von Stents der Magnesiumlegierung A (Mg10Dy1Nd1Zn0,2Zr) mit und ohne einer anorganischen Zwischenschicht aus Magnesiumfluorid und einer weiteren Beschichtung aus Poly-L-Lactid (PLLA) mit eingebrachtem Medikament (Rapamycin, 1,4 μg/mm$^2$) im Vergleich zu Stents aus der Legierung L37 und einer Beschichtung aus PLLA sowie eingebrachten Medikament (Sirolimus, 1,4 μg/mm$^2$).

Gruppe A: Zwei Stents (A1 und A2) der Magnesiumlegierung L37 mit einer Beschichtung aus PLLA und einer abluminalen Schichtdicke von 6-12 μm.

Gruppe B: Zwei Stents (B1 und B2) der Magnesiumlegierung A mit einer Beschichtung aus 7,5 μm PLLA. Die Aufbringung der PLLA Beschichtung erfolgte im Sprühverfahren.

Gruppe C: Zwei Stents (C1 und C2) der Magnesiumlegierung A mit einer anorganischen Beschichtung (Oberflächenumwandlung) aus Magnesiumfluorid und einer darüberliegenden Schicht aus 7,5 μm PLLA. Die Magnesiumfluorid Beschichtung wurde wie in Beispiel 13 Gruppe B aufgebracht. Die Aufbringung der PLLA Beschichtung erfolgte im Sprühverfahren.

## Beispiele

### Beispiel 1: Herstellung der Legierungen

Die Legierungen wurden im sogenannten "Tütengussverfahren" hergestellt. Dieses Verfahren dient der Herstellung von Vormaterial für das spätere Strangpressen und zeichnet sich dadurch aus, dass das Material mit homogener Mikrostruktur und einer homogen Verteilung von Legierungselementen im Gussblock hergestellt werden kann. Damit eignet es außerordentlich dafür, kleinere Mengen hochwertige Bolzen für die Umformung herzustellen.

Bei diesem Verfahren werden die Magnesiumlegierungen (L1, L2, ... L44) in einem geschlichteten Stahltiegel erschmolzen. Als Tiegelwerkstoff kann praktisch jeder nickelfreie Stahl verwendet werden. Graphit wäre eine weitere Möglichkeit. Alle Schmelzoperationen finden unter Schutzgas statt. Die Schmelzbadtemperaturen liegen dabei im Bereich von 660-740°C. Bei Erreichen der Schmelzbadtemperatur wurden die Legierungselemente in Form von reinen Elementen oder als Masterlegierungen zugegeben. Nach der Zugabe der Legierungselemente wurde die Schmelze mechanisch gerührt. Die Rührzeit ist davon abhängig, wie lange die Elemente oder Masterlegierungen brauchen, um sich vollständig in der Schmelze aufzulösen. Nach dieser Vorbereitung wurde die Schmelze in eine dünnwandige Kokille überführt, die auf eine Temperatur von 600°C vorgeheizt wurde. Nach einer Dauer von ca. 60 Minuten wurde die Kokille in

ein Wasserbad eingetaucht, das eine Temperatur von 15-20°C aufwies. Durch das Eintauchen erstarrte die Kokille komplett.

Vor dem Strangpressen wurde die Oberfläche des Gussteils auf den Durchmesser des Rezipienten der Strangpresse abgedreht. Zudem wurde vor dem Strangpressen der Bolzen auf eine Temperatur von 250-500°C aufgeheizt und für 3 - 6 Stunden bei dieser Temperatur gehalten, um intermetallische Phasen aufzulösen bzw. Seigerungen zu homogenisieren. Im Anschluss daran erfolgte das Strangpressen und der so hergestellte Strang wurde an der Luft bis auf Raumtemperatur abgekühlt. Es wurden Drähte erhalten, welche danach in Rohre umgeformt wurden.

Die folgenden Legierungen wurden hergestellt:

Legierung L1:

| | |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Verunreinigungen umfassend Si, Ni, Fe, Cu sowie weitere Metalle und Nichtmetalle. |

Legierung L2:

| | |
|---|---|
| 88,6 Gew.-% | Magnesium |
| 10,0 Gew.-% | Dysprosium |
| 1,0 Gew.-% | Neodym |
| 0,2 Gew.-% | Zirkonium |
| 0,2 Gew.-% | Verunreinigungen umfassend Si, Ni, Fe, Cu sowie weitere Metalle und Nichtmetalle. |

Legierung L3:

| | |
|---|---|
| 87,6 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |
| 0,2 Gew.-% | Verunreinigungen umfassend Si, Ni, Fe, Cu sowie weitere Metalle und Nichtmetalle. |

Legierung L4:

| | |
|---|---|
| 89,7 Gew.-% | Magnesium |
| 6,0 Gew.-% | Dysprosium |
| 2,0 Gew.-% | Neodym |
| 2,0 Gew.-% | Zink |
| 0,3 Gew.-% | Verunreinigungen umfassend Si, Ni, Fe, Cu sowie weitere Metalle und Nichtmetalle. |

Legierung L5:

| | |
|---|---|
| 90,7 Gew.-% | Magnesium |
| 5,5 Gew.-% | Dysprosium |
| 3.0 Gew.-% | Neodym |
| 0,5 Gew.-% | Zirkonium |
| 0,3 Gew.-% | Verunreinigungen umfassend Si, Ni, Fe, Cu sowie weitere Metalle und Nichtmetalle. |

Legierung L6:

| | |
|---|---|
| 87,4 Gew.-% | Magnesium |
| 8,0 Gew.-% | Dysprosium |
| 2,2 Gew.-% | Neodym |
| 1,8 Gew.-% | Zink |
| 0,3 Gew.-% | Zirkonium |
| 0,3 Gew.-% | Verunreinigungen umfassend Si, Ni, Fe, Cu sowie weitere Metalle und Nichtmetalle. |

Legierung L7:

| | |
|---|---|
| 82,7 Gew.-% | Magnesium |
| 12,0 Gew.-% | Dysprosium |
| 2,5 Gew.-% | Neodym |
| 2,5 Gew.-% | Zink |
| 0,3 Gew.-% | Verunreinigungen umfassend Si, Ni, Fe, Cu sowie weitere Metalle und Nichtmetalle. |

Legierung L8:

| | |
|---|---|
| 85,2 Gew.-% | Magnesium |
| 11,5 Gew.-% | Dysprosium |
| 2,6 Gew.-% | Neodym |
| 0,4 Gew.-% | Zirkonium |
| 0,3 Gew.-% | Verunreinigungen umfassend Si, Ni, Fe, Cu sowie weitere Metalle und Nichtmetalle. |

Legierung L9:

| | |
|---|---|
| 83,1 Gew.-% | Magnesium |
| 15,2 Gew.-% | Dysprosium |
| 1,2 Gew.-% | Neodym |
| 0,2 Gew.-% | Zirkonium |
| 0,3 Gew.-% | Verunreinigungen umfassend Si, Ni, Fe, Cu sowie weitere Metalle und Nichtmetalle. |

Legierung L10:

| | |
|---|---|
| 88,9 Gew.-% | Magnesium |
| 8,0 Gew.-% | Dysprosium |
| 1,4 Gew.-% | Neodym |
| 1,2 Gew.-% | Zink |
| 0,2 Gew.-% | Zirkonium |
| 0,3 Gew.-% | Verunreinigungen umfassend Si, Ni, Fe, Cu sowie weitere Metalle und Nichtmetalle. |

Legierung L11:

| | |
|---|---|
| 90,6 Gew.-% | Magnesium |
| 8,0 Gew.-% | Dysprosium |
| 1,0 Gew.-% | Neodym |
| 0,2 Gew.-% | Zink |
| 0,2 Gew.-% | Zirkonium |

Legierung L12:

| | |
|---|---|
| 89,3 Gew.-% | Magnesium |
| 8,0 Gew.-% | Dysprosium |
| 1,0 Gew.-% | Neodym |
| 1,0 Gew.-% | Europium |
| 0,5 Gew.-% | Zink |
| 0,2 Gew.-% | Zirkonium |

Legierung L13:

| | |
|---|---|
| 86,0 Gew.-% | Magnesium |
| 12,0 Gew.-% | Dysprosium |
| 1,0 Gew.-% | Neodym |
| 0,8 Gew.-% | Zink |
| 0,2 Gew.-% | Zirkonium |

Legierung L14:

| | |
|---|---|
| 90,1 Gew.-% | Magnesium |
| 6,0 Gew.-% | Dysprosium |
| 1,0 Gew.-% | Neodym |
| 1,0 Gew.-% | Europium |
| 1,5 Gew.-% | Zink |
| 0,4 Gew.-% | Zirkonium |

Legierung L15:

| | |
|---|---|
| 86,8 Gew.-% | Magnesium |
| 10,0 Gew.-% | Dysprosium |
| 1,0 Gew.-% | Neodym |
| 1,0 Gew.-% | Europium |
| 1,0 Gew.-% | Zink |
| 0,2 Gew.-% | Zirkonium |

Legierung L16:

| | |
|---|---|
| 82,8 Gew.-% | Magnesium |
| 14,0 Gew.-% | Dysprosium |
| 0,5 Gew.-% | Neodym |
| 0,5 Gew.-% | Europium |
| 2,0 Gew.-% | Zink |
| 0,2 Gew.-% | Zirkonium |

Legierung L17:

| | |
|---|---|
| 87,3 Gew.-% | Magnesium |
| 10,0 Gew.-% | Dysprosium |
| 1,5 Gew.-% | Neodym |
| 1,0 Gew.-% | Zink |
| 0,2 Gew.-% | Zirkonium |

Legierung L18:

| | |
|---|---|
| 87,45 Gew.-% | Magnesium |
| 10,0 Gew.-% | Dysprosium |
| 1,5 Gew.-% | Neodym |
| 1,0 Gew.-% | Zink |
| 0,05 Gew.-% | Eisen |

Legierung L19:

| | |
|---|---|
| 83,1 Gew.-% | Magnesium |
| 15,0 Gew.-% | Dysprosium |
| 0,9 Gew.-% | Neodym |
| 1,0 Gew.-% | Zirkonium |

Legierung L20:

| | |
|---|---|
| 95,0 Gew.-% | Magnesium |
| 4,5 Gew.-% | Dysprosium |
| 0,5 Gew.-% | Neodym |

Legierung L21:

| | |
|---|---|
| 83,7 Gew.-% | Magnesium |
| 10,0 Gew.-% | Dysprosium |
| 5,0 Gew.-% | Neodym |
| 1,0 Gew.-% | Zink |
| 0,3 Gew.-% | Zirkonium |

Legierung L22:

| | |
|---|---|
| 87,25 Gew.-% | Magnesium |
| 10,0 Gew.-% | Dysprosium |
| 1,5 Gew.-% | Neodym |
| 1,0 Gew.-% | Zink |
| 0,05 Gew.-% | Eisen |
| 0,2 Gew.-% | Zirkonium |

Legierung L23:

| | |
|---|---|
| 85,8 Gew.-% | Magnesium |
| 12,0 Gew.-% | Dysprosium |
| 1,0 Gew.-% | Neodym |
| 1,0 Gew.-% | Zink |
| 0,2 Gew.-% | Zirkonium |

Legierung L24:

| | |
|---|---|
| 82,1 Gew.-% | Magnesium |

(fortgesetzt)

| | |
|---|---|
| 15,0 Gew.-% | Dysprosium |
| 0,9 Gew.-% | Neodym |
| 1,0 Gew.-% | Zink |
| 1,0 Gew.-% | Zirkonium |

Legierung L25:

| | |
|---|---|
| 80,1 Gew.-% | Magnesium |
| 19,0 Gew.-% | Yttrium |
| 0,9 Gew.-% | Europium |

Legierung L26:

| | |
|---|---|
| 92,5 Gew.-% | Magnesium |
| 5,0 Gew.-% | Dysprosium |
| 2,5 Gew.-% | Europium |

Legierung L27:

| | |
|---|---|
| 82,1 Gew.-% | Magnesium |
| 15,5 Gew.-% | Dysprosium |
| 1,2 Gew.-% | Neodym |
| 1,0 Gew.-% | Zink |
| 0,2 Gew.-% | Zirkonium |
| 0,001 Gew.-% | Verunreinigungen umfassend Si, Ni, Fe, Cu sowie weitere Metalle und Nichtmetalle. |

Legierung L28:

| | |
|---|---|
| 82,0 Gew.-% | Magnesium |
| 10,0 Gew.-% | Dysprosium |
| 5,0 Gew.-% | Neodym |
| 1,0 Gew.-% | Zink |
| 2,0 Gew.-% | Zirkonium |

Legierung L29:

| | |
|---|---|
| 88,8 Gew.-% | Magnesium |
| 6,0 Gew.-% | Dysprosium |
| 4,0 Gew.-% | Europium |
| 1,0 Gew.-% | Zink |
| 0,2 Gew.-% | Zirkonium |

Legierung L30:

| | |
|---|---|
| 89,8 Gew.-% | Magnesium |
| 8,0 Gew.-% | Dysprosium |
| 1,0 Gew.-% | Europium |
| 1,0 Gew.-% | Zink |

(fortgesetzt)

| | |
|---|---|
| 0,2 Gew.-% | Zirkonium |

Legierung L31:

| | |
|---|---|
| 83,2 Gew.-% | Magnesium |
| 15,0 Gew.-% | Dysprosium |
| 0,4 Gew.-% | Neodym |
| 1,4 Gew.-% | Europium |

Legierung L32:

| | |
|---|---|
| 87,4 Gew.-% | Magnesium |
| 10,0 Gew.-% | Dysprosium |
| 1,0 Gew.-% | Europium |
| 0,5 Gew.-% | Neodym |
| 1,0 Gew.-% | Zink |
| 0,1 Gew.-% | Zirkonium |

Legierung L33:

| | |
|---|---|
| 87,0 Gew.-% | Magnesium |
| 10,0 Gew.-% | Dysprosium |
| 0,3 Gew.-% | Europium |
| 1,5 Gew.-% | Neodym |
| 1,0 Gew.-% | Zink |
| 0,2 Gew.-% | Zirkonium |

Legierung L34:

| | |
|---|---|
| 86,0 Gew.-% | Magnesium |
| 12,0 Gew.-% | Dysprosium |
| 1,0 Gew.-% | Europium |
| 0,8 Gew.-% | Zink |
| 0,2 Gew.-% | Zirkonium |

Legierung L35:

| | |
|---|---|
| 93,24 Gew.-% | Magnesium |
| 0,35 Gew.-% | Dysprosium |
| 2,05 Gew.-% | Neodym |
| 0,40 Gew.-% | Gadolinium |
| 0,35 Gew.-% | Zirkonium |
| 3,60 Gew.-% | Yttrium |
| 0,01 Gew.-% | Erbium |

Legierung L36:

| | |
|---|---|
| 92,2 Gew.-% | Magnesium |

(fortgesetzt)

| | |
|---|---|
| 0,5 Gew.-% | Dysprosium |
| ..0,5 Gew.-% | Gadolinium |
| 2,2 Gew.-% | Neodym |
| 0,5 Gew.-% | Zirkonium |
| 4,1 Gew.-% | Yttrium |

Legierung L37:

| | |
|---|---|
| 91,8 Gew.-% | Magnesium |
| 0,7 Gew.-% | Dysprosium |
| 0,7 Gew.-% | Gadolinium |
| 2,0 Gew.-% | Neodym |
| 0,7 Gew.-% | Zirkonium |
| 4,1 Gew.-% | Yttrium |

Legierung L38:

| | |
|---|---|
| 95,1 Gew.-% | Magnesium |
| 1,2 Gew.-% | Gadolinium |
| 2,5 Gew.-% | Neodym |
| 0,6 Gew.-% | Zirkonium |
| 0,3 Gew.-% | Calcium |
| 0,3 Gew.-% | Zink |

Legierung L39:

| | |
|---|---|
| 96,9 Gew.-% | Magnesium |
| 2,5 Gew.-% | Neodym |
| 0,4 Gew.-% | Zirkonium |
| 0,2 Gew.-% | Zink |

Legierung L40:

| | |
|---|---|
| 97,45 Gew.-% | Magnesium |
| 0,75 Gew.-% | Neodym |
| 1,80 Gew.-% | Mangan |

Legierung L41:

| | |
|---|---|
| 97,45 Gew.-% | Magnesium |
| 0,75 Gew.-% | Cer |
| 1,80 Gew.-% | Mangan |

Legierung L42:

| | |
|---|---|
| 90,0 Gew.-% | Magnesium |
| 3,0 Gew.-% | Gadolinium |
| 2,4 Gew.-% | Yttrium |

(fortgesetzt)

| | |
|---|---|
| 0,4 Gew.-% | Zirkonium |
| 4,2 Gew.-% | Scandium |

Legierung L43:

| | |
|---|---|
| 90,0 Gew.-% | Magnesium |
| 3,0 Gew.-% | Neodym |
| 2,4 Gew.-% | Yttrium |
| 0,4 Gew.-% | Zirkonium |
| 5,2 Gew.-% | Scandium |
| 2,0 Gew.-% | Indium |

Legierung L44:

| | |
|---|---|
| 96,0 Gew.-% | Magnesium |
| 4,0 Gew.-% | Lithium |

Die Legierungen L1 bis L44 wurden zum einen mit einer anorganischen Magnesiumfluoridbeschichtung und zum anderen mit einer anorganischen Magnesiumfluoridbeschichtung und einer organischen Parylen-C-Beschichtung hergestellt. Sämtliche Legierungen L1 bis L44 zeigen eine verlangsamte Auflösungskinetik der Stents mit anorganischer Magnesiumfluoridbeschichtung im Vergleich zu den unbeschichteten Stents. Ferner zeigen die Stents mit einer anorganischen Magnesiumfluoridbeschichtung und einer organischen Parylen-C-Beschichtung eine abermals verlangsamte Auflösungskinetik gegenüber den Stents mit anorganischer Magnesiumfluoridbeschichtung und ohne organische Parylen-C-Beschichtung. Zudem ist die Auflösungskinetik deutlich verlangsamt im Vergleich zu einem Stent, der nur eine organische Parylen-C-Beschichtung aufweist. Die Anwesenheit eines Antirestenose-Wirkstoffs in oder auf der organischen Parylen-C-Beschichtung scheint keinen merklichen Einfluss auf die Resorptionskinetik zu haben. Die folgenden Beispiele beschreiben exemplarisch die Herstellung und Untersuchung derartiger Stents.

**Beispiel 2: Rohrherstellung**

Aus den Legierungen L1 bis L10 gemäß Beispiel 1 wurden stranggepresste Drähte hergestellt. In diesen stranggepressten Drähten wird eine Präzisionsbohrung in Längsrichtung eingebracht, welche bereits die Wandstärke der späteren Stents mitbestimmt. Durch mehrere Umformschritte wird ein Rohr mit vorgegebenem Durchmesser und bestimmter Wandstärke hergestellt. Zwischen den einzelnen Umformschritten findet eine wiederkehrende Wärmebehandlung statt.

**Beispiel 3: Stentfertigung**

Ein gemäß Beispiel 2 hergestelltes Rohr wird in eine Aufnahme in der Lasermaschine fixiert. Ein gepulster Festkörperlaser (FKL) schneidet aus dem Rohr die Konturen des Stentdesigns heraus. Das Laserschneiden erfolgt unter Schutzgasatmosphäre.

Das Stentdesign ist in einem NC-Programm (numerical control = Numerische Steuerung) hinterlegt. Dieses gibt dem Laser die Verfahrwege vor, nach denen das Rohr strukturiert wird. Durch das Laserstrahlschneiden kommt es zur Gradbildung, insbesondere auf der Rohrinnenseite, entlang der gesamten Schneidkontur. Dies kann dazu führen, dass Reststücke und Ausschnitte nach Beendigung des Schneidvorgangs in der Kontur haften bleiben. Die Reststücke und Ausschnitte werden mechanisch entfernt und der Stent von Fertigungsrückständen gereinigt. In einer ersten optischen Sichtkontrolle wird eine Inspektion der Schneidkontur durchgeführt.

Im Folgenden wird der Stent elektro-chemisch poliert. Der Stent wird anodisch beschaltet und in ein Säurebad getaucht. Über eine im Bad fixierte Kathode wird ein Stromkreis geschlossen. Der Stromkreis bleibt für mehrere Minuten aufrechterhalten. Die Elektropolitur ist ein umgekehrter galvanischer Prozess bei dem kontrolliert Material von der Oberfläche des anodisch beschalteten Bauteils abgetragen wird. Dabei wird durch das Verfahren bedingt bevorzugt an scharf kantigen Ecken und Kanten abgetragen. Der Stent erhält eine glatte Oberfläche und abgerundete Kanten entlang der Konturen. Nach der Politur wird der Stent gereinigt und von Säurerückständen befreit. In der Endreinigung werden alle noch verbliebenden Fertigungsrückstände von der Stentoberfläche entfernt. In einer letzen optischen Sichtkontrolle wird

die Stentgeometrie vermessen und die Oberfläche auf Reinheit geprüft.

**Beispiel 4: Bestimmung der Korngröße**

Die Auszählung der Korngröße wurde im Querschliff im Linienschnittverfahren vorgenommen. Körner die am Ende der Geraden nur halb geschnitten sind, wurden hierbei als halbe Körner gezählt. Die Vergrößerung wurde so gewählt, dass durch das Linienraster mindestens 50 Körner geschnitten wurden. Es wurden mindestens 5 Stellen, mit einer Gesamtanzahl von mindestens 250 Schnittpunkten, auf der Probe ausgewertet.

**Beispiel 5: Bestimmung der Korrosionsrate**

Bei Raumtemperatur wurden über einen Zeitraum von 3 Tagen die Korrosionsraten verschiedener Legierungen in physiologischer Kochsalzlösung bestimmt (s. Tabelle 1). Getestet wurde eine Legierung enthaltend 90,8 Gew.-% Mg, 8 Gew.-% Dy, 1 Gew.-% Nd und 0,2 Gew.-% Zr, eine Legierung enthaltend 89,8 Gew.-% Mg, 8 Gew.-% Dy, 1 Gew.-% Nd, 1 Gew.-% Eu und 0,2 Gew.-% Zr, eine Legierung enthaltend 86,8 Gew.-% Mg, 12 Gew.-% Dy, 1 Gew.-% Nd und 0,2 Gew.-% Zr und eine Legierung enthaltend 87,8 Gew.-% Mg, 10 Gew.-% Dy, 1 Gew.-% Nd, 1 Gew.-% Eu und 0,2 Gew.-% Zr. Zusätzlich wurden Legierungen getestet enthaltend 1,0 Gew.-% Neodym, 1,0 Gew.-%Zink, 0,2 Gew.-% Zirkonium, zwischen 5 und 20 % Dysprosium und den Rest Magnesium (s. Abbildung 7). Korrosionsprodukte wurden durch Eintauchen der Proben in Chromsäure (180 g/L) für 20 min bei Raumtemperatur entfernt. Die durchschnittliche Korrosionsrate wurde in Millimeter pro Jahr mit Hilfe der folgenden Gleichung berechnet:

$$CR = \frac{8.76 \times 10^4 \times \Delta\, g}{A \cdot t \cdot \rho}$$

Tabelle 1: Korrosionsrate der erfindungsgemäßen Legierungen, gemessen über 3 Tage bei Raumtemperatur und in 0,9% NaCl; die Angaben zu den Komponenten der Legierungen sind in Gew.-% und Mg ist als Hauptkomponente füllt immer bis auf 100% der Legierung auf. Getestet wurden die Legierungen nach dem Guss ohne Wärmebehandlung, angegeben sind die Mittelwerte und Standardabweichungen der unterschiedlichen Legierungen.

| Nr. | Zusammensetzung | Korrosionsrate (mm/Jahr) |
|---|---|---|
| L11 | Mg8Dy1Nd0,2Zn0,2Zr | 9,25 ±0,38 |
| L15 | Mg10Dy1Nd1Eu1Zn0,2Zr | 0,81±0,06 |
| L23 | Mg12Dy1Nd1Zn0,2Zr | 2,94±1,88 |
| L16 | Mg8Dy1Nd1Eu1Zn0,1Zr | 4,9±1,62 |
| L14 | Mg6Dy1Nd1Eu1,5Zn0,4Zr | 9,56±0,29 |
| L16 | Mg14Dy0,5Nd0,5Eu2Zn0,2Zr | 1,25±0,12 |
| L18 | Mg10Dy1,5Nd1Zn0,05Fe | 12,41±2,16 |
| L20 | Mg4,5Dy0,5Nd | 25,56±2,34 |
| L24 | Mg15Dy0,9Nd1Zr1Zn | 2,98±1,78 |
| L25 | Mg20Y0,9Eu | 44,71±3,22 |
| L28 | Mg20Gd5Nd1Zn2Zr | 38,96±1,34 |
| L30 | Mg8Dy1Eu1Zn0,2Zr | 3,88±1,87 |
| L22 | Mg10Dy1,5Nd1Zn0,2Zr0,05Fe | 4,47±2,11 |
| L34 | Mg12Dy1Eu0,8Zn0,2Zr | 5,46±1,22 |
| L29 | Mg6Dy4Eu1Zn0,2Zr | 12,20±1,36 |
| L33 | Mg10Dy0,3Eu1,5Nd1Zn0,2Zr | 1,25±0,67 |
| L26 | Mg5Dy2,5Eu | 23.56±1,56 |
| L31 | Mg25Dy0,4Nd1,4Eu | 48,71±1,87 |

**Beispiel 6: Mechanische Kennwerte der Legierungen**

Die Legierungen und Gussteile wurden hergestellt, wie im Beispiel 1 beschrieben und stranggepresst. Die Wärmebehandlung T4 erfolgte bei 510°C über 8h und eventuell anschließend die Wärmebehandlung T6 bei 200°C über einen Zeitraum von 72h. Nach der T4-Wärmebenadlung wurden die Proben unmittelbar in Wasser abgeschreckt. Alle Proben wurden von der gleichen Position der Blöcke entnommen.

Die Zugversuche wurden bei Raumtemperatur gemäß DIN EN 10002-1 (entspricht ISO 6892 und ASTM E8) und Druckversuche wurden bei Raumtemperatur gemäß DIN 50106 (entspricht ISO 604 und ASTM D695) durchgeführt. Es wurden mindestens 3 Proben für jeden Wert getestet. Die Zugfestigkeit wurde aus der maximal erreichten Zugkraft im Zugversuch bezogen auf den ursprünglichen Querschnitt der Probe berechnet.

Tabelle 2: Mechanische Kennwerte erfindungsgemäßer Legierungen. Getestet wurden die Legierungen als Probe nach dem Strangpressen (ST, ohne Wärmebehandlung) und nach unterschiedlicher Wärmebehandlung, T4 (lösungsgeglüht) und T6 (eine weitere Wärmebehandlung nach T4, auch "Auslagern" genannt). Die Angaben zu den Komponenten der Legierungen sind in Gew.-% und Mg als Hauptkomponente ergänzt die Mengenangabe immer bis auf 100% der Legierung. SD steht für die Standardabweichung der Mittelwerte, die in der Spalte zuvor angegeben sind (n=3).

| | Zusammensetzung | Streckgrenze (MPa) | SD | Zug festigkeit (MPa) | SD | Bruch dehnung (%) | SD |
|---|---|---|---|---|---|---|---|
| ST | Mg8Dy1Nd0,2Zn0,2Zr | 107,33 | 1,8 | 208,5 | 0,85 | 28,12 | 3,41 |
| T4 | | 87,54 | 0,46 | 176,84 | 2,03 | 18,83 | 1,79 |
| T6 | | 97,95 | 1,67 | 194,11 | 1,1 | 19,33 | 0,68 |
| ST | Mg10Dy1Nd1Eu1Zn0,2Zr | 169,30 | 0,74 | 283,89 | 0,68 | 16,96 | 1 |
| T4 | | 151,97 | 1,77 | 259,50 | 2,57 | 18,02 | 0,29 |
| T6 | | 159,23 | 2,23 | 275,55 | 1,78 | 18,15 | 2,77 |
| ST | Mg12Dy1Nd1Zn0,2Zr | 126,07 | 1,8 | 226,04 | 0,35 | 28,55 | 0,08 |
| T4 | | 98,38 | 0,43 | 188,45 | 0,5 | 20,47 | 0,91 |
| T6 | | 114,6 | 1,69 | 205,2 | 1,25 | 17,99 | 0,79 |
| ST | Mg8Dy1Nd1Eu1Zn0,1Zr | 132,24 | 1,1 | 227,21 | 0,59 | 19,75 | 1,11 |
| T4 | | 114,93 | 1,25 | 210,73 | 1,51 | 20,89 | 1,01 |
| T6 | | 136,77 | 1,77 | 223,28 | 0,67 | 23,64 | 2,01 |
| ST | Mg6Dy1Nd1Eu1,5Zn0,4Zr | 128,14 | 8,02 | 202,74 | 2,91 | 24,62 | 2,09 |
| T4 | | 80,97 | 2,27 | 173,47 | 2,02 | 23,78 | 3,52 |
| T6 | | 84,26 | 2,57 | 178,26 | 1,35 | 26,32 | 2,5 |
| ST | Mg14Dy0,5Nd0,5Eu2Zn0,2Zr | 165,64 | 4,95 | 218,17 | 3,07 | 18,9 | 1,14 |
| T4 | | 110,78 | 1,87 | 201,28 | 1,19 | 21,62 | 1,07 |
| T6 | | 153,15 | 3,55 | 264,09 | 0,71 | 17,66 | 1,33 |
| ST | Mg10Dy1,5Nd1Zn0,05Fe | 145,46 | 3,55 | 237,21 | 0,75 | 28,9 | 1,73 |
| T4 | | 102,78 | 4,38 | 193,36 | 5,84 | 27,57 | 0,88 |
| T6 | | 108,84 | 1,68 | 200,16 | 2,97 | 25,56 | 1,66 |
| ST | Mg4,5Dy0,5Nd | 68,39 | 7,9 | 208,48 | 2,03 | 28,4 | 0,72 |
| T4 | | 60,31 | 1,71 | 179,04 | 0,83 | 23,17 | 0,38 |
| T6 | | 75,13 | 1,32 | 250,34 | 1,42 | 13,34 | 0,74 |
| ST | Mg15Dy0,9Nd1Zr1Zn | 136,93 | 1,6 | 227,07 | 0,42 | 22,9 | 3,03 |
| T4 | | 95,79 | 1,94 | 200,59 | 2,59 | 21,57 | 0,34 |

(fortgesetzt)

| | Zusammensetzung | Streckgrenze (MPa) | SD | Zug festigkeit (MPa) | SD | Bruch dehnung (%) | SD |
|---|---|---|---|---|---|---|---|
| T6 | | 112,09 | 0,41 | 206,11 | 0,19 | 19,56 | 0,66 |
| ST | Mg20Y0,9Eu | 159,75 | 1,99 | 238,55 | 0,76 | 11,57 | 0,58 |
| T4 | | 123,19 | 4,83 | 214 | 1,42 | 19,62 | 2,74 |
| T6 | | 144,08 | 4,37 | 220,2 | 2,58 | 15,58 | 0,94 |
| ST | Mg20Gd5Nd1Zn2Zr | 297,75 | 8,12 | 338,53 | 5,67 | 1,53 | 0,27 |
| T4 | | 195,82 | 15,65 | 276,89 | 0,91 | 6,58 | 0,95 |
| T6 | | 327,07 | 17,57 | 378,45 | 14,94 | 0,76 | 0,32 |
| ST | Mg8Dy1Eu1Zn0,2Zr | 112,85 | 1,15 | 198,9 | 0,43 | 24,07 | 1,05 |
| T4 | | 93,5 | 1,01 | 182,38 | 0,91 | 24,02 | 0,81 |
| T6 | | 99 | 0,99 | 185,7 | 0,4 | 25,9 | 1,16 |
| ST | Mg10Dy1,5Nd1Zn0,2Zr0,05Fe | 127,8 | 4,62 | 215,84 | 1 | 19,39 | 1,4 |
| T4 | | 96,72 | 4,02 | 192,99 | 2,87 | 25,92 | 0,98 |
| T6 | | 112,34 | 3,1 | 201,35 | 2,18 | 24,44 | 1,91 |
| ST | Mg12Dy1Eu0,8Zn0,2Zr | 182,30 | 1,52 | 293,62 | 1,37 | 22,39 | 2,06 |
| T4 | | 164,48 | 1,44 | 268,66 | 0,45 | 23,70 | 1,63 |
| T6 | | 172,34 | 2,12 | 271,35 | 1,82 | 23,34 | 1,79 |
| ST | Mg6Dy4Eu1Zn0,2Zr | 115,09 | 1,39 | 208,3 | 1,68 | 2,30 | 0,51 |
| T4 | | 97,55 | 0,74 | 189,39 | 0,84 | 4,78 | 1,71 |
| T6 | | 112,58 | 1,59 | 196,71 | 2,31 | 3,41 | 0,69 |
| ST | Mg10Dy0,3Eu1,5Nd1Zn0,2Zr | 168,54 | 6,15 | 277,11 | 2,09 | 16,46 | 2,33 |
| T4 | | 136,36 | 5,11 | 244,89 | 2,37 | 20,67 | 3,15 |
| T6 | | 152,22 | 2,42 | 253,91 | 2,33 | 18,56 | 1,87 |
| ST | Mg5Dy2,5Eu | 74,25 | 1,63 | 283,50 | 1,44 | 21,60 | 1,27 |
| T4 | | 60,19 | 1,69 | 264,46 | 0,91 | 23,16 | 1,43 |
| T6 | | 65,38 | 1,83 | 266,64 | 1,36 | 22,85 | 1,64 |
| ST | Mg25Dy0,4Nd1,4Eu | 106,34 | 2,98 | 211,15 | 1,65 | 18,90 | 1,55 |
| T4 | | 88,74 | 1,69 | 178,56 | 2,03 | 20,03 | 2,31 |
| T6 | | 94,21 | 1,34 | 191,25 | 1,67 | 19,54 | 1,99 |

**Beispiel 7: Tierversuchsstudie**

8 Stents hergestellt gemäß Beispiel 2 und 3 wurden in die Koronarien von 4 Hausschweinen implantiert. Die Stents hatten einen Durchmesser von 3,0 mm und eine Länge von 14 mm (Länge des Katheterballons 15 mm), waren nicht beschichtet (BMS) und waren aus einer Legierung der folgenden Zusammensetzung hergestellt:

| | |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |

Der gewählte "follow up" Zeitpunkt für alle 4 Tiere war 4 Wochen nach Implantation. Einen Tag vor der Stentimplantation wurde den Schweinen eine Einzeldosis Clopidogrel (300mg) und Aspirin (250mg) oral verabreicht. Unter Vollnarkose wurde chirurgisch ein Zugang zu der Femoralarterie gelegt und einem Bolus von Natrium-Heparin (10 000 IU) verabreicht. Ein 6F koronarer Führungskatheter wurde durch Femoralarterie in die Aorta descendens eingeführt. Die Koronarangiographie wurde durch eine Injektion nicht-ionischen Kontrastmittels per Hand durchgeführt, um die anatomischen Gegebenheiten für die Durchführung des Verfahrens zu erhalten.

Die Stents wurden im *Ramus interventricularis anterior* (RIVA oder engl. LAD) und *Ramus circumflexus* (RCX oder engl. LCx) implantiert. Der Dilatationsdruck des Ballons zur Implantation des Stents wurde so gewählt, dass eine Stent-Ballon zu Arterie Verhältnis von 1,2 zu 1 erreicht wurde. Die Schweine bekamen die Möglichkeit sich anschließend zu erholen. Während der gesamten 4 Wochen "follow up" wurde den Tieren täglich100mg Aspirin und 75mg Clopidogrel pro 30g Körpergewicht oral verabreicht.

Nach 4 Wochen "follow up", wurden die Kontrollangiographie und die optische Kohärenztomographie (OCT) durchgeführt.

Im Rahmen des OCT Verfahren wurde ein 0,3556 mm (0,014 Inch) Führungsdraht wird in den RIVA und den RCX eingefügt und durch die implantierten Stents in den distalen Teil des Gefäßes geführt. Ein intravaskulärer OCT-Katheter wurde anschließend über den Führungsdraht bis distal des Stent vorgeschoben. Die Injektionspumpe wurde eingeschaltet, um Kontrastmittel mit einer Geschwindigkeit von 3,0 ml/s zu injizieren und somit das Blut vorübergehend zu verdrängen. Die gesamte Länge der Läsion wird mit Hilfe einer automatischen Rückzugshilfe und 10 mm/s abgebildet. Nach den Aufnahmen wird der OCT-Katheter zurückgezogen, und die Bilder gespeichert. Die Tiere wurden dann euthanasiert, und die Koronararterien explantiert.

Die explantierten Arterien wurden mit einem Druck von 13,33 kPa (100 mmHg) für 1h mit 7% Formalin perfusionsfixiert. Die Stents wurden für die Lichtmikroskopie verarbeitet. Die Arterien wurden für die Lichtmikroskopie in drei Teile geschnitten: proximale, mittlere und distale Stent-Segmente. Die gestenteten Abschnitte wurden in Methylmetacrylat (Technovit 9100) eingebettet. Von den Segmenten der gestenteten Arterien werden
unter Verwendung eines Rotationsmikrotoms 4-6 μm Schnitte angefertigt und mit Hämatoxylin und Eosin angefärbt.

Im Rahmen der Analyse wurden Details der Studie aufgelistet, wie die Stentposition, der Dilatationsdruck und die Dilatationszeit, sowie alle Komplikationen während der Implantation.

*Quantitative Koronarangioplastie (QCA)*

Eine QCA wurde zur Analyse der In-Stent-Restenose durchgeführt. Die folgenden Parameter wurden dabei bestimmt: Gefäßdurchmesser vor und nach Stentimplantation, den minimalen Lumendurchmesser (MLD) nach Stentimplantation und nach *follow up* und den Durchmesser eines Vergleichssegments nach *follow up.* Hierbei ist der minimale Lumendurchmesser der kleinste absolute Gefäßinnendurchmesser im Bereich des dilatierten Segments, gemittelt aus den zwei orthogonalen Projektionsebenen. LLL (late lumen loss) ist ein Maß der Verengung des Lumens durch neointimale Hyperplasie. Gemessen wird der Lumendurchmesser direkt nach der Intervention und 4 Wochen postinterventionell, die Differenz beider wird als LLL angegeben. Die Länge des stenosierten oder dilatierten Segments wurde kontrolliert und die prozentuale Stenose kalkuliert.

*Optische Kohärenztomographie (OCT)*

Die Bilder der optischen Kohärenztomographie wurden an Hand der relevanten Richtlinie (JACC, 2012) ausgewertet. Die folgenden Parameter wurden erhoben: Stent-Malapposition, Bedeckung der Stentstreben, Hervortreten des Gewebes, die arterielle Dissektion, Thrombose. Die quantitative Analyse der OCT-Bilder umfasst den minimalen und maximalen Stentdurchmesser und die Fläche des Lumens. Die folgenden Parameter wurden berechnet: maximale Stenoseausdehnung (area stenosis) und Stentsymmetrie. Für die quantitative Analyse wurde der "schlechteste" Querschnitt pro Testgruppe bestimmt.

Berechnung der Stenoseausdehnung (%AS):

%AS= Fläche der Intima/Stentfläche = (Stentfläche–Fläche des Lumens)/Stentfläche

Berechnung der Stentsymmetrie:

Stentsymmetrie = (Maximaler Stentdurchmesser-Minimaler Stentdurchmesser)/ Maximaler Stentdurchmesser

Fibrinablagerung, Entzündungsgrad (Intima und Adventitia), Einblutungen und Nekrose wurden analog zu publizierten Richtlinien ausgewertet.

*Histomorphometrie*

Die Histomorphometrie wurde mittels computer-assistierter Flächenmessung durchgeführt. Das Lumen, die Fläche der Membrana elastica interna und Membrana elastica externa und die maximale Dicke der Neointima wurden gemessen. Die Ausdehnung der Neointima und der Tunica media und die prozentuale Stenose wurden berechnet.

Ergebnisse

Der Dilatationsdruck der aufgewendet wurde lag zwischen 1216 kPa (12 atm) und 1824 kPa (18 atm). Die Balloninflation dauerte 30 sec an. Generell war die Handhabung des Stents und Ballons exzellent, eine sehr gute Schubfähigkeit und sehr kurze Dauer der Deflation war zu verzeichnen.

Tabelle 3: Ergebnisse der Quantitativen Koronarangioplastie (QCA), angegeben sind die Mittelwerte und Standardabweichungen (SD); MLD=minimaler Lumendurchmesser, RD=Durchmesser eines Vergleichssegments, % DS=prozentualer Stenosedurchmesser, FUP=follow-up, LLL=Late Lumen Loss

| | **Prä-MLD (mm)** | **Post-MLD (mm)** | **FUP-MLD (mm)** | **FUP-RD (mm)** | **FUP-%DS (%)** | **LLL (mm)** |
|---|---|---|---|---|---|---|
| Unbeschichtete Stents (BMS) | **2,68** | **2,93** | **2,08** | **2,92** | **28,75** | **0,85** |
| SD | 0,11 | 0,07 | 0,53 | 0,20 | 16,79 | 0,47 |

Tabelle 4: Qualitative Analyse der Optischen Kohärenztomographie (OCT) je implantiertem Stent

| **Tier-Nr.** | **Arterie** | **Gruppe** | **Stent-Malapposition** | **Hervortreten des Gewebes** | **In-Stent Thrombose** | **In-Stent Dissektion** | **Rand-Dissektion** | **Endothelialisierung** |
|---|---|---|---|---|---|---|---|---|
| MEKO-1 | LAD | BMS | 0 | 0 | 0 | 0 | 0 | komplett |
| MEKO-1 | LCx | BMS | 0 | 0 | 0 | 0 | 0 | lückenhaft |
| MEKO-2 | LAD | BMS | 0 | 0 | 0 | 0 | 0 | komplett |
| MEKO-2 | LCx | BMS | 0 | 0 | 0 | 0 | 0 | komplett |
| MEKO-3 | LAD | BMS | 0 | 0 | 0 | 0 | 0 | komplett |
| MEKO-3 | LCx | BMS | 0 | 0 | 0 | 0 | 0 | komplett |
| MEKO-4 | LAD | BMS | 0 | 0 | 0 | 0 | 0 | komplett |
| MEKO-4 | LCx | BMS | 0 | 0 | 0 | 0 | 0 | komplett |

Tabelle 5: Qualitative Analyse der Optischen Kohärenztomographie (OCT) in Bezug zur Anzahl der implantierten Stents (n=8; alle Werte in Prozent)

| | **Stent-Malapposition** | **Hervortreten des Gewebes** | **In-Stent Thrombose** | **In-Stent Dissektion** | **Rand-Dissektion** | **Abgeschlossene Endothelialisierung** |
|---|---|---|---|---|---|---|
| BMS n=8 | 0 | 0 | 0 | 0 | 0 | 87,5 |

Aus den Tabellen 3, 4 und 5 lässt sich entnehmen, dass zum einen bei Verwendung eines erfindungsgemäßen Stents keine der getesteten Komplikationen auftrat und zum anderen, dass eine Endothelialisierung nach 4 Wochen fast immer vollständig abgeschlossen war, was bedeutete, dass das erhöhte Risiko der In-Stent-Thrombose durch nicht abgeschlossene Endothelialisierung oder Endzündungsreaktionen nicht mehr vorliegt. Vergleichbare Ergebnisse wurden auch mit Stents aus einer Magnesiumlegierung enthaltend Europium an Stelle von Neodym erhalten.

Tabelle 6: weitere Ergebnisse der Qualitative Analyse der Optischen Kohärenztomographie (OCT), angegeben sind die Mittelwerte und Standardabweichungen (SD).

| Type | Min. Stent durchmesser (mm) | max. Stent durchmesser (mm) | Stent fläche ($mm^2$) | Lumen fläche ($mm^2$) | %AS (%) | Stent symmetrie |
|---|---|---|---|---|---|---|
| BMS n=8 | **2,54** | **2,72** | **7,58** | **5,08** | **34,0** | **0,07** |
| SD | 0,34 | 0,35 | 1,80 | 1,69 | 13,2 | 0,02 |

**Beispiel 8:** Beschichtung von erfindungsgemäßen Stents über Oberflächenumwandlungen

Magnesiumfluoridschicht ($MgF_2$)

Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet.

Magnesiumfluoridschicht ($MgF_2$) und Glühen

Zur Erzeugung dieser Schicht wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Daraufhin wurde der Stent für 24,5 Stunden an Luftatmosphäre geglüht.

Magnesiumfluoridschicht ($MgF_2$)

Zur Erzeugung dieser Schicht wurde der Stent für 1,5 Stunden in ein Sauerstoffplasma gelegt. Das Plasma soll die Oberfläche des Stents oxidieren, sodass eine MgO Schicht entsteht. Anschließend wurde der Stent für 24 Stunden unter 50°C in eine 40%ige Flusssäure (50 ml) getaucht. Der Behälter mit der Flusssäure schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet.

Magnesiumfluoridschicht ($MgF_2$) mit einer Ammoniumfluoridlösung

Zur Erzeugung dieser Schicht wurde der Stent für 24 Stunden unter 50°C in eine 10%ige Ammoniumfluoridlösung (50 ml) getaucht. Der Behälter mit der Lösung schwamm wiederum in einem beheizten Wasserbad, um die Temperatur von 50°C zu gewährleisten. Nach den 24 Stunden wurde der Stent entnommen und mit deionisiertem Wasser abgespült und anschließend an Luft getrocknet. Abgebildet sind die Messwerte an gelöstem Magnesium über die Zeit von einem Stent der Probe B.

**Beispiel 9: Degradationstest von Stents, die einer Sauerstoffionen-Implantation unterzogen wurden**

Die in Beispiel 8 beschichteten Stents wurden in Degradationstest untersucht.

Die Degradationstests wurden mit einer Degradationstest-Maschine (DTM) aus dem Hause MeKo Laserstrahl-Materialbearbeitungen e.K. durchgeführt. Die DTM ist mit einer Peristaltikpumpe, einem Temperatursensor, Heizsystem, Durchflusssensor und einem Kamerasystem ausgestattet.

Der für die Untersuchungen verwendete Koronarstent wurde bei der Firma MeKo Laserstrahl-Materialbearbeitungen e.K. gefertigt. Bei dem Stentmaterial handelt es sich um Magnesiumlegierung A (Mg10Dy1Nd1Zn0,2Zr).

Der Stent aus der Magnesiumlegierung A wurde einer Sauerstoffionen-Implantation unterzogen. Dabei werden Sauerstoff-Ionen in die Oberfläche geschossen, wo diese dann die Oberfläche modifizieren bzw. eine Magnesiumoxid Schicht entstehen soll. Die Masse des Stents betrug nach der Ionenimplantation 5,97 mg (gemessen mit der Sartorius Feinwaage CPA225D (Serial Number: 31906122).

Die Füllmenge des Testfluids PBS (Phosphate Buffered Saline) betrug 300 ml. Die Temperatur des Fluids wird bei 37$\pm$2°C gehalten. Der Durchfluss [ml/min], die Temperatur des Fluids [°C] und die digitalen Bildaufnahmen [1 Bild/min] werden vom System kontinuierlich aufgezeichnet und gespeichert. Das Testfluid PBS setzt sich zusammen aus: 8.0 [g/l] NaCl, 0.2 [g/l] KCl, 1.15 [g/l] $Na_2HPO_4$ und 0.2 [g/l] $KH_2PO_4$.

Die pH-Werte wurden mit dem Messgerät Mettler Toledo SevenGo SG2, Serial No. B426752831 und der Messsonde: Mettler Toledo InLab 413 SG/2m IP67, No. 51340288 zu unterschiedlichen Zeitpunkten vermessen.
Die pH-Werte wurden durch Zugabe von HCl bzw. NaOH periodisch auf 7.40 angepasst. So konnten diese in den Grenzen von 7.2 bis 7,6 gehalten werden

Photometrie Messung

Die Photometrie Messung zur Konzentrationsmessung der Magnesium-Konzentrationen über den Versuchszeitraum wurde mit einem Spektrophotometer durchgeführt.
Die Messergebnisse sind in Figur 3 graphisch dargestellt. Im Vergleich zum Durchschnitt aus drei unbeschichteten Stents aus der Magnesiumlegierung A (BMS) zeigt sich ebenfalls eine verlangsamte Degradation. Zunächst (innerhalb der ersten zwei Stunden) löste sich bei dem $O^{2-}$ ionenimplantierten sowie bei den BMS etwa gleich viel Magnesium, erst danach löste sich bei dem ionenimplantierten weniger Magnesium. Außerdem zeigt ein Vergleich zum Flusssäure-behandelten Stent, ein schnelleres Auflösen der ionenimplantierten Stents.
Der Vergleich der Kamera-Aufnahmen in Figur 4 zeigt, dass durch die Sauerstoff-Implantation (Probe B, erste Zeile) der Stent deutlich haltbarer in der korrosiven Umgebung (PBS) ist als der unbehandelte Stent (Probe A, zweite Zeile). Auch der Stent mit $MgF_2$ Oberfläche (Probe C, dritte Zeile) degradiert deutlich langsamer als der unbehandelte Stent.

Kameraaufnahmen

Die Aufnahmen in Figur 4 bestätigen die photometrischen Messungen. Allerdings ließe sich aus den reinen Messwerten nicht erkennen, dass der $O_2$-ionenimplantierte Stent so viel länger standhält, bevor der erste Strut bricht (Bare Metal Stent: nach 2 Stunden; $MgF_2$ beschichteter Stent: 12 Stunden; $O_2$-ionenimplantierter Stent: 25 Stunden) und bis der Stent vollständig vor der Kamera degradiert ist.
Eine Erklärung könnte sein, dass bis zu der schützenden Oxid-Schicht (Magnesiumoxid), welche durch die implantierten Sauerstoff Ionen erzeugt wird, sich das Magnesium genauso schnell löst, wie bei einem unbehandelten Stent. Wenn dann die Magnesiumoxid Schicht erreicht ist, löst sich das Magnesium sehr viel langsamer. Außerdem scheint sich das Magnesium deutlich homogener, also über die gesamte Stentfläche, zu lösen. Beim Bare Metal Stent ist die Magnesiumoxid Schicht vermutlich deutlich dünner. Sie stellt nur für die ersten zwei Stunden eine Art Barriere dar. Anschließend brechen an beliebigen Stellen immer mehr Stege.
Die Fluorid Schicht hemmt in der ersten Zeit (bis ca. 25 Stunden) die Degradation am stärksten. Allerdings kommt es dennoch zu früheren Strutbrüchen. Die Magnesiumfluorid Schicht ist sehr spröde und kann beim Dilatieren des Stents teilweise aufbrechen. An diesen Stellen degradiert der Stent dann schneller.

Fazit

Bei Sauerstoff Ionen-implantierten Stents geht das Magnesium von Anfang an in Lösung. Dieses scheint aber homogener abzulaufen, da erste Strutbrüche deutlich später auftreten (nach etwa 25 Stunden) als bei vergleichbaren Stents. Zum Vergleich: Erste Strubrüche bei Bare Metal Stents treten nach etwa 2 Stunden auf, bei Flusssäure behandelten Stents nach etwa 11- 18 Stunden.

**Beispiel 10: Degradationstests von Stents nach unterschiedlichen Oberflächenbehandlungen**

Es wurden jeweils 3 Stents der Magnesiumlegierung A (Mg10Dy1Nd1Zn0,2Zr) in drei Lösungen behandelt (38-40% HF Lösung, 10% $Na_2CO_3$ Lösung und 10% $Na_3PO_4$ Lösung).
Die Behandlung in 38-40% Flusssäure wurde wie folgt vorgenommen:
Die 3 Stents wurden jeweils in einen Kunststoffbehälter gelegt. Die Kunststoffbehälter mit Deckel (aus PP) wurden mit jeweils 50 ml 38-40% Flusssäure befüllt und anschließend in ein auf 50°C temperiertes Wasserbad gelegt. Das Wasser wurde in einem Glasreservoir durch eine Heizplatte auf 50°C geheizt und während des Behandlungsprozesses bei 50°C gehalten. Durch einen Rührfisch im Reservoir konnte eine leichte Bewegung der Kunststoffbehälter im Wasserbad ermöglicht werden. Nach 24 Stunden wurden die Kunststoffbehälter dem Wasserbad entnommen. Die Stents wurden wiederum aus der Flusssäure entnommen, mit deionisiertem Wasser abgespült, mit Druckluft getrocknet und in einem Glasröhrchen verpackt.
Die Behandlung in 10% Natriumcarbonat ($Na_2CO_3$) Lösung wurde wie folgt vorgenommen:
Zunächst wurde Natriumcarbonat ($Na_2CO_3$) in deionisiertem Wasser gelöst, sodass eine 10% Lösung entstand. Der pH Wert wurde durch Zugabe von Natriumhydroxid (NaOH) angepasst. Anschließend wurden die Stents jeweils in einen Kunststoffbehälter mit Deckel (aus PP) gelegt und mit jeweils 50 ml 10% $Na_2CO_3$ Lösung befüllt. Die Behälter wurden

ebenfalls in ein auf 50°C temperiertes Wasserbad gesetzt und für 26 Stunden darin belassen. Der weitere Ablauf ist identisch zu der Flusssäurebehandlung (siehe oben).

Die Behandlung in 10%iger Natriumphosphat ($Na_3PO_4$) Lösung wurde wie folgt vorgenommen:

Zunächst wurde Natriumphosphat ($Na_3PO_4$) in deionisiertem Wasser gelöst, sodass eine 10% Lösung entstand. Anschließend wurden die Stents jeweils in einen Kunststoffbehälter mit Deckel (aus PP) gelegt und mit jeweils 50 ml 10% $Na_3PO_4$ Lösung befüllt. Die Behälter wurden ebenfalls in ein auf 50°C temperiertes Wasserbad gesetzt und für 24 Stunden darin belassen. Der weitere Ablauf ist identisch zu der Flusssäurebehandlung (siehe oben).

Die Degradationsmessungen wurden wie in Beispiel 9 durchgeführt.

Photometrische Messung

Die Ergebnisse der Photometrischen Messung sind in Figur 5 graphisch dargestellt. Die Unterschiede in der Degradationsgeschwindigkeit sind im Falle der $MgCO_3$ bzw. $Mg_3(PO_4)_2$ Proben eher gering. Eine deutliche Verzögerung der Degradation konnte nur mit der Flusssäurebehandlung bei 50°C für 24 Stunden ($MgF_2$ - Proben) erfolgen.

Auffällig ist außerdem, dass die Werte der gelösten Massen an Magnesium untereinander im Falle der Flusssäure Behandlung keine große Streuung besitzen. Die in Natriumphosphat behandelten Proben weisen die stärksten Unterschiede der Degradationsgeschwindigkeiten untereinander auf, was sich schon durch Begutachtung der Mikroskop Aufnahmen vor Degradationsbeginn vermuten ließ.

Kameraaufnahmen

Anhand der Figur 6 lässt sich sehr gut die Degradation nachvollziehen. In einer Zeile befinden sich jeweils drei Kameraaufnahmen derselben Probe.

Prinzipiell bestätigen sich die Beobachtungen aus den photometrischen Messungen. Der Stent mit Flusssäure-Behandlung (erste Zeile) löst sich deutlich langsamer auf, als die übrigen Stents. Bei diesem Stent tritt der erste Strutbruch nach 11 bis 19 Stunden auf. Eine Probe der $Mg_3(PO_4)_2$ - Gruppe löste sich bereits nach 6 Stunden vollständig vor der Kamera auf, was die Photometrie- Messung dieser Probe bestätigt.

Fazit

Eine deutliche Verbesserung der Degradationsgeschwindigkeit ließ sich nur mit der Flusssäure Behandlung erzielen. Die Zeit bis zum ersten Strutbruch konnte zumindest verfünffacht (von 2 h zu 11 - 19 h) werden. Auch der Verlauf der Degradation von $MgF_2$- oberflächenbehandelter Stents ist für die Aufrechterhaltung der radialen Festigkeit des Stents besser. Zunächst degradieren die Stents langsam (im Vergleich zum Bare Metal Stent und zur $MgCO_3$- sowie $MgPO_4$-Modifikation).

Der Vergleich der Aufnahmen zeigt, dass durch die Magnesiumfluorid Oberflächenumwandlung (Probe B, erste Zeile) der Stent deutlich haltbarer in der korrosiven Umgebung (PBS) ist als der unbehandelte Stent (Probe D, vierte Zeile). Die Magnesiumphosphat- und Magnesiumcarbonat Schicht scheint die Degradation nicht wesentlich zu verlangsamen, was sich mit den photometrischen Messungen der gelösten Massen an Magnesium über die Zeit (Figur 5) deckt.

**Beispiel 11: Degradationsexperimente für $MgF_2$-Beschichtung, hergestellt mit Ammoniumfluorid und Flusssäure jeweils bei unterschiedlicher Einwirkdauer**

Die Stents wurden auf unterschiedliche Weise behandelt (Bezeichnungen orientieren sich an Figur 10).

Probe A und C: Zwei Stents der Magnesiumlegierung A (Mg10Dy1Nd1Zn0,2Zr) wurden jeweils in einen Kunststoffbehälter gelegt. Die Kunststoffbehälter mit Deckel (aus PP) wurden mit jeweils 50 ml 38-40% Flusssäure befüllt und anschließend in ein auf 50°C temperiertes Wasserbad gelegt. Das Wasser wurde in einem Glasreservoir durch eine Heizplatte auf 50°C geheizt und während des Behandlungsprozesses bei 50°C gehalten. Durch einen Rührfisch im Reservoir konnte eine leichte Bewegung der Kunststoffbehälter im Wasserbad ermöglicht werden. Nach 5 Stunden (Probe C) bzw. nach 24 Stunden (Probe A) wurden die Kunststoffbehälter dem Wasserbad entnommen. Die Stents wurden wiederum aus der Flusssäure entnommen, mit deionisiertem Wasser abgespült, mit Druckluft getrocknet und in einem Glasröhrchen verpackt.

Probe B und D: Zunächst wurde Ammoniumfluorid ($NH_4F$) in deionisiertem Wasser gelöst, sodass eine 10% Lösung entstand. Anschließend wurden die zwei Stents der Magnesiumlegierung A (Mg10Dy1Nd1Zn0,2Zr) jeweils in einen Kunststoffbehälter mit Deckel (aus PP) gelegt und mit jeweils 50 ml 10% $NH_4F$ Lösung befüllt. Die Behälter wurden ebenfalls in ein auf 50°C temperiertes Wasserbad gesetzt und für 5 Stunden (Probe D) bzw. für 24 Stunden (Probe B) darin belassen. Der weitere Ablauf ist identisch zu der Flusssäurebehandlung (siehe Probe A und C).

Es wurden Stents aus der Magnesiumlegierung A in zwei verschiedenen Lösungen (40% Flusssäure bzw. 10%

Ammoniumfluorid) für 5 bzw. 24 Stunden bei 50 °C behandelt. Die Stents wurden in einem geschlossenen Kunststoffbehälter gelagert und mittels einer Rührplatte geschwenkt.

Nachfolgend wurden mit dem Rasterelektronenmikroskop (REM) Tescan Vega 3" mit integriertem EDX von Thermo Fischer die Zusammensetzung der Oberflächenbeschichtung bestimmt. Die Oberflächenschicht wurde unter einer Elektronenbeschleunigungsspannung von 15 kV untersucht.

Es wurden Stents mit folgenden Oberflächenbeschichtungen erhalten:

5h mit Ammoniumfluorid: 48.8% F, 32.4% Mg, 11.0% O, 7.7% C
24h mit Ammoniumfluorid: 50.3% F, 24.5% Mg, 19.7% O, 0.5% Dy, 5.1% C
5h mit Flusssäure: 54.7% F, 24.6% Mg, 10.2% O, 10.6% C
24h mit Flusssäure: 57.8% F, 24.0% Mg, 9.5% O, 8.8% C

Die Degradationsmessungen wurden wie in Beispiel 9 durchgeführt.

Die Ergebnisse sind in Figur 10 dargestellt. Der Stent, welcher für 24 Stunden in Flusssäure behandelt wurde, zeigt die langsamste Degradation in diesem Vergleich In den ersten 10 Stunden löst sich nur etwa halb so viel Magnesium wie bei dem Stent, welcher nur für 5 Stunden behandelt wurde. Die mit Ammoniumfluorid behandelten Stents unterscheiden sich untereinander nicht wesentlich. Im Vergleich zu der 24- stündigen Flusssäure Behandlung fallen die die anderen Behandlungen in Bezug auf die Verlangsamung der Degradation ab.

**Beispiel 12: Degradationstests mit Ion-implantierten Stents**

Bei der Ionenimplantation wurden Ionen (Sauerstoff, Fluor und Kohlenstoff) in Stents der Magnesiumlegierung A (Mg10Dy1Nd1Zn0,2Zr) eingebracht. Dabei werden die entsprechenden Ionen, welche mittels einer Ionenquelle erzeugt werden, durch ein elektrisches Feld stark beschleunigt und mit hoher Energie in das Substrat (in diesem Fall der Stent) geschossen. So soll eine Schutzschicht entstehen, welche den Stent vor Degradation schützt.

Für die Degradationsversuche wurden Stents mit Kohlenstoff-, Sauerstoff- und Fluor-Ionen beschossen. Für den Degradationsvergleich wurden die Stents in drei Guppen aufgeteilt. Für den Versuch wurde immer das gleiche Design und gleiches Substratmaterial (Magnesiumlegierung A) für die Stents verwendet. Alle Stents wurden lasergeschnitten, wärmebehandelt und elektropoliert. Die Gruppenbezeichnungen beziehen sich auf die Figur 11:

- Probe A: 3 Stents mit einer Sauerstoff-Implantation ($2x10^{17}$ O-Ionen pro $cm^2$)
- Probe B: 2 Stents mit einer Fluor-Implantation ($6x10^{16}$ F-Ionen pro $cm^2$)
- Probe C: 3 Stents ohne jegliche Beschichtung (Referenz)

<u>Photometrische Messung</u>

Die Degradationsmessungen wurden wie in Beispiel 9 durchgeführt und sind in Figur 11 dargestellt. Abgebildet wurden die Mittelwerte (bei Gruppe A: Mittelwert aus 3 Messwerten, bei Gruppe B: Mittelwert aus 2 Messwerten) zu den entsprechenden Zeitpunkten. Die Gruppe C wurde als Referenz wie auch bei den vorherigen Beispielen herangezogen.

Es zeigt sich bei der Gruppe A und Gruppe B eine Verzögerung der Degradationsraten im Vergleich zu den unbeschichteten Stents (Gruppe C).

<u>Fazit</u>

Der Beschuss mit Fluor und Sauerstoff-Ionen (Ionenimplantation) von Stents der Magnesiumlegierung A (Mg10Dy1Nd1Zn0,2Zr) kann die Degradationsraten im Vergleich zu unbeschichteten Stents merklich herabsenken.

**Beispiel 13: Degradationstests von Stents nach zusätzlicher Wärmebehandlung und anschließender Fluoridierung**

Anders als in Beispiel 10 wurden bei dieser Methode Stents der Magnesiumlegierung A (Mg10Dy1Nd1Zn0,2Zr) zunächst geglüht und anschließend in Flusssäure behandelt. Für den Versuch wurde das gleiche Design und gleiches Substratmaterial (Magnesiumlegierung A) für alle Stents verwendet. Alle Stents der nachfolgenden Gruppen wurden lasergeschnitten, wärmebehandelt und elektropoliert.

Fünf Stents der Magnesiumlegierung A (Mg10Dy1Nd1Zn0,2Zr) wurden zunächst (zusätzlich zu der ersten Glühung) an Luftatmosphäre für vier Stunden bei 400°C in einem Rohrofen geglüht. Anschließend wurden die Stents in zwei Gruppen aufgeteilt und unterschiedlich behandelt.

<u>Gruppe A:</u> Zwei der zuvor zusätzlich wärmbehandelten Stents wurden jeweils in einen Kunststoffbehälter gelegt.

Die Kunststoffbehälter mit Deckel (aus PP) wurden mit jeweils 50 ml 38-40% Flusssäure befüllt und verschlossen. Die Kunststoffbehälter wurden in einem Schüttelinkubator befestigt und bei 50°C und für 24 Stunden bei einer Umdrehung von 120 U/min geschüttelt. Nach der Behandlung wurden die Stents aus der Flusssäure entnommen, mit deionisiertem Wasser abgespült und in Ethanol geschwenkt. Daraufhin wurden die Stents in einem Trockenschrank bei 80°C für 30 Minuten getrocknet.

Gruppe B: Drei der zuvor zusätzlich wärmbehandelten Stents wurden identisch behandelt wie die Stents der Gruppe A, jedoch wurde eine 48%ige Flusssäure zur Fluoridierung verwendet.

Gruppe C: Unbehandelte Stents der Magnesiumlegierung A (Mg10Dy1Nd1Zn0,2Zr) mit gleichem Design und gleicher Material-Lot wie Gruppe A und B.

Photometrische Messung

Die Degradationsmessungen wurden wie in Beispiel 9 durchgeführt und sind in Figur 12 dargestellt. Bei der Probenbezeichnung A handelt es sich um die Mittelwerte aus den Messwerten der zwei wie oben beschrieben behandelten Stents. Bei der Probe B handelt es sich als Referenz um Mittelwerte aus den Messwerten dreier unbeschichteter Stents des gleichen Designs und ebenfalls aus der Magnesiumlegierung A (Mg10Dy1Nd1Zn0,2Zr).

Es zeigt sich anhand der in Lösung gegangener Masse an Magnesium-Ionen über die Zeit (Figur 12), dass die fluoridierten Stents der Gruppe A und B eine deutliche verringerte Degradationsrate aufweisen als die unbeschichteten Stents (Gruppe C). Gerade innerhalb der ersten 5 Stunden zeigt sich, dass die Stents der Gruppe A und B nur etwa ein Drittel der Masse an Magnesium-Ionen freigesetzt haben als die unbeschichteten Stents. Weiter konnte die Degradationszeit bei Gruppe B im Vergleich zur Gruppe C insgesamt deutlich verlängert werden.

Fazit

Eine zusätzliche Glühung in Luftatmosphäre und anschließender Fluoridierung konnte die Degradationsgeschwindigkeit der Stents während der ersten Stunden deutlich reduzieren, was in Bezug auf das Einwachsen des Stents in-vivo von Vorteil sein könnte. Durch die Fluoridierung der Stents in 48% Flusssäure konnte nicht nur die anfängliche Degradationsgeschwindigkeit herabgesetzt werden, es wurde auch die gesamte Degradationszeit verlangsamt.

**Beispiel 14: Degradationstests von Stents mit einer Zwischenschicht aus Magnesiumfluorid und einer weiteren Beschichtung aus Parylen C.**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert.

In diesem Versuch sollte gezeigt werden, welchen Einfluss eine Zwischenschicht aus Magnesiumfluorid unter einer 2,5 μm-Schicht aus Parylen C hat.

Dazu wurden zwei Gruppen von beschichteten Stents gegenübergestellt:

Gruppe A: Stents der Magnesiumlegierung A (Mg10Dy1Nd1Zn0,2Zr) wurden jeweils in einen Kunststoffbehälter gelegt. Die Kunststoffbehälter mit Deckel (aus PP) wurden mit jeweils 50 ml 38-40% Flusssäure befüllt und anschließend in ein auf 50°C temperiertes Wasserbad gelegt. Das Wasser wurde in einem Glasreservoir durch eine Heizplatte auf 50°C geheizt und während des Behandlungsprozesses bei 50°C gehalten. Durch einen Rührfisch im Reservoir konnte eine leichte Bewegung der Kunststoffbehälter im Wasserbad ermöglicht werden. Nach 24 Stunden wurden die Kunststoffbehälter dem Wasserbad entnommen. Die Stents wurden wiederum aus der Flusssäure entnommen, mit deionisiertem Wasser abgespült, mit Druckluft getrocknet und in einem Glasröhrchen verpackt. Die Schichtdicke der Fluorid-Schicht betrug 1-2 μm.

Anschließend wurden die Stents mit chloriertem Poly-p-xylylen (Parlyen C) beschichtet. Parylen C kann direkt aus der Gasphase auf dem Substrat abgeschieden werden, was zu einer sehr gleichmäßigen Beschichtung führt. Über die Behandlungsdauer lässt sich die Schichtdicke variieren.

Gruppe B: Die Stents der Gruppe B wurden direkt (also ohne eine Zwischenschicht aus Magnesiumfluorid) mit dem Polymer Parylen C beschichtet. Die Schichtdicke des Polymers war gleich, da diese im selben Beschichtungszyklus beschichtet wurden wie die Stents der Gruppe A.

Photometrische Messung

Die Degradationsmessungen wurden wie in Beispiel 9 durchgeführt und sind in Figur 13 dargestellt. Bei der Probenbezeichnung A handelt es sich um die Mittelwerte aus den Messwerten der gelösten Masse an Magnesium zweier Stents der Gruppe A. Bei der Probe B handelt es sich um Mittelwerte aus den Messwerten der gelösten Masse an Magnesium zweier Stents der Gruppe B.

Die Messung der in Lösung gegangener Magnesium-Ionen zeigt, dass die Stents der Gruppe A (Stents mit einer Zwischenschicht aus Magnesiumfluorid) deutlich langsamer degradieren als Stents der Gruppe B (ohne Zwischenschicht aus Magnesiumfluorid). In etwa zeigt sich eine Halbierung der Degradationsgeschwindigkeit.

Fazit

Die Schichtdicke, die Schichtaufbringung und die Schichthomogenität des Polymers waren bei beiden Gruppen identisch, da diese im gleichen Beschichtungszyklus parallel beschichtet wurden. Das heißt, die langsamere Degradationsrate der Stents der Gruppe A im Vergleich zur Gruppe B kann allein auf die Zwischenschicht aus Magnesiumfluorid zurückgeführt werden. Die Magnesiumfluorid-Schicht eignet sich also auch als Zwischenschicht für weitere Beschichtungen (wie beispielsweise Polymer-Beschichtungen).

**Beispiel 15: Degradationstests von Stents mit einer Zwischenschicht aus Magnesiumfluorid und einer weiteren Beschichtung aus einem resorbierbaren Polymer**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert. Die Schichtdicken des jeweilig aufgebrachten Polymers waren bei allen Gruppen gleich.

Gruppe A: Stents der Magnesiumlegierung A (Mg10Dy1Nd1Zn0,2Zr) mit einer Schicht aus Poly- L-Lactid (PLLA), welche im Sprühverfahren auf den Stent gebracht wurde.

Gruppe B: Stents der Magnesiumlegierung A (Mg10Dy1Nd1Zn0,2Zr) mit einer Schicht aus Poly-ε-Caprolacton (PCL), welche im Sprühverfahren auf den Stent gebracht wurde.

Gruppe C: Stents der Magnesiumlegierung A (Mg10Dy1Nd1Zn0,2Zr) mit einer Schicht aus Poly-L-Lactid (PLLA), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

Gruppe D: Stents der Magnesiumlegierung A (Mg10Dy1Nd1Zn0,2Zr) mit einer Schicht aus Poly-ε-Caprolacton (PCL), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

Photometrische Messung

Die Degradationsmessungen wurden wie in Beispiel 9 durchgeführt und sind in Figur 14 dargestellt. Bei der Probenbezeichnung A handelt es sich um die Messwerte der gelösten Masse an Magnesium eines Stents der Gruppe A.

Bei der Probe B handelt es sich die Messwerte der gelösten Masse an Magnesium eines Stents der Gruppe B.

Bei der Probe C handelt es sich um Mittelwerte aus den Messwerten der gelösten Masse an Magnesium zweier Stents der Gruppe C.

Bei der Probe D handelt es sich um Mittelwerte aus den Messwerten der gelösten Masse an Magnesium zweier Stents der Gruppe D.

Die Messung der in Lösung gegangener Magnesium-Ionen (Figur 14) zeigt, dass die Stents mit einer Zwischenschicht aus Magnesiumfluorid (Gruppe C und D) deutlich langsamer degradieren als die Stents ohne Zwischenschicht. Die Kombination Magnesiumfluorid und Poly-ε-Caprolacton (Gruppe D) zeigt in diesem Vergleich die langsamste Degradationsrate gefolgt von Magnesiumfluorid und Poly-l-lactid (Gruppe C).

Fazit

In diesem Versuch konnte gezeigt werden, dass durch eine Zwischenschicht aus Magnesiumfluorid die Degradationszeit von Stents der Magnesiumlegierung A mit einer resorbierbaren Polymerbeschichtung weiter erheblich verlangsamt werden kann. Die Schichtdicke des jeweiligen Polymers war bei allen Stents identisch (10 μm). Durch die Zwischenschicht aus Magnesiumfluorid konnte sowohl die Degradationszeit bei den mit PLLA beschichteten Stents (Gruppe A vs. Gruppe C) als auch bei den mit PCL beschichteten Stents (Gruppe B vs. Gruppe D) verlangsamt werden.

Eine Schicht aus Magnesiumfluorid auf einem Stent der Magnesiumlegierung A (Mg10Dy1Nd1Zn0,2Zr) eignet sich also auch als Zwischenschicht für eine resorbierbare Polymer-Beschichtung, um die Degradationsgeschwindigkeit erheblich zu verlangsamen.

**Beispiel 16: Degradationstests von Stents mit einer Zwischenschicht aus Magnesiumfluorid und einer weiteren Beschichtung aus Poly-L-Lactid (PLLA) und eingebrachtem Medikament**

Für den Versuch wurde das gleiche Design und gleiche Substratmaterial (Magnesiumlegierung A) für alle Stents

verwendet. Alle Stents der nachfolgenden Gruppen wurden lasergeschnitten, wärmebehandelt und elektropoliert.

Gruppe A: Stents der Magnesiumlegierung A (Mg10Dy1Nd1Zn0,2Zr) mit einer 7 μm Schicht aus Poly-L-Lactid (PLLA) mit eingebrachtem Medikament (Rapamycin), welche im Sprühverfahren aufgebracht wurde und einer Zwischenschicht aus Magnesiumfluorid. Die Magnesiumfluorid-Schicht wurde wie in Beispiel 13 (Gruppe A) auf die Stents gebracht.

Gruppe B: Stents der Magnesiumlegierung A (Mg10Dy1Nd1Zn0,2Zr) mit einer 7 μm Schicht aus Poly-L-Lactid (PLLA) mit eingebrachtem Medikament (Rapamycin), welche im Sprühverfahren auf den Stent gebracht wurde.

Photometrische Messung

Die Degradationsmessungen wurden wie in Beispiel 9 durchgeführt und sind in Figur 16 dargestellt. Bei der Probenbezeichnung A handelt es sich um Mittelwerte der Messwerte der gelösten Masse an Magnesium zweier Stents der Gruppe A.

Bei der Probe B handelt es sich um Mittelwerte der Messwerte der gelösten Masse an Magnesium dreier Stents der Gruppe B.

Die photometrische Konzentrationsmessung (Figur 16) zeigt, dass die Stents mit Zwischenschicht aus Magnesiumfluorid langsamer degradieren als die Stents ohne einer solchen Schicht bei gleicher Schichtdicke der Polymerbeschichtung (7 μm).

Fazit

In diesem Versuch konnte gezeigt werden, dass durch eine Zwischenschicht aus Magnesiumfluorid die Degradationszeit von Stents der Magnesiumlegierung A mit einer resorbierbaren Polymerbeschichtung aus Poly-L-Lactid (PLLA) und einer Schichtdicke von 7 μm erheblich verlangsamt werden kann.

**Beispiel 17: Degradationstests von Stents mit einer Zwischenschicht aus Magnesiumfluorid und einer weiteren Beschichtung aus Poly-ε-Caprolacton (PCL) mit eingebrachtem Medikament und unterschiedlichen Schichtdicken**

Für den Versuch wurde das gleiche Design und gleiches Substratmaterial (Magnesiumlegierung A) für alle Stents verwendet. Alle Stents der nachfolgenden Gruppen wurden lasergeschnitten, wärmebehandelt, und elektropoliert.

Gruppe A: Stents der Magnesiumlegierung A (Mg10Dy1Nd1Zn0,2Zr) mit einer 4 μm Schicht aus Poly-ε-Caprolacton (PCL), welche im Sprühverfahren auf den Stent gebracht wurde.

Gruppe B: Stents der Magnesiumlegierung A (Mg10Dy1Nd1Zn0,2Zr) mit einer 7 μm Schicht aus Poly-ε-Caprolacton (PCL), welche im Sprühverfahren auf den Stent gebracht wurde.

Gruppe C: Stents der Magnesiumlegierung A (Mg10Dy1Nd1Zn0,2Zr) mit einer 4 μm Schicht aus Poly-ε-Caprolacton (PCL), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

Gruppe D: Stents der Magnesiumlegierung A (Mg10Dy1Nd1Zn0,2Zr) mit einer 7 μm Schicht aus Poly-ε-Caprolacton (PCL), welche im Sprühverfahren auf den Stent gebracht wurde und einer Zwischenschicht aus Magnesiumfluorid. Die Magnesiumfluorid-Schicht wurde wie in Beispiel 13 (Gruppe A) auf die Stents gebracht.

Photometrische Messung

Die Degradationsmessungen wurden wie in Beispiel 9 durchgeführtund sind in Figur 15 dargestellt. Bei der Probenbezeichnung A handelt es sich um die Messwerte der gelösten Masse an Magnesium eines Stents der Gruppe A.

Bei der Probe B handelt es sich die Messwerte der gelösten Masse an Magnesium eines Stents der Gruppe B.

Bei der Probe C handelt es sich um Mittelwerte aus den Messwerten der gelösten Masse an Magnesium zweier Stents der Gruppe C.

Bei der Probe D handelt es sich um Mittelwerte aus den Messwerten der gelösten Masse an Magnesium zweier Stents der Gruppe D.

Die photometrische Konzentrationsmessung (Figur 15) zeigt, dass die Stents mit Zwischenschicht aus Magnesiumfluorid langsamer degradieren als die Stents ohne einer solchen Schicht bei gleicher Schichtdicke der Polymer Beschichtung (4 μm bzw. 7 μm).

**Beispiel 18: Degradationstests von Stents mit einer anorganischen und einer weiteren Beschichtung aus einem resorbierbaren Polymer bei unterschiedlicher Zusammensetzung der anorganischen Beschichtung**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert.

Gruppe A: Stents der Magnesiumlegierung A (Mg10Dy1Nd1Zn0,2Zr) mit einer anorganischen Beschichtung (Oberflächenumwandlung) aus Magnesiumhydroxid und einer 5 μm Schicht aus Poly-L-Lactid (PLLA), die im Sprühverfahren aufgebracht wurde. Die Magnesiumhydroxid Oberfläche wurde in einem nasschemischen auf die Stents gebracht. Dazu wurden die polierten Stents für 2 min in 30%ige H2O2 Lösung bei Raumtemperatur getaucht. Anschließend wurden diese mit H2O gespült und in Ethanol getaucht und bei 80°C für eine Stunde in einem Trockenschrank getrocknet.

Gruppe B: Stents der Magnesiumlegierung A (Mg10Dy1Nd1Zn0,2Zr) mit einer anorganischen Beschichtung (Oberflächenumwandlung) aus Magnesiumcarbonat/ Magnesiumhydroxid und einer 5 μm Schicht aus Poly-L-Lactid (PLLA), die im Sprühverfahren aufgebracht wurde. Die Oberflächenumwandlung wurde mittels eines nasschemischen Verfahrens realisiert. Dabei wurden wie polierten Stents in gesättigte NaHCO3-Lösung für 5 min und 37°C getaucht. Anschließend wurden die Stents mit H2O gespült und in Ethanol getaucht. Darauf folgte die Trocknung bei 100°C für eine Stunde im Trockenschrank.

Gruppe C: Stents der Magnesiumlegierung A (Mg10Dy1Nd1Zn0,2Zr) mit einer anorganischen Beschichtung (Oberflächenumwandlung) aus Magnesiumphosphat/ Magnesiumhydroxid und einer 10 μm Schicht aus Poly-L-Lactid (PLLA), die im Sprühverfahren aufgebracht wurde.

Die Oberflächenumwandlung wurde mittels eines nasschemischen Verfahrens realisiert. Dabei wurden wie polierten Stents in gesättigte Na2HPO4-Lösung für eine Stunde und 37°C getaucht. Anschließend wurden die Stents mit H2O gespült und in Ethanol getaucht. Darauf folgte die Trocknung bei 100°C für eine Stunde im Trockenschrank.

Gruppe D: Stents der Magnesiumlegierung A (Mg10Dy1Nd1Zn0,2Zr) mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe A) auf die Stents gebracht.

Gruppe E: Stent der Magnesiumlegierung A (Mg10Dy1Nd1Zn0,2Zr) mit einer anorganischen Beschichtung (Oberflächenumwandlung) aus Magnesiumfluorid. Die Magnesiumfluorid-Schicht wurde wie in Beispiel 13 (Gruppe A) auf die Stents gebracht.

Photometrische Messung

Die Degradationsmessungen wurden wie in Beispiel 9 durchgeführt und sind in Figur 17 dargestellt. Bei der Probenbezeichnung A handelt es sich um die Messwerte der gelösten Masse an Magnesium bezogen auf die Ausgangsoberfläche eines Stents der Gruppe A (A1 und A2).

Bei der Probe B handelt es sich um die Messwerte der gelösten Masse an Magnesium bezogen auf die Ausgangsoberfläche eines Stents der Gruppe B (B1 und B2).

Bei der Probe C handelt es sich um Messwerte der gelösten Masse an Magnesium bezogen auf die Ausgangsoberfläche zweier Stents der Gruppe C (C1 und C2).

Bei der Probe D handelt es sich um Messwerte der gelösten Masse an Magnesium bezogen auf die Ausgangsoberfläche zweier Stents der Gruppe D (D1 und D2).

Bei der Probe E handelt es sich um Messwerte der gelösten Masse an Magnesium bezogen auf die Ausgangsoberfläche eines Stents der Gruppe E.

Die photometrische Konzentrationsmessung (Figur 17) zeigt, dass die Stents mit Zwischenschicht aus Magnesiumfluorid langsamer degradieren als Stents mit anderen Zwischenschichten.

Fazit

In diesem Versuch konnte gezeigt werden, dass durch eine anorganische Beschichtung aus Magnesiumfluorid auf dem Stent die Degradationszeit von Stents der Magnesiumlegierung A mit einer resorbierbaren Polymerbeschichtung im Vergleich zu anorganischen Beschichtungen bestehend aus Magnesiumhydroxid, Magnesiumcarbonat und Magnesiumphosphat erheblich verlangsamt werden kann.

Eine Beschichtung aus Magnesiumfluorid und eine darüber liegende Beschichtung aus Poly-L-Lactid (PLLA) auf einem Stent der Magnesiumlegierung A (Mg10Dy1Nd1Zn0,2Zr) eignet sich besonders, um die Degradationsgeschwindigkeit erheblich zu verlangsamen.

**Beispiel 19: Beschichtung von Stents mit einem bioresorbierbaren Polymer und einer darauf liegenden Magnesiumfluorid-Schicht**

Die Stents (Magnesiumlegierung A) wurden lasergeschnitten, wärmebehandelt, und elektropoliert. Anschließend erfolgte die Aufbringung des Polymers (PLLA) mit eingebettetem Medikament (Rapamycin, 1,4 μg / $mm^2$) im Sprühverfahren. Die darauf folgende Aufbringung einer Magnesiumfluorid-Schicht erfolgte mit IBAD (ion beam assisted deposition). Der IBAD Prozess umfasst eine dünne Schichtbildung unter simultanem Ionen-Bombardement dieser Schicht aus einer Ionenquelle. In diesem Fall erfolgte die Schichtbildung durch Verdampfen von Magnesiumfluorid aus einem Molybdän-Tiegel unter einem Druck von z.B. 1 E-05 Pa. Die Aufbringungsrate kann variiert werden und liegt z.B. zwischen 0,3 und 1,5 nm / s. Ebenfalls kann die Schichtdicke über die Zeit eingestellt werden. In dem vorliegenden Fall lag die Schichtdicke bei etwa 800 nm. Die Temperatur der Probe blieb stets unterhalb von 70 °C, um die Eigenschaften des Polymers und des Medikaments nicht zu beeinflussen. Die vorliegende Magnesiumfluorid-Schicht erwies sich als spröde Schicht. Beim Krimpen der Stents vom Ausgangsdurchmesser von 1,8 mm auf 1,1 mm ließen sich bereits mittels Lichtmikroskops Risse und sogar Ablösungen der $MgF_2$-Schicht feststellen. Beim darauffolgenden Dilatieren auf einen Durchmesser von 3,2 mm konnte festgestellt werden, dass sich in Bereichen der größten Umformung des Stents die $MgF_2$-Schicht vom Polymer abgelöst hatte. Es kann deshalb davon ausgegangen werden, dass diese Schicht in dieser Kombination nicht zu einer Degradationsverzögerung führt. Ein Abplatzen der $MgF_2$-Schicht vom Polymer kann außerdem zu einer lokalen und somit nicht gleichmäßigen Freisetzung des Medikaments führen. Abgeplatzte Teile könnten ebenfalls zu einer Embolie führen.

**Beispiel 20: Degradationstests von Stents mit einer Zwischenschicht aus Magnesiumfluorid und einer weiteren Beschichtung aus Parylen N.**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert.

In diesem Versuch sollte gezeigt werden, welchen Einfluss eine Zwischenschicht aus Magnesiumfluorid unter einer 0,1 μm-Schicht aus Parylen N hat.

Dazu wurden zwei Gruppen von beschichteten Stents gegenübergestellt:

<u>Gruppe A:</u> Zwei Stents der Magnesiumlegierung A (Mg10Dy1Nd1Zn0,2Zr) wurden wie in Beispiel 13 Gruppe B behandelt, um eine Magnesiumfluoridschicht zu erhalten.

Anschließend wurden die Stents mit dem Polymer Parylen N (Poly-p-xylylen) beschichtet. Parylen N kann direkt aus der Gasphase durch Kondensation auf dem Substrat abgeschieden werden, was zu einer sehr gleichmäßigen Beschichtung führt. Über die Behandlungsdauer lässt sich die Schichtdicke variieren.

<u>Gruppe B:</u> Die zwei Stents der Gruppe B wurden direkt (also ohne eine Zwischenschicht aus Magnesiumfluorid) mit dem Polymer Parylen N beschichtet. Die Schichtdicke des Polymers war gleich, da diese im selben Beschichtungszyklus beschichtet wurden wie die Stents der Gruppe A.

<u>Gruppe C:</u> Die zwei Stents wurden behandelt wie in Gruppe A, allerdings wurde kein Polymer appliziert.

<u>Gruppe D:</u> Die drei Stents wiesen keinerlei Beschichtung auf (keine Magnesiumfluorid-Schicht und keine Polymer-Schicht).

<u>Photometrische Messung</u>

Die Degradationsmessungen wurden wie in Beispiel 9 durchgeführt und sind in Figur 18 dargestellt. Bei der Probenbezeichnung A handelt es sich um die Mittelwerte aus den Messwerten der gelösten Masse an Magnesium zweier Stents der Gruppe A. Bei der Probe B handelt es sich um Mittelwerte aus den Messwerten der gelösten Masse an Magnesium zweier Stents der Gruppe B. Bei der Probe C handelt es sich um Mittelwerte aus den Messwerten der gelösten Masse an Magnesium zweier Stents der Gruppe C. Bei der Probe D handelt es sich um Mittelwerte aus den Messwerten der gelösten Masse an Magnesium dreier Stents der Gruppe D.

Die Messung der in Lösung gegangenen Magnesium-Ionen zeigt, dass die Stents der Gruppe A (Stents mit einer Zwischenschicht aus Magnesiumfluorid) deutlich langsamer degradieren als Stents der Gruppe B (ohne Zwischenschicht aus Magnesiumfluorid). In etwa zeigt sich eine 2,5-fache Verringerung der Degradationsgeschwindigkeit. Die Stents der Gruppe A degradieren in etwa gleich schnell wie die Stents der Gruppe C. Die Stents der Gruppe D degradieren am schnellsten.

<u>Fazit</u>

Die Schichtdicke, die Schichtaufbringung und die Schichthomogenität des Polymers waren bei beiden Gruppen identisch, da diese im gleichen Beschichtungszyklus parallel beschichtet wurden. Das heißt, die langsamere Degradationsrate der Stents der Gruppe A im Vergleich zur Gruppe B kann allein auf die Zwischenschicht aus Magnesiumfluorid zurückgeführt werden. Durch den Vergleich zu unbeschichteten sowie zu nur mit einer Magnesiumfluorid-Schicht versehenen Stents wird ersichtlich, dass ausschließlich durch die Kombination aus Zwischenschicht (Magnesiumfluorid) und Polymer (in diesem Fall Parylen N) eine derartige Verringerung der Degradation möglich ist. Die einzelnen Beschichtungen zeigen alleinig aufgebracht nicht die entsprechende Degradationshemmung.

**Beispiel 21: Degradationstests von Stents mit einer Zwischenschicht aus Magnesiumfluorid und einer weiteren Beschichtung aus Polyethylenimin (PEI) und Polyacrylsäure (PAA).**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert.

In diesem Versuch sollte gezeigt werden, welchen Einfluss eine Zwischenschicht aus Magnesiumfluorid unter einer Doppel-Schicht aus Polyethylenimin (PEI, Mw 25.000) und Polyacrylsäure (PAA, Mv 450.000) hat. Es wurden Stents mit und ohne einer Zwischenschicht aus Magnesiumfluorid miteinander verglichen. Die Magnesiumfluoridschicht wurde wie in Beispiel 20 (Gruppe A) aufgebracht.

Die Doppelschicht aus PEI und PAA wurde im Layer-by-Layer Verfahren (Schicht für Schicht) aufgebracht. Die Stents (unbeschichtet und mit Magnesiumfluorid beschichtet) wurden nacheinander in eine wässrige Lösung mit 5 mg/ml PAA für zwei Minuten getaucht, anschließend für eine Minute in deionisiertes Wasser, daraufhin für zwei Minuten in eine wässrige Lösung mit 5 mg/ml PEI und abschließend für eine Minute in deionisiertes Wasser getaucht. Diese Abfolge wurde insgesamt fünf beziehungsweise zehn Mal wiederholt und die Stents daraufhin an Luft getrocknet.

Gruppe A: Ein Stent der Magnesiumlegierung A (Mg10Dy1Nd1Zn0,2Zr) und einer Schicht aus PEI und PAA. Die Doppelschicht aus PEI und PAA wurde im Layer-by-Layer Verfahren (Schicht für Schicht) aufgebracht. Der Stent wurde nacheinander in eine wässrige Lösung mit 5 mg/ml PAA für zwei Minuten getaucht, anschließend für eine Minute in deionisiertes Wasser, daraufhin für zwei Minuten in eine wässrige Lösung mit 5 mg/ml PEI und abschließend für eine Minute in deionisiertes Wasser getaucht. Diese Abfolge wurde insgesamt fünf Mal wiederholt und der Stent daraufhin an Luft getrocknet.

Gruppe B: Ein Stent der Magnesiumlegierung A (Mg10Dy1Nd1Zn0,2Zr), welcher wie in Beispiel 13 Gruppe B behandelt wurde um eine Zwischenschicht aus Magnesiumfluorid zu erzeugen. Anschließend folgte die gleiche Beschichtungsabfolge wie bei Stent A (5x PEI und PAA).

Gruppe C: Gleichartig beschichteter Stent wie in Gruppe A allerdings mit 10 Beschichtungsdurchgängen zur Erzeugung der PEI- und PAA-Schicht.

Gruppe D: Gleichartig beschichteter Stent wie in Gruppe B allerdings mit 10 Beschichtungsdurchgängen zur Erzeugung der PEI- und PAA-Schicht.

Gruppe E: Stents der Magnesiumlegierung A (Mg10Dy1Nd1Zn0,2Zr) ohne jegliche weitere Beschichtung.

Photometrische Messung

Die Degradationsmessungen wurden wie in Beispiel 9 durchgeführt und sind in Figur 19 dargestellt. Die Bezeichnungen der Messwerte entsprechen denen der Stentbezeichnungen. Bei den Messwerten der Gruppe E handelt es sich um Mittelwerte aus den Messwerten dreier Stents.

Die Stents mit einer Dreifachbeschichtung aus Magnesiumfluorid und PEI und PAA degradieren insgesamt langsamer als die, die nur eine PEI und PAA Schicht aufweisen.

Fazit

In diesem Versuch konnte gezeigt werden, dass eine Zwischenschicht aus Magnesiumfluorid auch im Falle einer Verwendung von Polyacrylsäure und Polyethylenimin als Beschichtung zusätzlich degradationshemmend wirkt, wenn diese mit Stents ohne einer Zwischenschicht aus Magnesiumfluorid vergleichen werden.

**Beispiel 22: Degradationstests von Stents unterschiedlicher Legierungen mit und ohne einer Zwischenschicht aus Magnesiumfluorid und einer weiteren Beschichtung aus Poly-L-Lactid.**

Für den Versuch wurde das gleiche Design für alle Stents verwendet. Alle Stents der nachfolgenden Gruppen wurden lasergeschnitten, wärmebehandelt und elektropoliert. Die Stents unterschieden sich teilweise in der verwendeten Magnesium Legierung.

Gruppe A: Stents der Magnesiumlegierung A (Mg10Dy1Nd1Zn0,2Zr) mit einer 5 μm Schicht aus Poly-L-Lactid (PLLA), die im Sprühverfahren aufgebracht wurde.

Gruppe B: Stents der Magnesiumlegierung A (Mg10Dy1Nd1Zn0,2Zr) mit einer anorganischen Beschichtung

(Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe B) auf die Stents gebracht.

Gruppe C: Stents der Magnesiumlegierung L37 mit einer 5 μm Schicht aus Poly-L-Lactid (PLLA), die im Sprühverfahren aufgebracht wurde.

Gruppe D: Stents der Magnesiumlegierung L37 mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe B) auf die Stents gebracht.

Gruppe E: Stents der Magnesiumlegierung AZ91 (Mg9Al1Zn) mit einer 5 μm Schicht aus Poly-L-Lactid (PLLA), die im Sprühverfahren aufgebracht wurde.

Gruppe F: Stents der Magnesiumlegierung AZ91 (Mg9Al1Zn) mit einer anorganischen Beschichtung (Oberflächenumwandlung) aus Magnesiumfluorid und einer 5 μm Schicht aus Poly-L-Lactid (PLLA), die im Sprühverfahren aufgebracht wurde. Die Magnesiumfluorid-Schicht wurde wie in Beispiel 13 (Gruppe B) auf die Stents gebracht.

Photometrische Messung

Die Degradationsmessungen wurden wie in Beispiel 9 durchgeführt und sind in Figur 20 dargestellt.

Bei der Probenbezeichnung A handelt es sich um Mittelwerte der gelösten Masse an Magnesium bezogen auf die Zeit von jeweils zwei Proben aus der Gruppe A.

Das gleiche gilt für die weiteren Proben (B, C, D, E, F).

Für alle hier verwendeten Legierungen gilt, dass Stents dieser Legierung langsamer korrodieren, sofern eine Zwischenschicht aus Magnesiumfluorid appliziert wurde (vergleiche A und B, sowie C und D sowie E und F). Stents der Legierung L37 degradierten insgesamt schneller als die der Legierungen A und AZ91 (Mg9Al1Zn).

Fazit

In diesem Versuch konnte gezeigt werden, dass eine Zwischenschicht aus Magnesiumfluorid in Verbindung mit einer organischen Beschichtung auch im Falle unterschiedlicher Legierungen als Stentmaterial die Degradation entscheidend verlangsamt. Wird keine Zwischenschicht appliziert, so ist die Degradationsrate deutlich schneller.

Dieser Versuch zeigt, dass die eine Zwischenschicht aus Magnesiumfluorid auch auf andere Magnesiumlegierungen außer der Legierung A anwendbar ist.

**Beispiel 23: Degradationstests von Stents mit und ohne einer Zwischenschicht aus Magnesiumfluorid und einer weiteren Beschichtung aus Poly(lactid-co-glycolid) (PLGA)**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert.

Gruppe A: Stents der Magnesiumlegierung A (Mg10Dy1Nd1Zn0,2Zr) mit einer Schicht aus Poly(lactid-co-glycolid) (PLGA), die mittels Dip-Coating aufgebracht wurde. Dabei wurde der Stent in eine Lösung aus PLGA (85:15) und Trichlormethan (5 mg/ml) getaucht, mit 20 mm/min aus der Lösung gezogen und anschließend bei 40°C an Luft getrocknet.

Gruppe B: Stents der Magnesiumlegierung A (Mg10Dy1Nd1Zn0,2Zr) mit einer anorganischen Beschichtung (Oberflächenumwandlung) aus Magnesiumfluorid und einer darüberliegenden Schicht aus Poly(lactid-co-glycolid) (PLGA). Die Magnesiumfluorid Beschichtung wurde wie in Beispiel 13 Gruppe B aufgebracht. Die Aufbringung der PLGA Beschichtung erfolgte wie bei der Gruppe A.

Gruppe C: Stents der Magnesiumlegierung A (Mg10Dy1Nd1Zn0,2Zr) ohne jegliche Beschichtung.

Photometrische Messung

Die Degradationsmessungen wurden wie in Beispiel 9 durchgeführt und sind in Figur 21 dargestellt.

Bei der Probenbezeichnung A handelt es sich um Mittelwerte der gelösten Masse an Magnesium bezogen auf die Zeit von jeweils zwei Proben aus der Gruppe A.

Das gleiche gilt für die weiteren Proben (B,C).

Die beschichteten Stents degradieren langsamer als die unbeschichteten, wobei die mit einer Zwischenschicht aus Magnesiumfluorid am langsamsten degradieren.

Fazit

In diesem Versuch konnte gezeigt werden, dass eine Zwischenschicht aus Magnesiumfluorid in Verbindung mit

einer organischen Beschichtung (in diesem Fall PLGA) deutlich langsamer degradieren als Stents ohne eine solche bzw. ohne jegliche Beschichtung. Der Versuch zeigt, dass die Kombination aus Magnesiumfluorid als Zwischenschicht und PLGA als darüberliegende organische Schicht besonders geeignet ist, die Degradation zu verlangsamen.

**Beispiel 24: Degradationstests von Stents mit und ohne einer Zwischenschicht aus Magnesiumfluorid und einer weiteren Beschichtung aus Polymethacrylamid (PMAA)**

Für den Versuch wurde das gleiche Design und gleiche Substratmaterial (Magnesiumlegierung A) für alle Stents verwendet. Alle Stents der nachfolgenden Gruppen wurden lasergeschnitten, wärmebehandelt und elektropoliert.
Gruppe A: Drei Stents der Magnesiumlegierung A (Mg10Dy1Nd1Zn0,2Zr) mit einer Schicht aus PMAA, welche mittels Dip-Coating aufgebracht wurde (A1, A2, A3).
Gruppe B: Stents der Magnesiumlegierung A (Mg10Dy1Nd1Zn0,2Zr) mit einer anorganischen Beschichtung (Oberflächenumwandlung) aus Magnesiumfluorid und einer darüberliegenden Schicht aus PMAA (B1, B2, B3).
Gruppe C: Stents der Magnesiumlegierung A (Mg10Dy1Nd1Zn0,2Zr) ohne jegliche Beschichtung.

Photometrische Messung

Die Degradationsmessungen wurden wie in Beispiel 9 durchgeführt und sind in Figur 22 dargestellt.
Bei der Probenbezeichnung C handelt es sich um Mittelwerte der gelösten Masse an Magnesium bezogen auf die Zeit von drei Proben aus der Gruppe C.
Die doppelt beschichteten Stents (Magnesiumfluorid und PMAA) degradieren langsamer als die unbeschichteten. Die ausschließlich mit PMAA beschichteten Stents zeigen keine Verzögerung der Degradationsrate im Vergleich zu unbeschichteten Stents.

Fazit

In diesem Versuch konnte gezeigt werden, dass eine Zwischenschicht aus Magnesiumfluorid in Verbindung mit einer organischen Beschichtung (in diesem Fall PMAA) langsamer degradieren als Stents ohne eine solche bzw. ohne jegliche Beschichtung. Der Versuch zeigt, dass die Kombination aus Magnesiumfluorid als Zwischenschicht und PMAA als darüberliegende organische Schicht geeignet ist, die Degradation zu verlangsamen (zumindest in den ersten Stunden des Versuches). Allerdings sind andere Polymere als darüberliegende Schicht besser geeignet.

**Beispiel 25: Degradationstests von Stents mit und ohne einer Zwischenschicht aus Magnesiumfluorid und einer weiteren Beschichtung aus Poly-L-Lactid im Vergleich zu Stents der Legierung L37 mit einer Beschichtung aus Poly-L-Lactid**

Für den Versuch wurde das gleiche Design und gleiche Substratmaterial (Magnesiumlegierung A) für alle Stents der Gruppen B und C verwendet. Das verwendete Material der Stents der Gruppe A war die Legierung L37. Alle Stents wurden lasergeschnitten, wärmebehandelt und elektropoliert und anschließend beschichtet.
Gruppe A: Zwei Stents der Magnesiumlegierung L37 mit einer Beschichtung aus PLLA und einer abluminalen Schichtdicke von 6-12 μm.
Gruppe B: Zwei Stents der Magnesiumlegierung A (Mg10Dy1Nd1Zn0,2Zr) mit einer Beschichtung aus 7,5 μm PLLA und eingebrachtem Medikament (Rapamycin, 1,4 $\mu g/mm^2$). Die Aufbringung der PLLA-Beschichtung erfolgte im Sprühverfahren.
Gruppe C: Stents der Magnesiumlegierung A (Mg10Dy1Nd1Zn0,2Zr) mit einer anorganischen Beschichtung (Oberflächenumwandlung) aus Magnesiumfluorid und einer darüberliegenden Schicht aus 7,5 μm PLLA mit eingebrachtem Medikament (Rapamycin, 1,4 $\mu g/mm^2$). Die Magnesiumfluorid Beschichtung wurde wie in Beispiel 13, Gruppe B aufgebracht. Die Aufbringung der PLLA Beschichtung erfolgte im Sprühverfahren.

Photometrische Messung

Die Degradationsmessungen wurden wie in Beispiel 9 durchgeführt und sind in Figur 23 dargestellt. Da hier Stents mit unterschiedlichen Designs verglichen wurden, wurde die Masse an gelösten Mg-Ionen auf die Ausgangsoberfläche der jeweiligen Stents bezogen.
Bei den Bezeichnungen A1 und A2 handelt es sich um die Messwerte der zwei Stents aus Gruppe A. Analog gilt dieses für die Gruppen B und C.
Sowohl die Stents der Gruppe B als auch die Stents der Gruppe C degradieren deutlich langsamer als die der Gruppe A. Die Stents mit einer Zwischenschicht aus Magnesiumfluorid (Gruppe C) degradieren am langsamsten.

Fazit

In diesem Versuch konnte gezeigt werden, dass Stents der Magnesiumlegierung A mit einer Zwischenschicht aus Magnesiumfluorid in Verbindung mit einer organischen Beschichtung (in diesem Fall PLLA) mit eingebrachtem Medikament langsamer degradieren als Stents ohne eine solche Zwischenschicht. Zudem degradieren Stents der Magnesiumlegierung A deutlich langsamer als Stents der Legierung L37 mit vergleichbarer Beschichtung ohne Zwischenschicht. Der Versuch zeigt, dass die Kombination aus Magnesiumfluorid als Zwischenschicht und PLLA als darüberliegende organische Schicht geeignet ist, die Degradation deutlich zu verlangsamen. Die Messwerte innerhalb der Gruppe C streuen in diesem Vergleich am geringsten, was auf eine sehr homogene Schicht (Doppelschicht) hindeutet.

## Patentansprüche

1. Stent aus einer biologisch abbaubaren Magnesiumlegierung mit einer anorganischen Beschichtung umfassend Magnesiumfluorid und mit einer organischen Beschichtung, wobei die Magnesiumlegierung mindestens 80 Gew.-% Magnesium enthält und wobei die anorganische Beschichtung den Stent bedeckt und die organische Beschichtung die anorganische Beschichtung bedeckt, wobei die organische Beschichtung eine oder mehrere Substanzen der folgenden Gruppe umfasst:
Polyvinylpyrrolidon, Glycerin, Polyhydroxyethyl-methacrylate, Polypropylenglycol, Polyvinylalkohol Polygluconat, Polyaminosäuren, Polyphosphatester, Polyvalerolactone, Poly-ε-Decalactone, Polyglycolsäure, Poly-L-Lactid, Poly D,L-Lactid, sowie Blends wie Poly(L-Lactid-co-glycolid), Poly(D,L-lactid-co-glycolid), Poly(L-Lactid-co-D,L-Lactid), Poly-ε-caprolacton, Polyhydroxybuttersäure, Polyhydroxyvalerate, Polyhydroxybutyrate-covalerate, Polymaleinsäureanhydride, Polyhydroxymethacrylate, Fibrin, Polycyanoacrylate, Polycaprolacton-dimethylacrylate, Polycaprolactonbutylacrylate, Polyetherestermulti-blockpolymere aus PEG und Polybutylenterephtalat, Polypivotolactone, Polyglycolsäuretrimethylcarbonate Polycaprolactonglycolide, Poly(DTH-Iminocarbonat), Poly(DTE-co-DT-carbonat), Poly(Bisphenol A-iminocarbonat), Polyorthoester, Polytrimethylcarbonate Polyiminocarbonate, Poly(N-vinyl)-Pyrrolidon, Polyesteramide, glycolierte Polyester, Polyphosphoester, Polyphosphazene, Poly[p-carboxyphenoxy)propan], Polyethylenoxidpropylenoxid, Polyalkenoxalate, Lipide, Wachse, Öle, mehrfach ungesättigte Fettsäuren, Eicosapentaensäure, Timnodonsäure, Docosahexaensäure, Arachidonsäure, Linolsäure, α-Linolensäure, γ-Linolensäure, Carrageenane, Fibrinogen, Agar-Agar, Stärke, Zein, Polyhydroxyalkanoate, Pectinsäure, Actinsäure, Carboxymethylsulfat, Hyaluronsäure, Heparansulfate und seine Derivate, Heparine, Dextran, β-Cyclodextrine, Gummi arabicum, Guar, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polyvinylketone, Polyvinylhalogenide, Polyvinyliden-halogenide, Polyvinylether, Polyisobutylene, Polyvinylaromaten, Polyvinylester, Polyoxymethylene, Polytetramethylenoxid, Polyethylen, Polypropylen, Polytetrafluorethylen, Polyolefin-Elastomere, Carboxymethylchitosane, Polyetheretherketone, Polyethylenterephtalat, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Polysulfone, Epoxyharze, ABS-Harze, EPDM-Gummis, Celluloseether, Cellulosetriacetate, Schellack, Poly-para-Xylylene (Parylene) wie Parylen N, Parylen C.

2. Stent gemäß Anspruch 1, wobei die organische Beschichtung eine oder mehrere Substanzen der folgenden Gruppe umfasst: Poly-ε-Caprolacton, Poly-L-Lactid-co-Glycolid, Poly-L-Lactid und Parylene.

3. Stent gemäß einem der Ansprüche 1 oder 2, wobei die Schichtdicke der anorganischen Beschichtung 0,01 μm bis 100 μm beträgt.

4. Stent gemäß einem der Ansprüche 1 - 3, wobei die Schichtdicke der organischen Beschichtung 0,01 μm bis 100 μm beträgt.

5. Stent gemäß einem der Ansprüche 1 - 4, wobei das Verhältnis der Schichtdicke der anorganischen Beschichtung zur organischen Beschichtung 20:1 bis 1:100.000 beträgt.

6. Stent gemäß einem der Ansprüche 1 - 5, wobei die organische Beschichtung keine Mikroporen, Löcher, Öffnungen oder Kanäle aufweist.

7. Stent gemäß einem der Ansprüche 1 - 6, wobei sich in oder auf der organischen Beschichtung mindestens ein antiinflammatorischer, antiproliferativer, antiangiogener, antirestenotischer, antineoplastische, antimigrativer und/oder antithrombogener Wirkstoff befindet.

8. Stent gemäß Anspruch 7, wobei der mindestens eine antiinflammatorische, antiproliferative, antiangiogene, antirestenotische, antineoplastische, antimigrative und/oder antithrombogene Wirkstoff ausgewählt ist aus der Gruppe bestehend aus Paclitaxel, Sirolimus, Biolimus A9, Myolimus, Novolimus, Pimecrolimus, Tacrolimus, Temsirolimus, Zotarolimus, Everolimus und Ridaforolimus.

9. Stent gemäß einem der Ansprüche 1 - 8, wobei die biologisch abbaubare Magnesiumlegierung

| | |
|---|---|
| 0,1 Gew.-% - 15,5 Gew.-% | Dysprosium |
| 0,01 Gew.-% - 1,5 Gew.-% | Neodym und/oder Europium |
| 0,0 Gew.-% - 2,0 Gew.-% | Zink |
| 0,0 Gew.-% - 1,0 Gew.-% | Zirkonium |
| mindestens 80,0 Gew.-% | Magnesium umfasst. |

10. Stent gemäß einem der Ansprüche 1 - 9, wobei es sich bei dem Stent um einen Stent für Blutgefäße, Harnwege, Atemwege, Gallenwege oder den Verdauungstrakt handelt.

11. Stent gemäß Anspruch 1, wobei die organische Beschichtung ein oder mehrere organische Polymere umfasst.

## Claims

1. A stent of biodegradable magnesium alloy having an inorganic coating comprising magnesium fluoride and having an organic coating, the magnesium alloy containing at least 80% by weight magnesium, and wherein the inorganic coating covers the stent and the organic coating covers the inorganic coating,
wherein the organic coating comprises one or more substances of the following group: polyvinyl pyrrolidone, glycerol, poly hydroxyethyl methacrylates, polyethylene glycol, polypropylene glycol, polyvinyl alcohol, polygluconate, polyamino acids, polyphosphate esters, polyvalerolactones, poly-ε-decalactones, poly(glycolic acid), poly(L-lactide), poly(D,L-lactide), and blends such as poly(L-lactide-co-glycolide), poly(D,L-lactide-co-glycolide), poly(L-lactide-co-D,L-lactide), poly(ε-caprolactone), polyhydroxybutyric acid, polyhydroxyvalerates, polyhydroxybutyrate-co-valerates, poly(maleic anhydrides), polyhydroxy methacrylates, fibrin, polycyanoacrylates, polycaprolactone dimethylacrylates, polycaprolactone butyl acrylates, poly(ether ester) multiblock polymers of PEG and poly(butylene terephthalate), polypivalolactones, polyglycolic acid-trimethylene carbonates, polycaprolactone glycolides, poly(DTH iminocarbonate), poly(DTE-co-DT-carbonate), poly(bisphenol A iminocarbonate), poly(ortho ester), poly(trimethylene carbonate) poly(imino carbonate), poly(N-vinylpyrrolidone), polyesteramides, glycolized polyesters, polyphosphoesters, polyphosphazenes, poly[(p-carboxyphenoxy) propane], poly(ethylene oxide)-poly(propylene oxide), polyalkene oxalates, lipids, waxes, oils, polyunsaturated fatty acids, eicosapentaenoic acid, timnodonic acid, docosahexaenoic acid, arachidonic acid, linoleic acid, α-linolenic acid, γ-linolenic acid, carrageenans, fibrinogen, agar-agar, starch, Zein, polyhydroxyalkanoates, pectic acid, actinic acid, carboxymethyl sulfate, hyaluronic acid, heparan sulfate and derivatives thereof, heparins, dextran, ß-cyclodextrins, gum arabic, guar, phospholipids, polyacrylic acid, polyacrylates, poly(methyl methacrylate), poly(butyl methacrylate), polyacrylamide, polyacrylonitrile, polyamides, polyetheramides, polyethylene amine, polyimides, polycarbonates, polyvinyl ketones, polyvinyl halides, polyvinylidene halides, polyvinyl ethers, polyisobutylenes, polyvinyl aromatic compounds, polyvinyl esters, polyoxymethylenes, polytetramethylene oxide, polyethylene, polypropylene, polytetrafluoroethylene, polyolefin elastomers, carboxymethylchitosans, polyetheretherketones, polyethylene terephthalate, carboxymethylcellulose, cellulose, rayon, rayon triacetates, cellulose nitrates, cellulose acetates, hydroxyethyl cellulose, cellulose butyrates, cellulose acetate butyrates, polysulfones, epoxy resins, ABS resins, EPDM rubbers, cellulose ethers, cellulose triacetates, shellac, poly-para-xylylenes (parylenes) such as parylene N, parylene C.

2. The stent according to claim 1, wherein the organic coating comprises one or more substances of the following group: poly(ε-caprolactone), poly(L-lactide-co-glycolide), poly(L-lactide), and parylenes.

3. The stent according to claim 1 or 2, wherein the layer thickness of the inorganic coating is 0.01 μm to 100 μm.

4. The stent according to any one of claims 1 - 3, wherein the layer thickness of the organic coating is 0.01 μm to 100 μm.

5. The stent according to any one of claims 1 - 4, wherein the ratio of the layer thickness of the inorganic coating to the organic coating is from 20:1 to 1:100,000.

6. The stent according to any one of claims 1 - 5, wherein the organic coating has no micropores, holes, openings or channels.

7. The stent according to any one of claims 1 - 6, wherein at least one antiinflammatory, antiproliferative, antiangiogenic, antirestenotic, antineoplastic, antimigrative and/or antithrombogenic active agent is present in or on the organic coating.

8. The stent according to claim 7, wherein the at least one anti-inflammatory, anti proliferative, antiangiogenic, antirestenotic, antineoplastic, antimigrative and/or antithrombogenic active agent is selected from the group consisting of paclitaxel, sirolimus, biolimus A9, myolimus, novolimus, pimecrolimus, tacrolimus, temsirolimus, zotarolimus, everolimus, and ridaforolimus.

9. The stent according to any one of claims 1 - 8, wherein the biodegradable magnesium alloy comprises

| | |
|---|---|
| 0.1% by wt. - 15.5% by wt. | dysprosium |
| 0.01% by wt. - 1.5% by wt. | neodymium and/or europium |
| 0.0% by wt. - 2.0% by wt. | zinc |
| 0.0% by wt. - 1.0% by wt. | zirconium |
| at least 80.0% by wt. | magnesium. |

10. The stent according to any one of claims 1 - 9, wherein the stent is a stent for blood vessels, urinary tracts, respiratory tracts, biliary tracts or digestive tract.

11. The stent according to claims 1, wherein the organic coating comprises one or more organic polymers.

## Revendications

1. Un stent en alliage de magnésium biodégradable présentant un revêtement inorganique comprenant du fluorure de magnésium et un revêtement organique, l'alliage de magnésium étant constitué au moins à 80 % en poids de magnésium, où le revêtement inorganique recouvre le stent et le revêtement organique recouvre le revêtement inorganique et
où le revêtement organique comprend une ou plusieurs substances du groupe suivant : polyvinylpyrrolidone, glycérol, méthacrylates de polyhydroxyéthyle, polyéthylène glycol, polypropylène glycol, alcool polyvinylique, polygluconate, polyaminoacides, esters de polyphosphate, polyvalérolactones, poly-ε-décalactones, poly(acide glycolique), poly(L-lactide), poly(D,L-lactide) et des mélanges tels que le poly(L-lactide-co-glycolide), poly(D,L-lactide-co-glycolide), poly(L-lactide-co-D,L-lactide), poly(ε-caprolactone), acide polyhydroxybutyrique, polyhydroxyvalérate, polyhydroxybutyrate-co-valérate, poly(anhydride maléique), polyhydroxy méthacrylate, fibrine, polycyanoacrylate, polycaprolactone diméthylacrylate, polycaprolactone butyle acrylate, polymères multiblocs poly(éther ester) de PEG et poly(butylène téréphtalate), polypivalolactones, carbonates d'acide polyglycolique-triméthylène, polycaprolactone glycolide, poly(DTH iminocarbonate), poly(DTE-co-DT-carbonate), poly(bisphénol A iminocarbonate), poly(orthoester), poly(triméthylène carbonate) poly(imino carbonate), poly(N-vinylpyrrolidone), polyesteramides, polyesters glycolisés, polyphosphoesters, polyphosphazènes, poly[(p-carboxyphénoxy) propane], poly(oxyde d'éthylène)-poly(oxyde de propylène), oxalates de polyalcène, lipides, cires, huiles, acides gras polyinsaturés, acide eicosapentaénoïque, acide timnodonique, acide docosahexaénoïque, acide arachidonique, acide linoléique, acide α-linolénique, acide γ-linolénique, carraghénanes, fibrinogène, agar-agar, amidon, zéine, polyhydroxyalcanoates, acide pectique, acide actinique, carboxyméthylsulfate, acide hyaluronique, sulfate d'héparane et ses dérivés, héparines, dextrane, β-cyclodextrines, gomme arabique, guar, phospholipides, acide polyacrylique, polyacrylates, poly(méthacrylate de méthyle), poly(méthacrylate de butyle), polyacrylamide, polyacrylonitrile, polyamides, polyétheramides, polyéthylèneamine, polyimides, polycarbonates, polyvinylcétones, polyvinylhalogénures, polyvinylidènehalogénures, éthers polyvinyliques, polyisobutylènes, composés polyvinyliques aromatiques, esters polyvinyliques, polyoxyméthylènes, oxyde de polytétraméthylène, polyéthylène, polypropylène, polytétrafluoroéthylène, élastomères polyoléfiniques, carboxyméthylchitosanes, polyétheréthercétones, polyéthylène téréphtalate, carboxyméthylcellulose, cellulose, rayonne, triacétates de rayonne, nitrates de cellulose, acétates de cellulose, hydroxyéthylcellulose, butyrates de cellulose, butyrates d'acétate de cellulose, polysulfones, résines époxy, résines ABS, caoutchoucs EPDM, éthers de cellulose, triacétates de cellulose, gomme laque, poly-para-xylylènes (parylènes) tels que le parylène N ou le parylène C.

**2.** Le stent conformé à la revendication 1, où le revêtement organique comprend au moins une substance du groupe suivant : poly(ε-caprolactone), poly(L-lactide-co-glycolide), poly(L-lactide) et parylènes.

**3.** Le stent conforme à la revendication 1 ou 2, où l'épaisseur de couche du revêtement inorganique est comprise entre 0,01 μm et 100 μm.

**4.** Le stent conforme à l'une des revendications 1 à 3, où l'épaisseur du revêtement organique est comprise entre 0,01 μm et 100 μm.

**5.** Le stent conforme à l'une des revendications 1 à 4, où le rapport des épaisseurs du revêtement inorganique et du revêtement organique est compris entre 20:1 et 1:100 000.

**6.** Le stent conforme à l'une des revendications 1 à 5, où le revêtement organique ne présente aucun micropore, aucun trou, aucune ouverture, ni aucun canal.

**7.** Le stent conforme à l'une des revendications 1 à 6, où au moins un agent actif anti-inflammatoire, antiprolifératif, anti-angiogénique, antisténotique, antinéoplastique, antimigratif et/ou antithrombogénique est présent dans ou sur le revêtement organique.

**8.** Le stent conforme à la revendication 7, où au moins un agent actif anti-inflammatoire, antiprolifératif, anti-angiogénique, antisténotique, antinéoplastique, antimigratif et/ou antithrombogénique est sélectionné parmi le groupe constitué de : paclitaxel, sirolimus, biolimus A9, myolimus, novolimus, pimecrolimus, tacrolimus, temsirolimus, zotarolimus, everolimus, et ridaforolimus.

**9.** Le stent conforme à l'une des revendications 1 à 8, où l'alliage de magnésium biodégradable comprend

| | |
|---|---|
| 0,1 % du poids | - 15,5 % du poids dysprosium |
| 0,01 % du poids | - 1,5 % du poids néodymium et/ou europium |
| 0,0 % du poids | - 2,0 % du poids zinc |
| 0,0 % du poids | - 1,0 % du poidszirconium |
| au moins 80,0 % du poids | magnésium. |

**10.** Le stent conforme à l'une des revendications 1 à 9, où ledit stent est conçu pour les vaisseaux sanguins, les voies urinaires, les voies respiratoires, les voies biliaires ou les voies digestives.

**11.** Le stent conforme à la revendication 1, où le revêtement organique comprend un ou plusieurs polymères organiques.

Figur 1

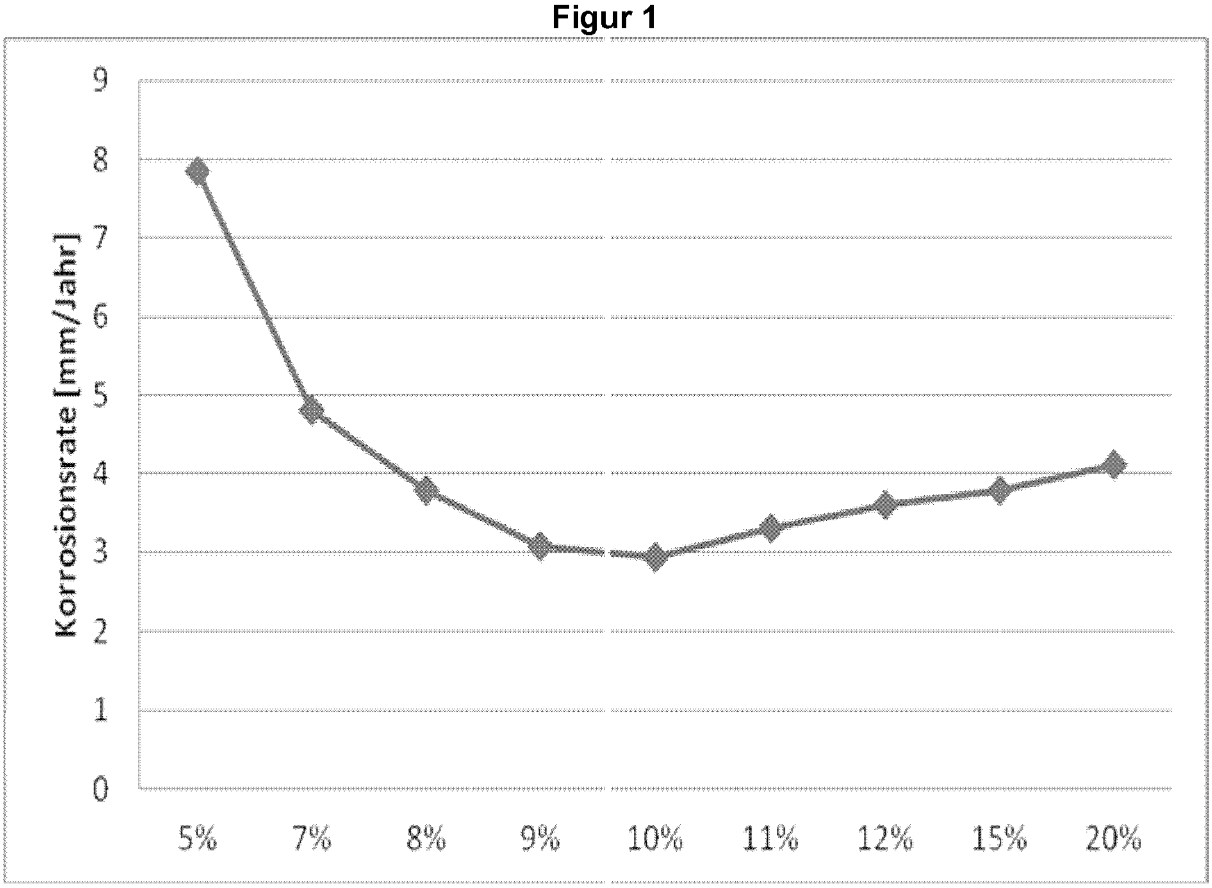


Figur 2

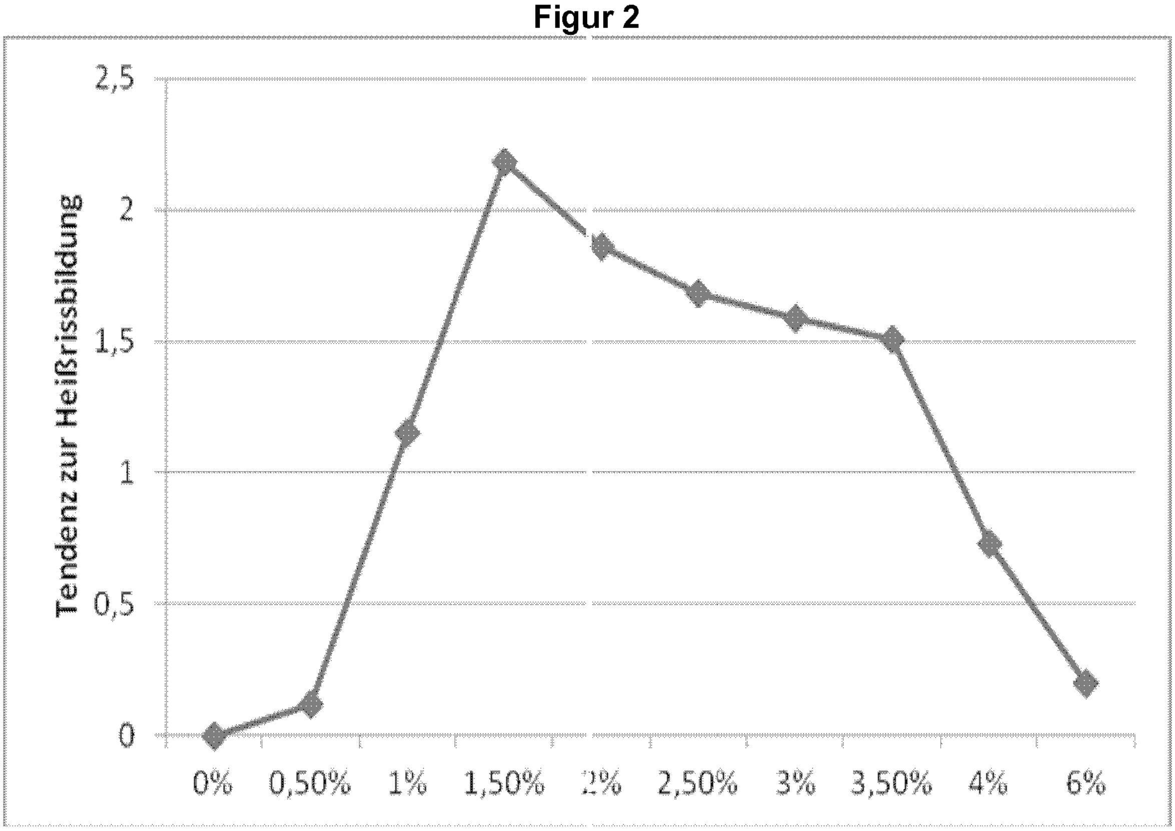

Figur 3

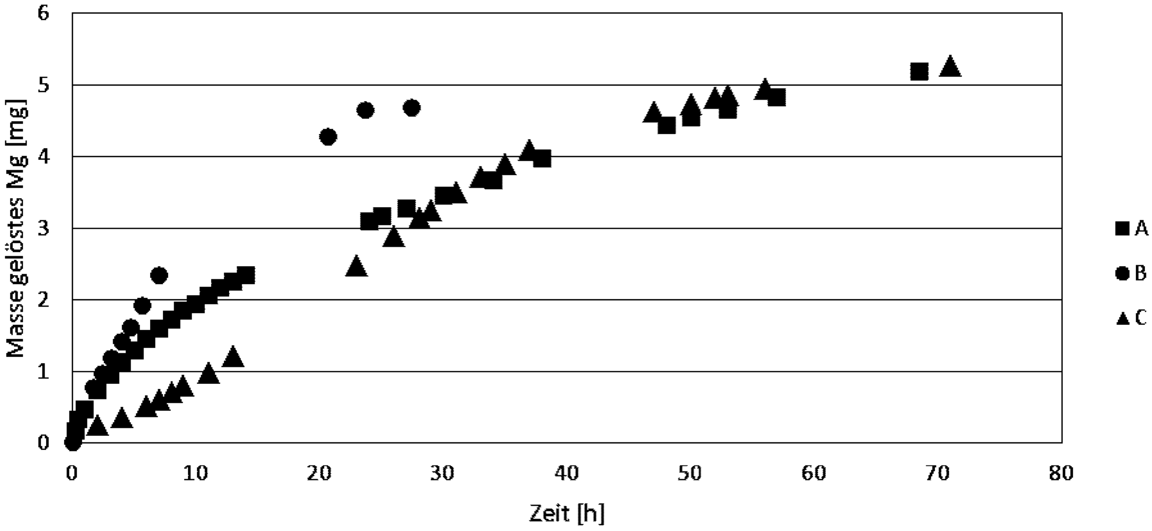


Figur 4

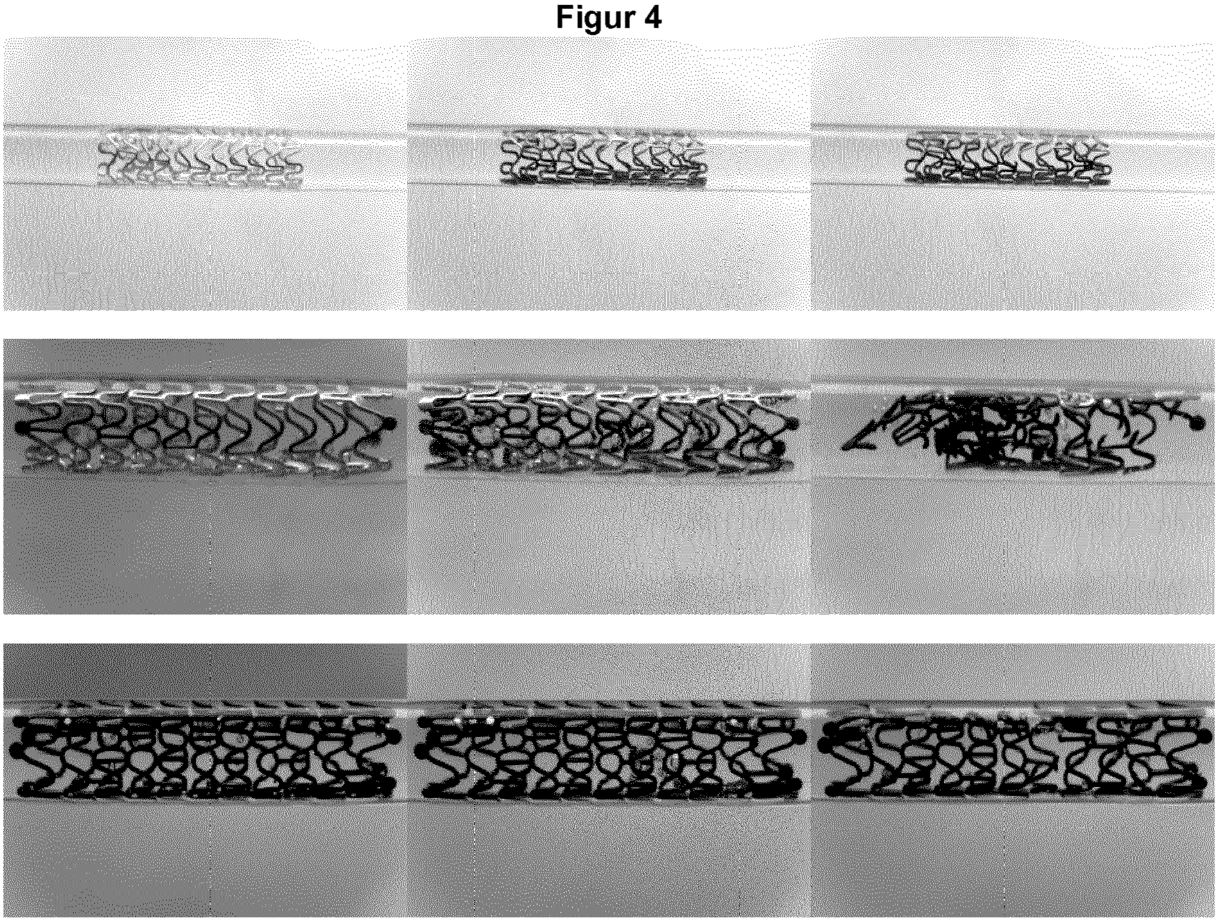

Figur 5

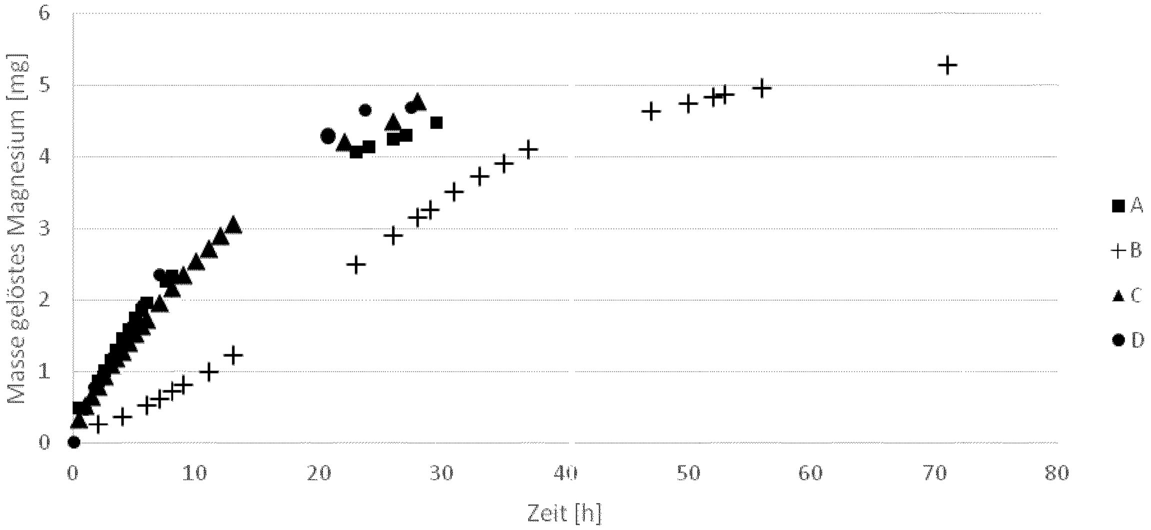


Figur 6

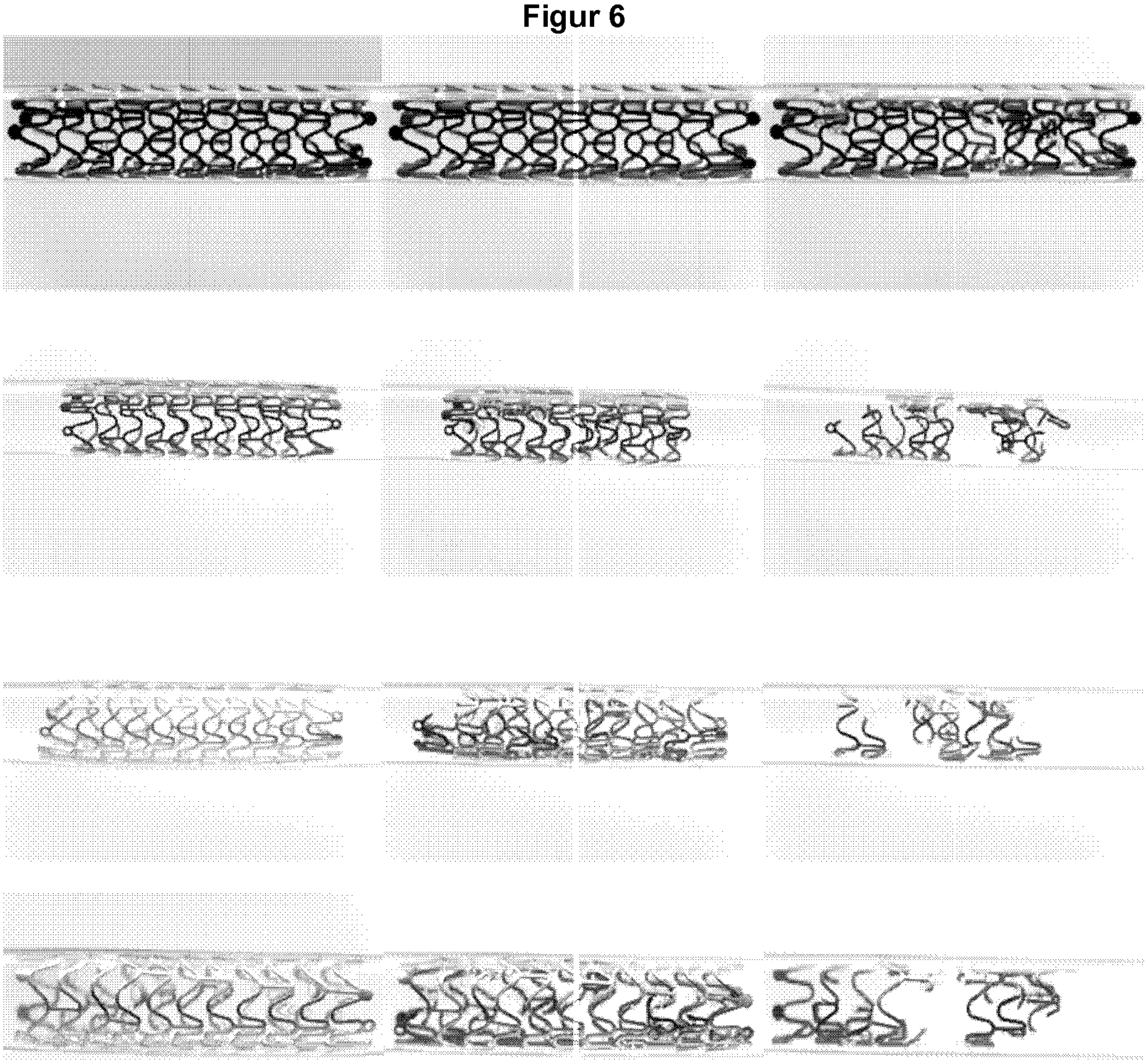

Figur 7

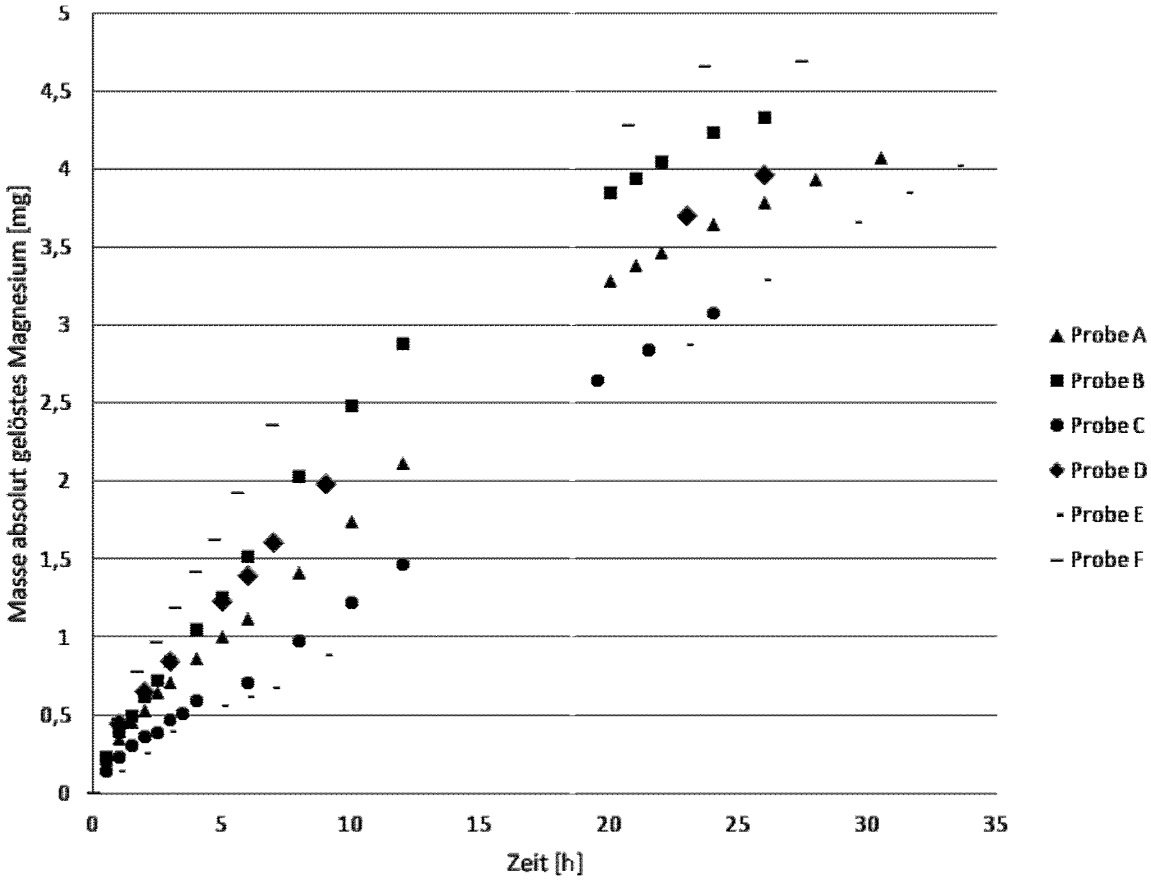


Figur 8

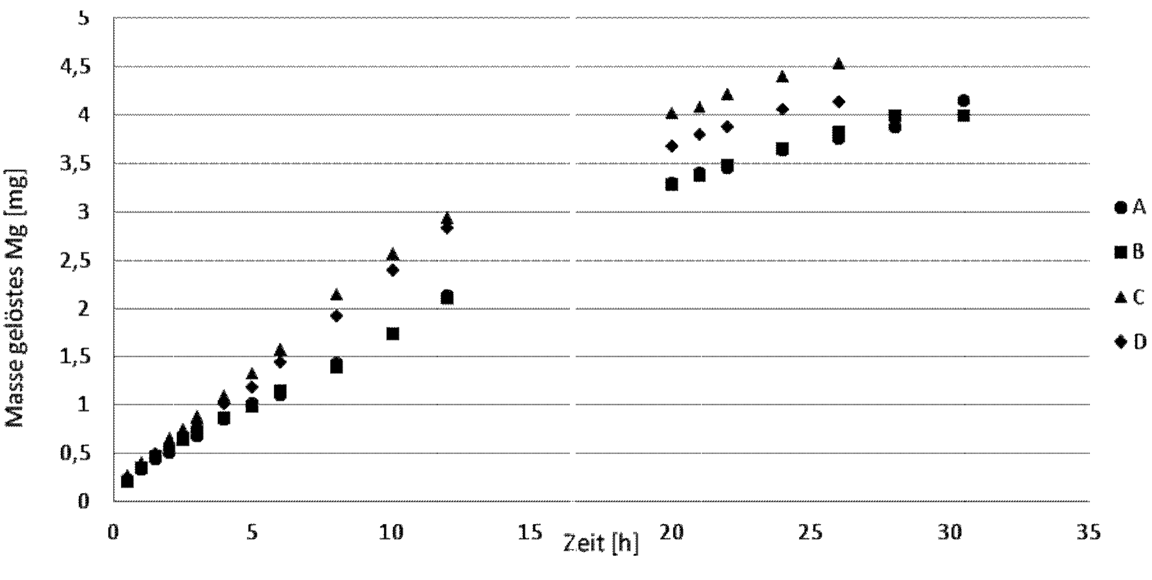

## Figur 9

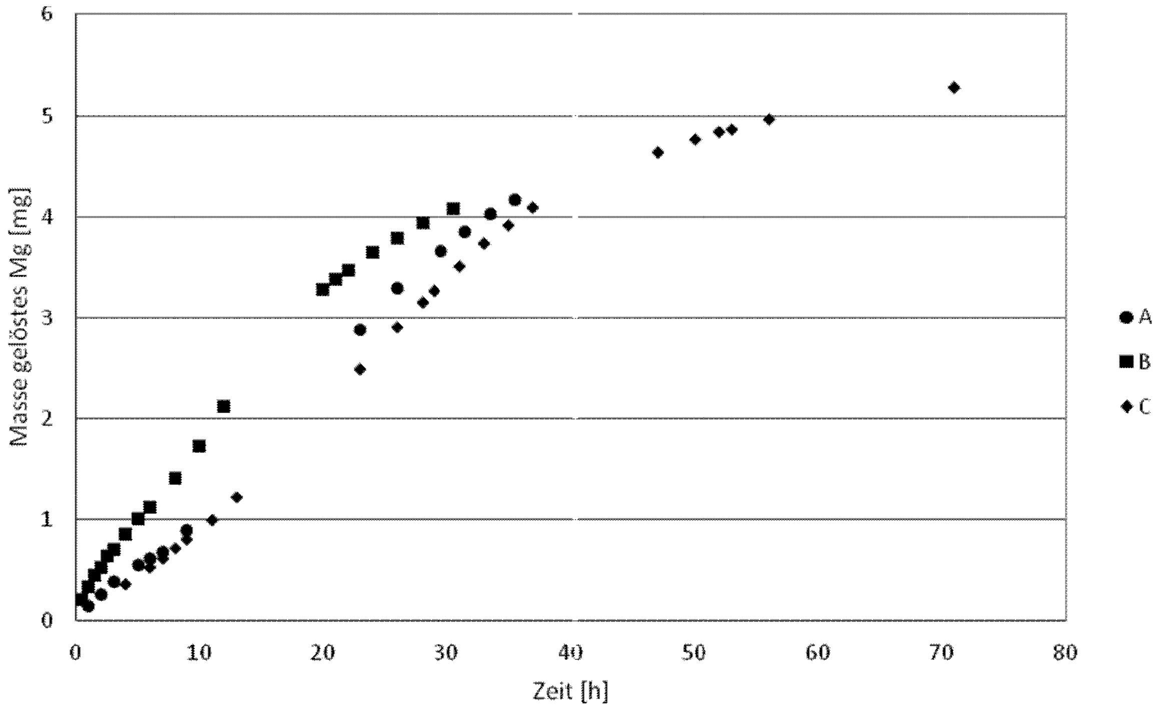


## Figur 10

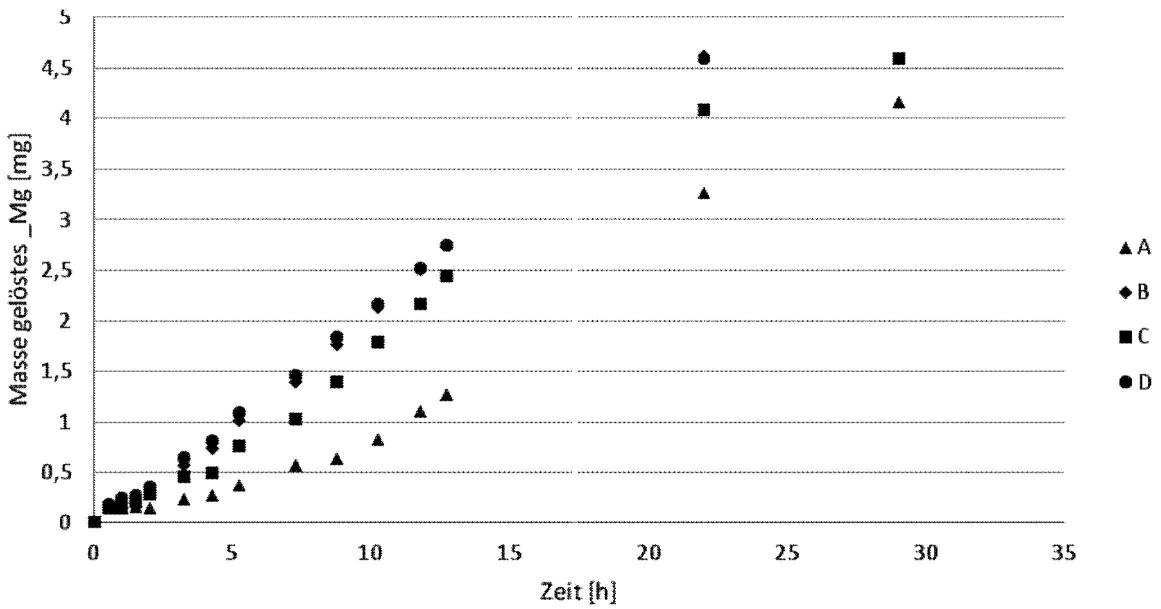

## Figur 11

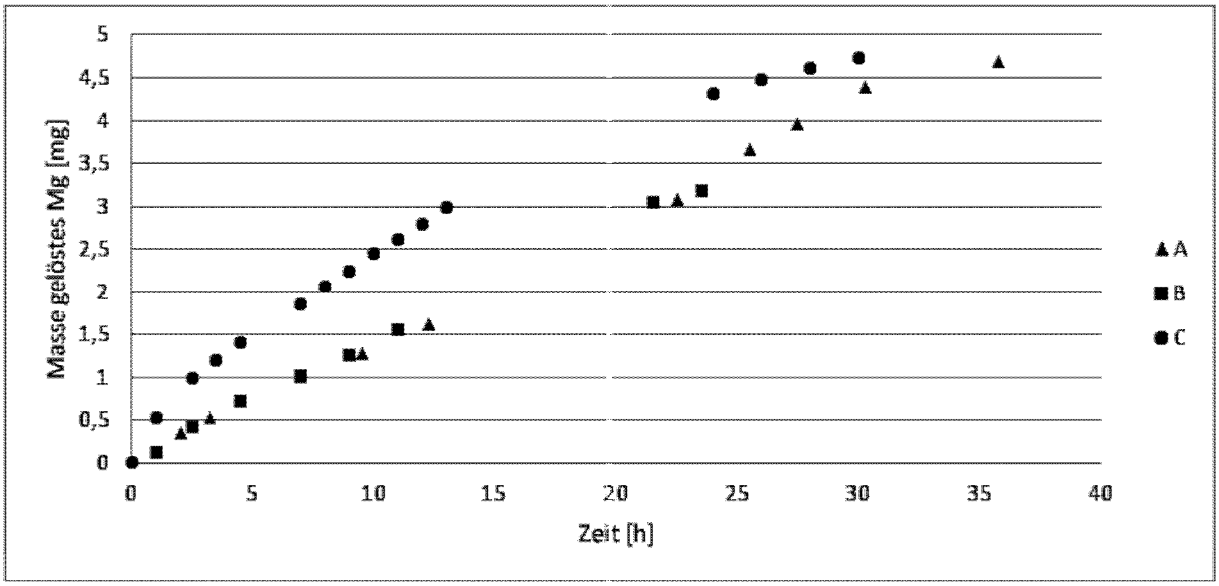


## Figur 12

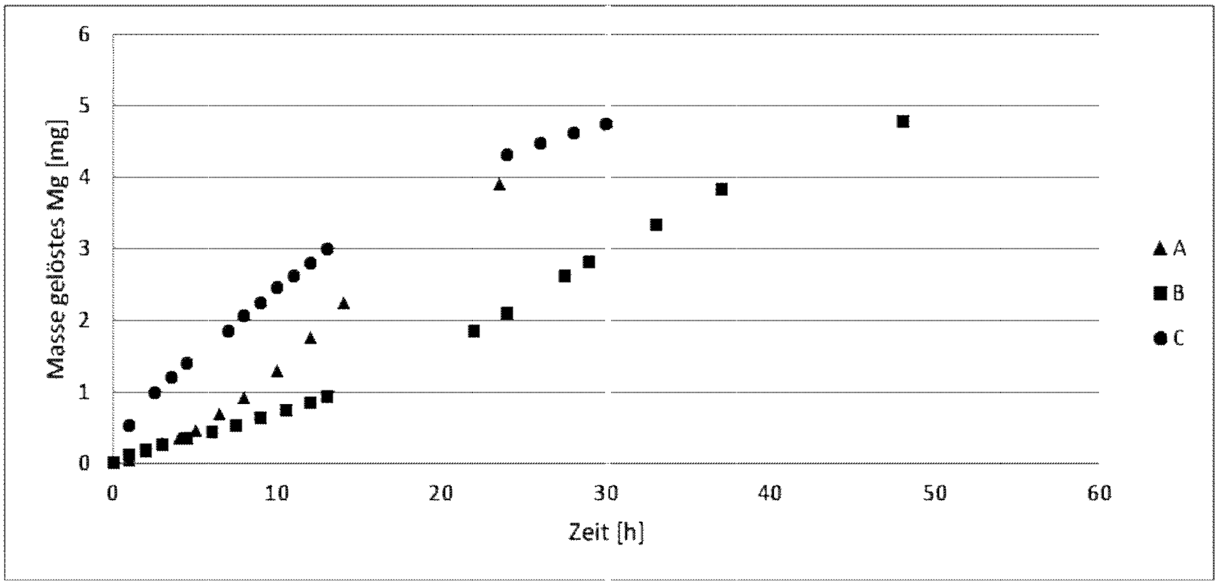

Figur 13:

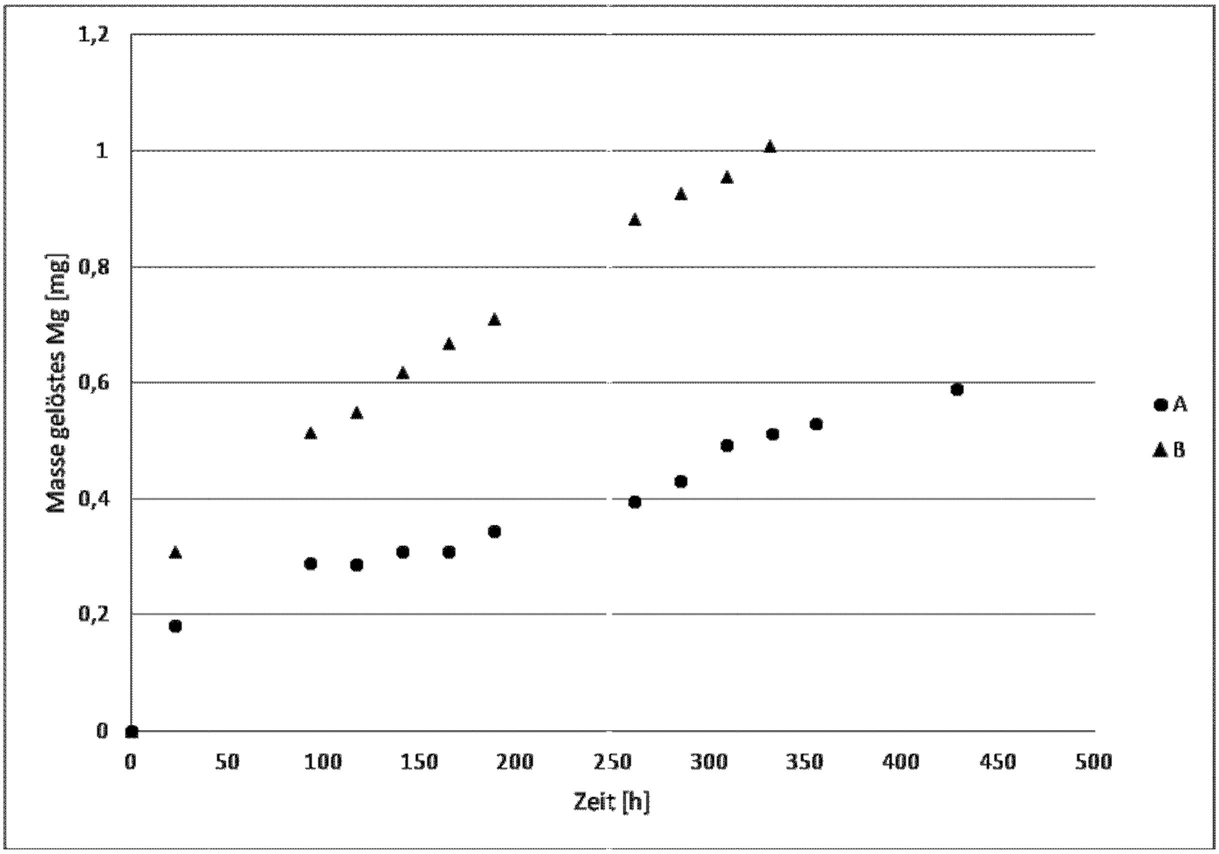


Figur 14

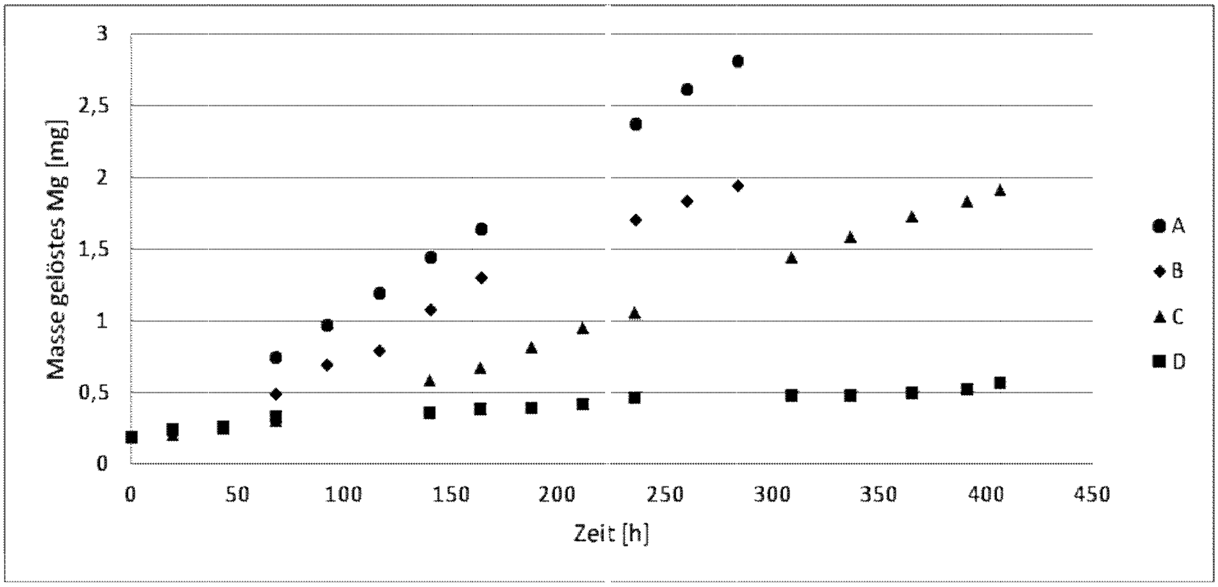

Figur 15

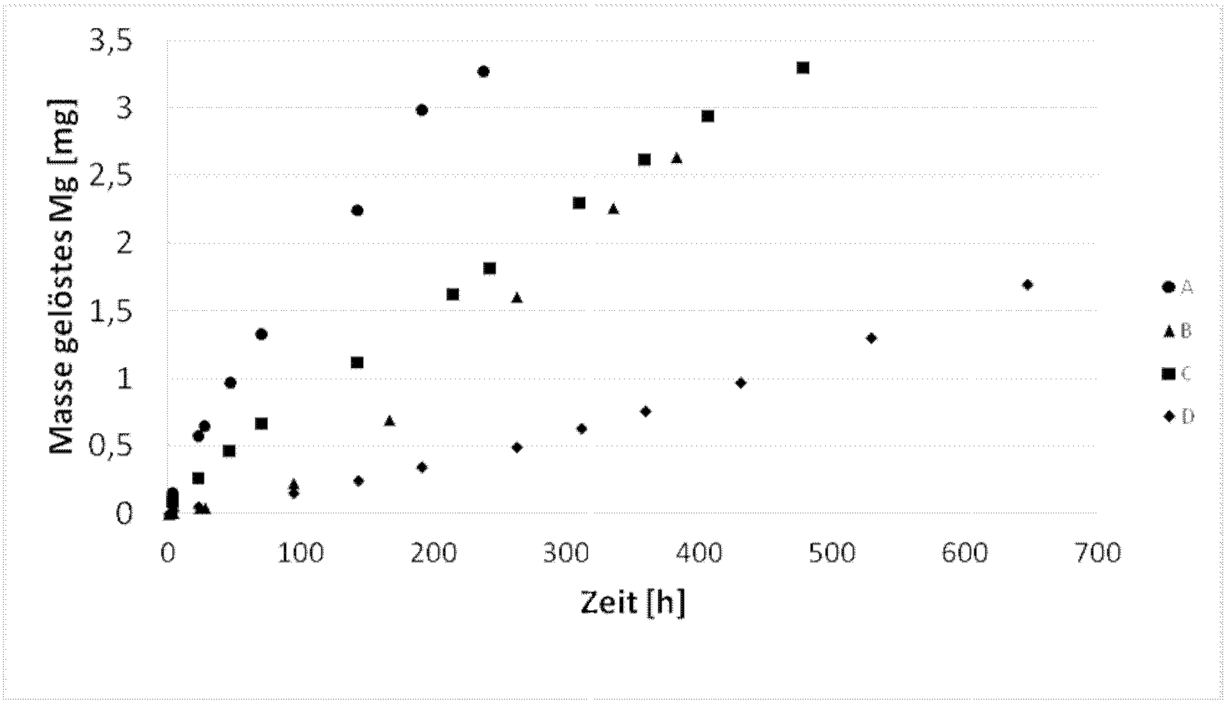
3,5
3
2,5
2
1,5
1
0,5
0
Masse gelöstes Mg [mg]
0
100
200
300
400
500
600
700
Zeit [h]
A
B
C
D

Figur 16

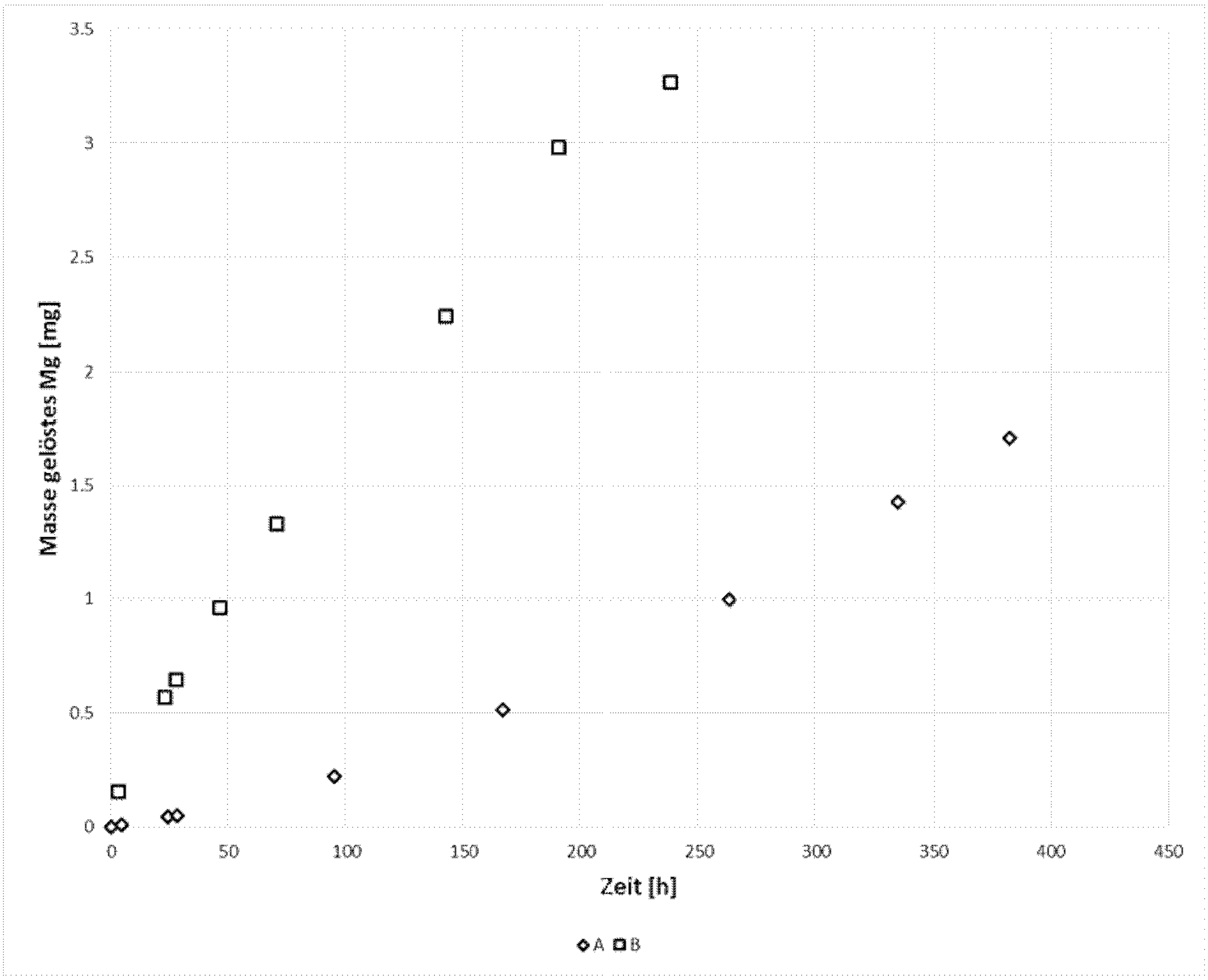
Masse gelöstes Mg [mg]
3.5
3
2.5
2
1.5
1
0.5
0
0
50
100
150
200
250
300
350
400
450
Zeit [h]
A
B

Figur 17

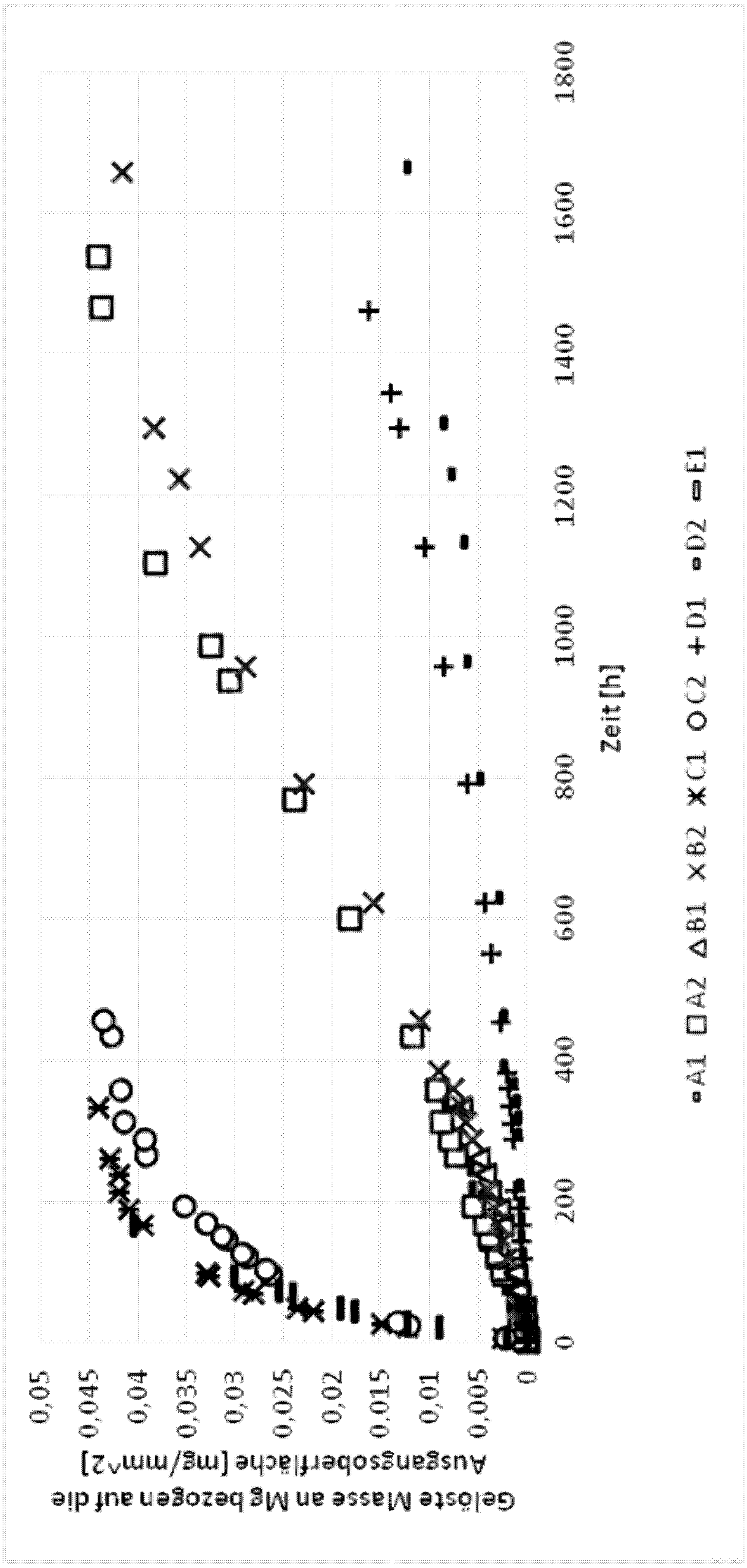
Gelöste Masse an Mg bezogen auf die Ausgangsoberfläche [mg/mm^2]
0
0,005
0,01
0,015
0,02
0,025
0,03
0,035
0,04
0,045
0,05
Zeit [h]
0
200
400
600
800
1000
1200
1400
1600
1800
A1
A2
B1
B2
C1
C2
D1
D2
E1

Figur 18

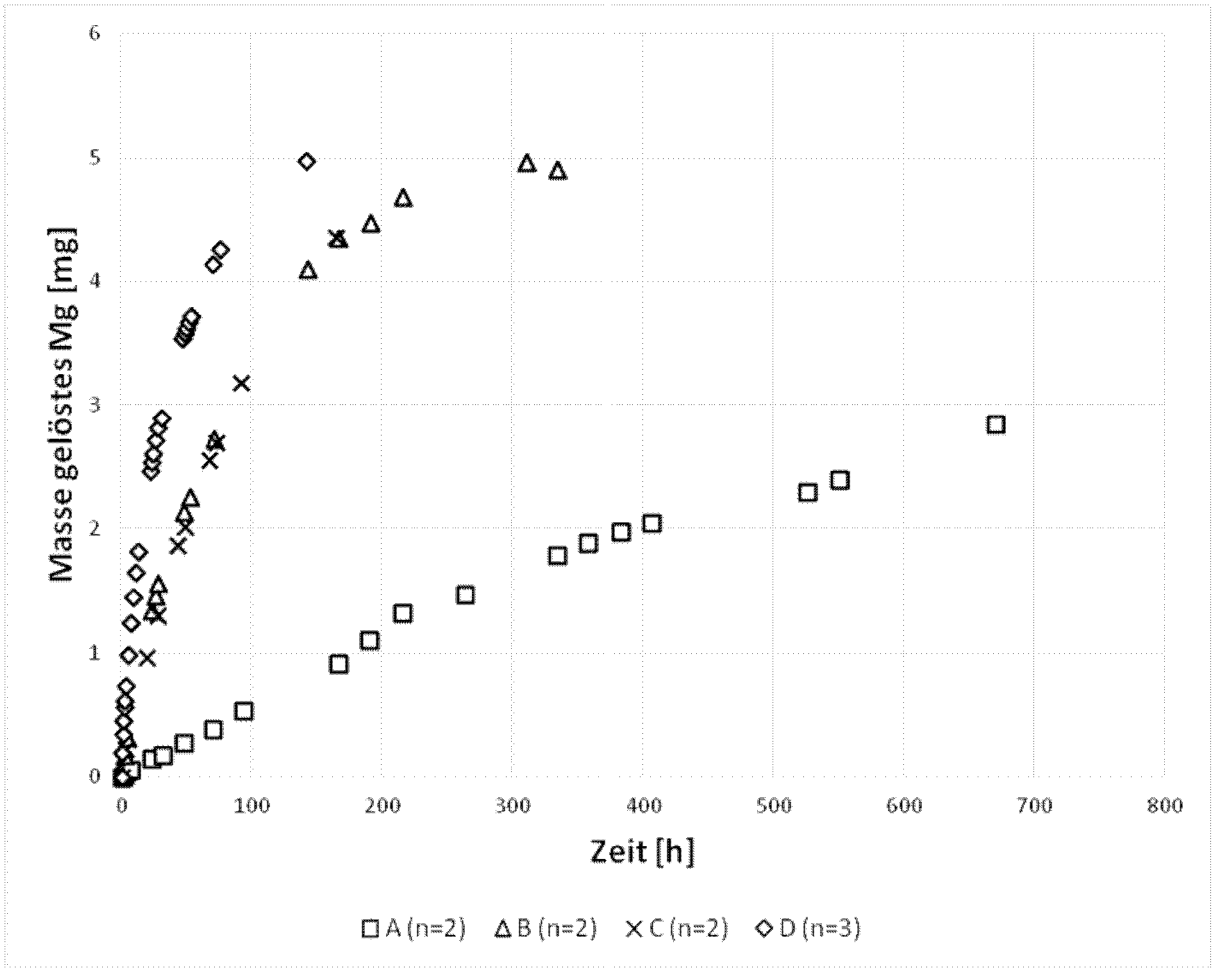
Masse gelöstes Mg [mg]
0
1
2
3
4
5
6
0
100
200
300
400
500
600
700
800
Zeit [h]
A (n=2)
B (n=2)
C (n=2)
D (n=3)

**Figur 19**

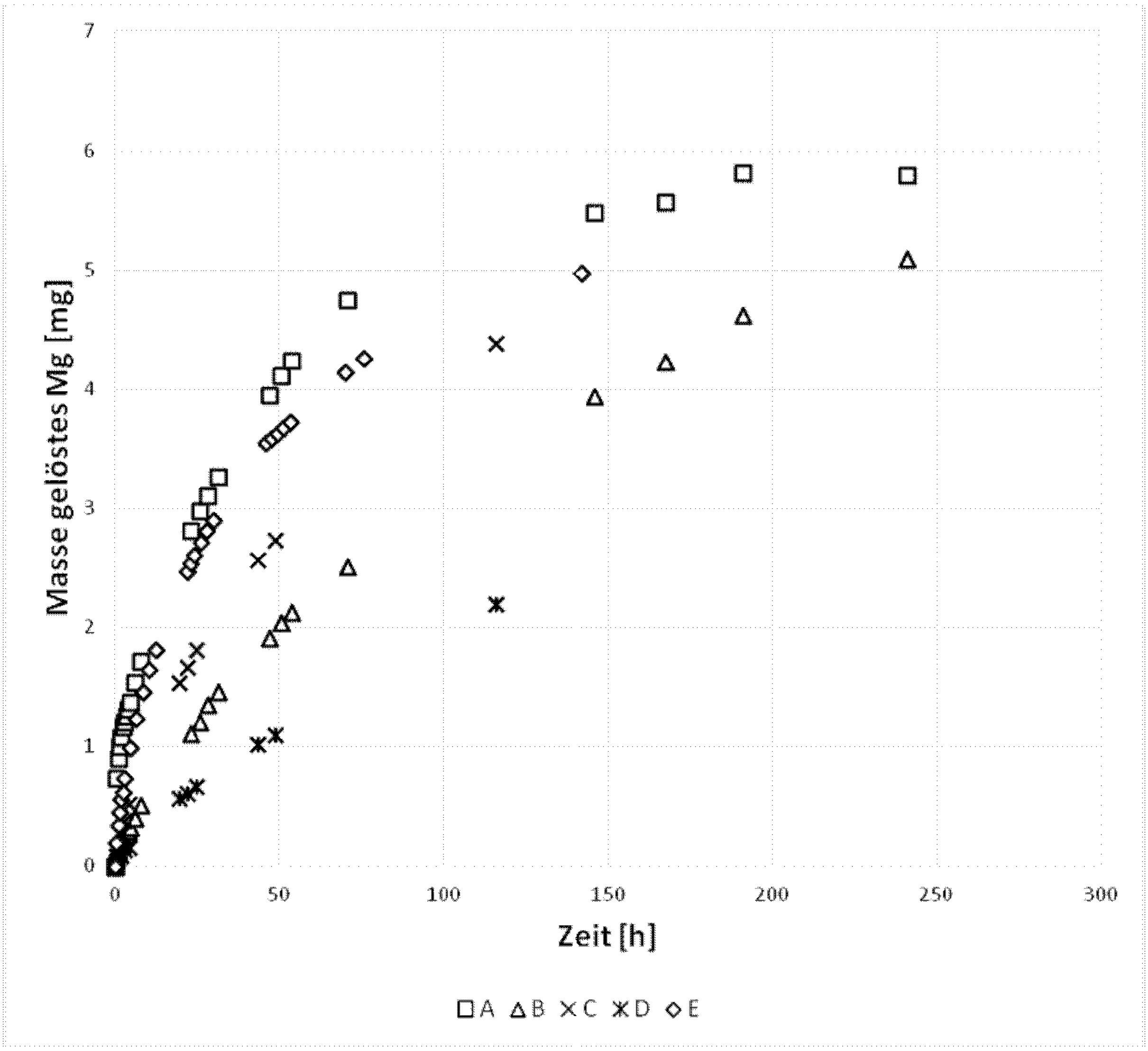
Masse gelöstes Mg [mg]
Zeit [h]
0
1
2
3
4
5
6
7
0
50
100
150
200
250
300
A
B
C
D
E

Figur 20

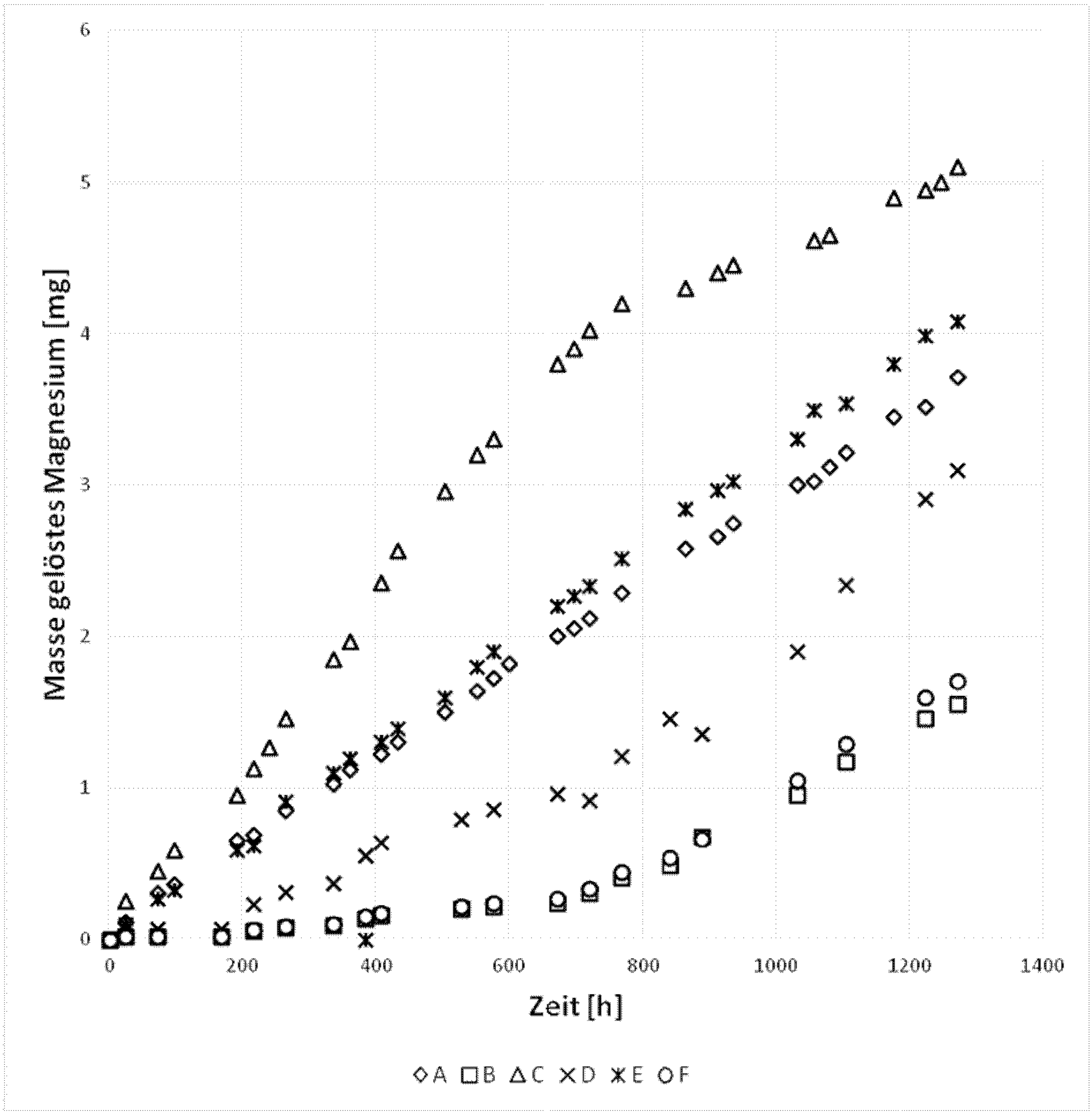
6
5
4
3
2
1
0
Masse gelöstes Magnesium [mg]
0
200
400
600
800
1000
1200
1400
Zeit [h]
◇A □B △C ×D ✱E ○F

Figur 21

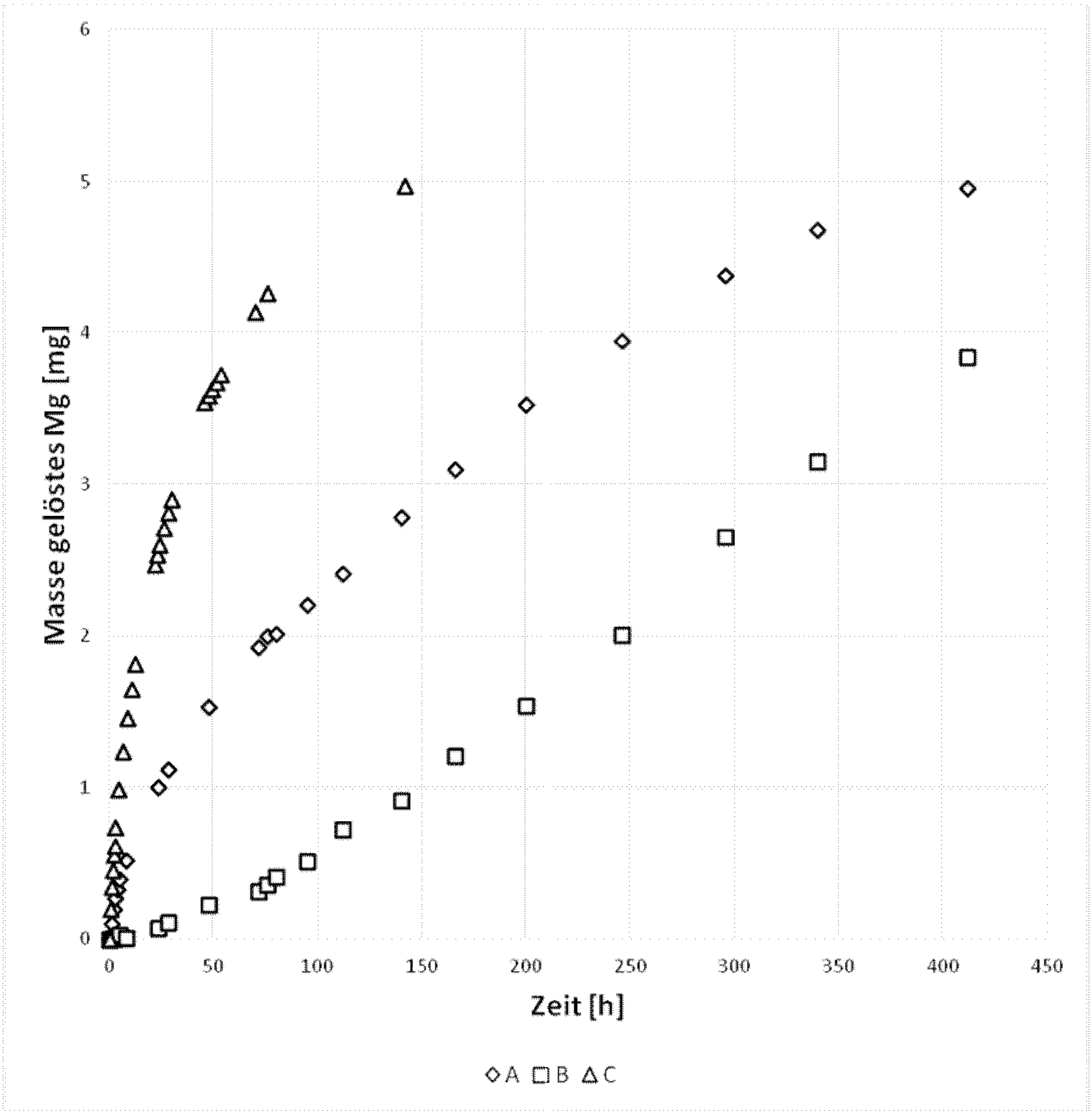
Masse gelöstes Mg [mg]
Zeit [h]
0
1
2
3
4
5
6
0
50
100
150
200
250
300
350
400
450
◇ A □ B △ C

## Figur 22

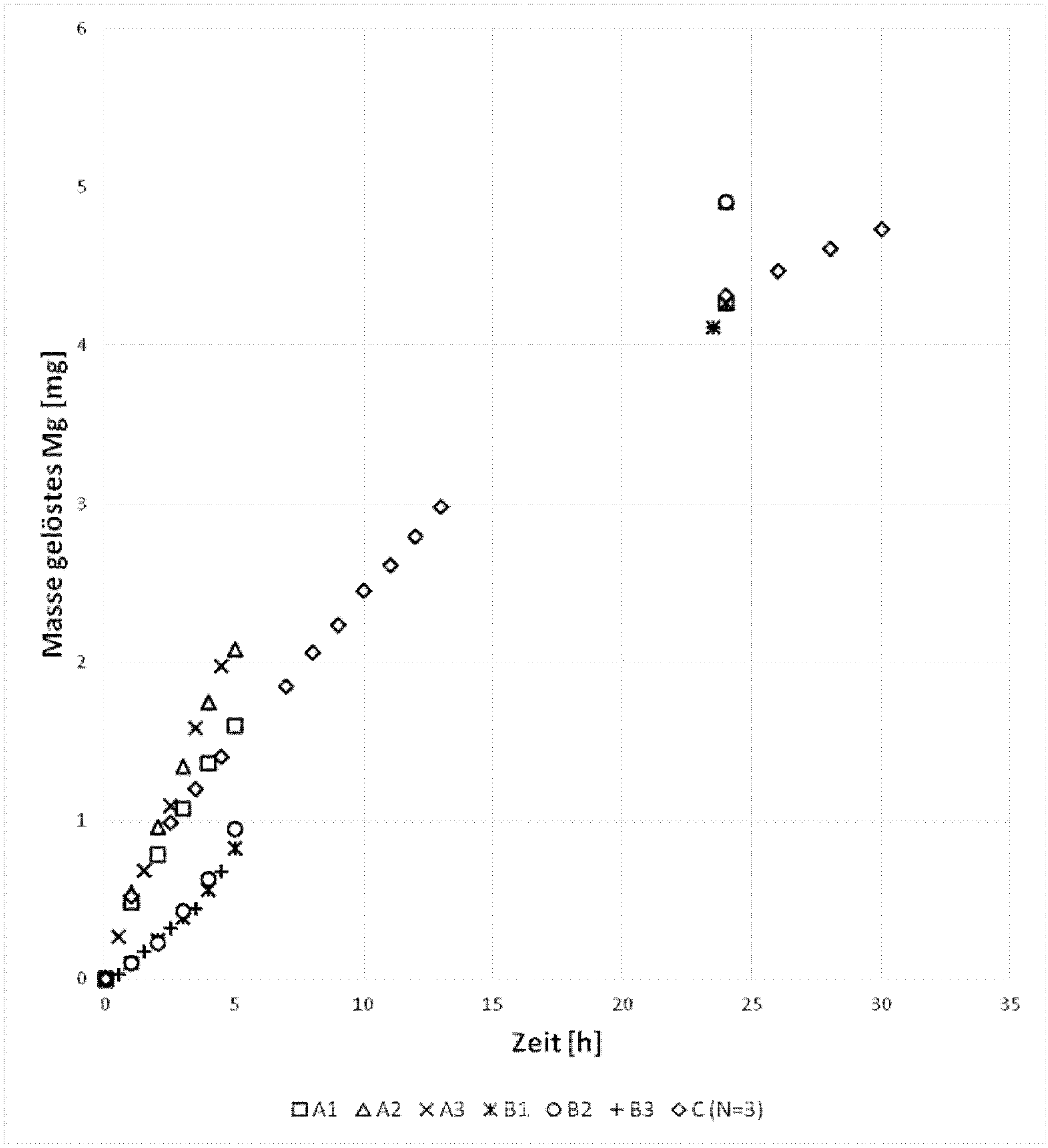
Masse gelöstes Mg [mg]
Zeit [h]
0
1
2
3
4
5
6
0
5
10
15
20
25
30
35
A1
A2
A3
B1
B2
B3
C (N=3)

Figur 23

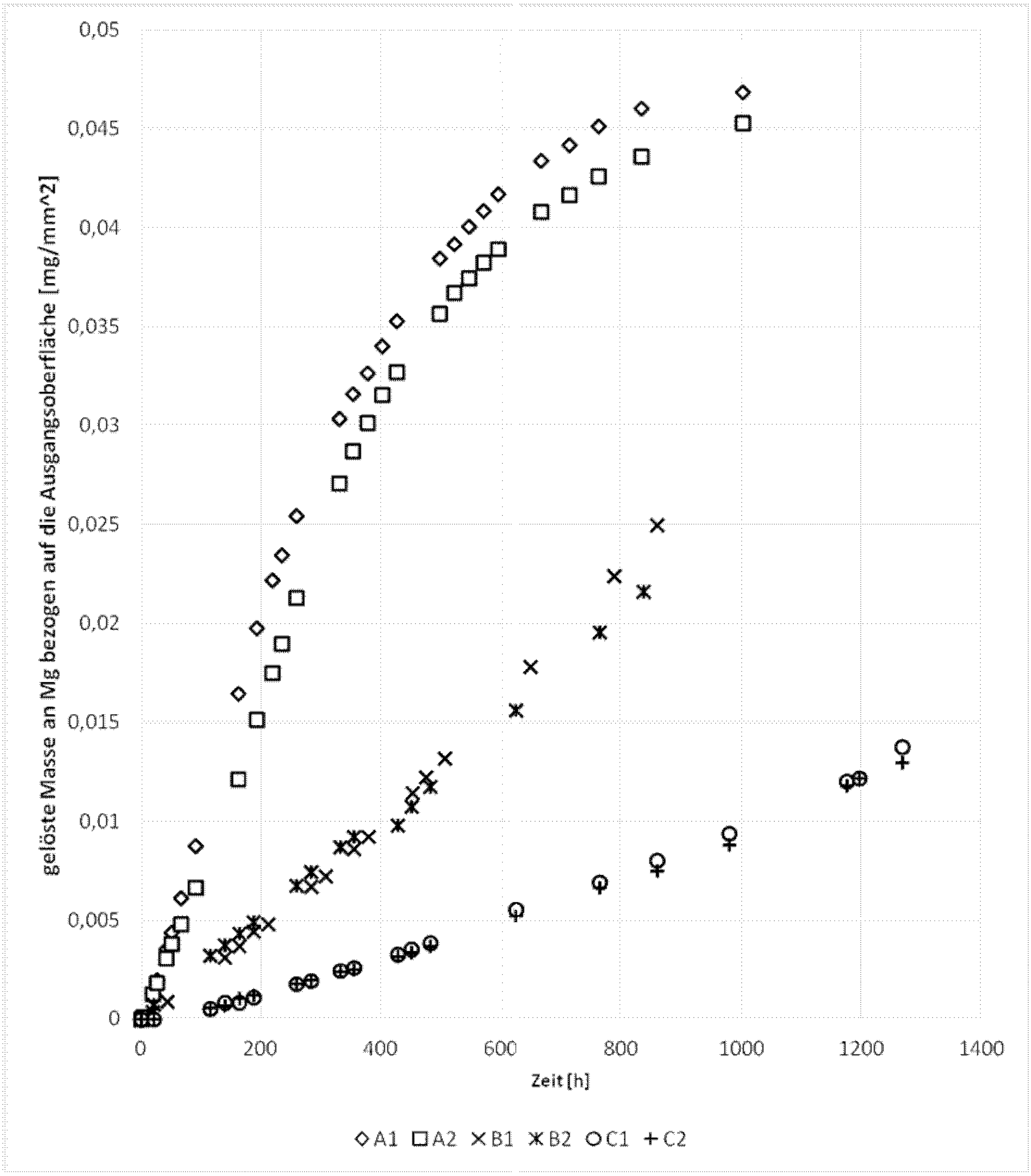
0,05
0,045
0,04
0,035
0,03
0,025
0,02
0,015
0,01
0,005
0
gelöste Masse an Mg bezogen auf die Ausgangsoberfläche [mg/mm^2]
0
200
400
600
800
1000
1200
1400
Zeit [h]
A1 A2 B1 B2 C1 C2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1419793 B1
- EP 2169090 A
- EP 2213314 A1
- EP 1842507 A1
- WO 2014143521 A1
- WO 2016073851 A2
- US 2010145436 A1
- WO 2010025078 A2
- WO 2013024125 A1
- CN 101468216


### In der Beschreibung aufgeführte Nicht-Patentliteratur


- **LIN et al.** beschreiben einen schützenden Effekt des Magnesiumfluoridfilms auf die JBDM-Legierung Mg2,5Nd0,2Zn0,4Zr. *ACS Applied Materials & Interfaces*, 2015, vol. 7, 5320-5330
- **NAN et al.** untersuchten den Einfluss einer Magnesiumfluorid- und einer Collagenbeschichtung auf die Bildung von Endothelzellen auf bioresorbierbaren Magnesiumstützen. *International Journal of Molecular Sciences*, 2014, vol. 15, 5263-5276